(19) Europäisches Patentamt
European Patent Office
Office européen des brevets

(11) EP 4 153 170 B1

(12) EUROPEAN PATENT SPECIFICATION

(45) Date of publication and mention of the grant of the patent:
**21.01.2026 Bulletin 2026/04**

(21) Application number: **21727147.7**

(22) Date of filing: **20.05.2021**

(51) International Patent Classification (IPC):
***A61K 31/403*** (2006.01) ***A61K 31/404*** (2006.01)
***A61K 31/407*** (2006.01) ***A61K 31/4178*** (2006.01)
***A61K 31/424*** (2006.01) ***A61K 31/437*** (2006.01)
***A61K 31/4439*** (2006.01) ***A61K 31/444*** (2006.01)
***A61P 1/00*** (2006.01) ***A61P 3/12*** (2006.01)
***A61P 9/00*** (2006.01) ***A61P 11/06*** (2006.01)
***A61P 13/10*** (2006.01) ***A61P 25/00*** (2006.01)
***A61P 43/00*** (2006.01) ***C07D 209/04*** (2006.01)
***C07D 209/34*** (2006.01) ***C07D 209/42*** (2006.01)
***C07D 401/04*** (2006.01) ***C07D 401/10*** (2006.01)
***C07D 471/04*** (2006.01) *C07D 491/107* (2006.01)
*C07D 498/04* (2006.01)

(52) Cooperative Patent Classification (CPC):
**A61K 31/403; A61K 31/404; A61K 31/407; A61K 31/4178; A61K 31/424; A61K 31/437; A61K 31/4439; A61K 31/444; A61P 1/00; A61P 3/12; A61P 9/00; A61P 11/06; A61P 13/10; A61P 25/00; A61P 43/00;** (Cont.)

(86) International application number:
**PCT/EP2021/063463**

(87) International publication number:
**WO 2021/234084 (25.11.2021 Gazette 2021/47)**

(54) **MAXI-K POTASSIUM CHANNEL OPENERS FOR THE TREATMENT OF FRAGILE X ASSOCIATED DISORDERS**

MAXI-K-KALIUMKANAL-ÖFFNER ZUR BEHANDLUNG VON MIT FRAGILEM X ASSOZIIERTEN ERKRANKUNGEN

OUVREURS DE CANAUX POTASSIQUES MAXI-K POUR LE TRAITEMENT DE TROUBLES LIÉS À L'X FRAGILE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**ME**

(30) Priority: **20.05.2020 EP 20175820**

(43) Date of publication of application:
**29.03.2023 Bulletin 2023/13**

(73) Proprietor: **Les Laboratoires Servier**
**92284 Suresnes Cedex (FR)**

(72) Inventors:
- **FLECK, Roman**
  **Jamaica Plain, Massachusetts 02130 (US)**
- **PROUDFOOT, John**
  **Newton, Connecticut 06470 (US)**

(74) Representative: **Vossius & Partner**
**Patentanwälte Rechtsanwälte mbB**
**Siebertstraße 3**
**81675 München (DE)**

(56) References cited:
**EP-A1- 0 747 354** **WO-A1-2005/080335**
**WO-A1-2008/129075** **WO-A1-2009/071687**
**WO-A1-2013/001412** **WO-A1-2020/183307**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 153 170 B1

WO-A2-03/008407 WO-A2-2005/097107
WO-A2-2009/056707 JP-A- 2000 191 661

- B. NARENDRAPRASAD REDDY ET AL: "Synthesis of Functionalized 6-Hydroxy-2-oxindole Derivatives by Phenoxide Cyclization", ORGANIC LETTERS, vol. 18, no. 24, 16 December 2016 (2016-12-16), US, pages 6264 - 6267, XP055743440, ISSN: 1523-7060, DOI: 10.1021/acs.orglett.6b03048
- EDWARD RICHMOND ET AL: "An asymmetric pericyclic cascade approach to 3-alkyl-3-aryloxindoles: generality, applications and mechanistic investigations", ORGANIC & BIOMOLECULAR CHEMISTRY, vol. 13, no. 6, 1 January 2015 (2015-01-01), pages 1807 - 1817, XP055743659, ISSN: 1477-0520, DOI: 10.1039/C4OB02526A
- LAWRENCE N J ET AL: "Synthesis of Diaryl Acetates and Oxoindoles via a Sequential VNSAR-SNAR Three-Component Coupling reaction", ORGANIC LETTERS, AMERICAN CHEMICAL SOCIETY, US, vol. 6, no. 26, 24 November 2004 (2004-11-24), pages 4957 - 4960, XP002380787, ISSN: 1523-7060, DOI: 10.1021/OL047890Y
- EDWARD RICHMOND ET AL: "Asymmetric Pericyclic Cascade Approach to Spirocyclic Oxindoles - Supplementary Information", ORGANIC LETTERS, vol. 14, no. 11, 22 June 2012 (2012-06-22), US, pages 1 - 92, XP055769741, ISSN: 1523-7060, DOI: 10.1021/ol300982f
- ABDERRAHMAN EL BOUAKHER ET AL: "A General and Efficient Method to Access Tetracyclic Spirooxindole Derivatives : A General and Efficient Route to Tetracyclic Spirooxindole Derivatives", EUROPEAN JOURNAL OF ORGANIC CHEMISTRY, vol. 2015, no. 3, 1 January 2015 (2015-01-01), DE, pages 556 - 569, XP055769874, ISSN: 1434-193X, DOI: 10.1002/ejoc.201403292
- MARVIN W. BARKER ET AL: "Heterocycles from ketenimines. 12. Pyrrolo[3,2-b]pyridines", THE JOURNAL OF ORGANIC CHEMISTRY, vol. 44, no. 7, 1 March 1979 (1979-03-01), pages 1175 - 1177, XP055769883, ISSN: 0022-3263, DOI: 10.1021/jo01321a037
- SYTNIK K M ET AL: "THE USING OF ACIDOCHROMAL CYCLOCONDENSATION IN THE SYNTHESIS OF 2-OXO-3,3DIPHENYL-2,3-DIHYDRO-1 H-THIENO[3,4-b]PYRROLE-6-CARBOXYLIC ACID DERIVATIVES [Use of acidochromatic cyclocondensation in the synthesis of 2-oxo-3,3-diphenyl-2,3-dihydro-1H-thieno[3,4-b]pyrrole-6-carboxylic acid derivatives]", ZHURNAL ORGANICHNOI TA FARMATSEVTICHNOI KHIMII, NATSIONAL'NII FARMATSEVTICHNII UNIVERSITET, RU, vol. 5, no. 1, 1 January 2007 (2007-01-01), pages 21 - 26, XP009525265, ISSN: 2308-8303

(52) Cooperative Patent Classification (CPC): (Cont.)
**C07D 209/34; C07D 209/42; C07D 401/04; C07D 401/10; C07D 471/04; C07D 491/107; C07D 498/04**

## Description

The present invention relates to the compounds of formula (I) as defined herein below for use in the treatment or prevention of a fragile X associated disorder, as well as the compounds of formula (Ia) as defined herein below and pharmaceutical compositions containing these compounds. The compounds provided herein can act as maxi-K potassium channel openers, which makes them suitable for use in therapy, particularly in the treatment or prevention of fragile X associated disorders, such as fragile X syndrome. The invention is as defined in the claims.

Fragile X syndrome (FXS) is the most common inherited cause of intellectual disability and the most common monogenic cause of autism globally (Riley C et al., Pediatrics, 2017, 139: S147-S52; Thurman AJ et al., Res Dev Disabil, 2014, 35: 1072-86). FXS is caused by a trinucleotide (CGG) repeat expansion in the 5' untranslated promoter region of the *FMR1* gene, which encodes the fragile X mental retardation protein (FMRP), an RNA-binding protein that regulates a wide range of mRNAs in neurons. The normal range of CGG triplet repeats in typically-developing (non-FXS) individuals is 5 to 44 (Grigsby J, Clin Neuropsychol, 2016, 30: 815-33), compared with >200 CGG repeats in individuals with FXS. A "full mutation" of >200 CGG repeats results in increased epigenetic modification (methylation) of the promoter, transcriptional silencing of *FMR1* and the commensurate loss or deficiency of FMRP. Although less common (<1%), FXS also can be caused by loss of function mutations and deletions in *FMR1*.

Maxi-K potassium channels are large-conductance, calcium ($Ca^{2+}$)-activated potassium channels, which can conduct large amounts of potassium ions ($K^+$) across the cell membrane and are therefore also known as big potassium ("BK") channels or calcium-activated big potassium ("BKCa") channels (Latorre R et al., Biol Res, 2006, 39(3): 385-401). Maxi-K potassium channels (BKCa channels) are widely distributed in the brain. They function as neuronal calcium sensors and contribute to repolarization of neurons and control of neuronal signaling via regulation of cellular excitability and neurotransmitter release. In the brain, the pharmacological opening of BKCa channels can protect neurons by enhancing an endogenous neuroprotective mechanism (Gribkoff VK et al., Nat Med, 2001, 7: 471-7).

The gene encoding BKCa (*KCNMA1*) was identified within a *de novo* balanced 9q23/10q22 translocation of a patient exhibiting both autism and intellectual deficiency (Laumonnier F et al., Am J Psychiatry, 2006, 163: 1622-9). Demonstration of *KCNMA1* haploinsufficiency and reduced BKCa channel function in this patient suggested an underlying BKCa channelopathy behind the clinical phenotypes of autism and intellectual disability.

BKCa is a known molecular target of FMRP (Deng PY et al., Neuron, 2013, 77: 696-711). Functionally, presynaptic BKCa channels have been shown to play an important role in FMRP regulation of action potential duration, neurotransmitter release, and short-term plasticity in hippocampal pyramidal neurons. Single channel recordings in CA3 pyramidal neurons showed that BKCa potassium channel open probability was reduced by loss of FMRP and that FMRP acts on BKCa potassium channels by modulating the channel's gating kinetics; genetic upregulation of BKCa was shown to normalize synaptic and circuit deficits in a mouse model of FXS (Deng PY et al., J Physiol, 2016, 594: 83-97). Positive modulation of BKCa function may thus represent a novel way to address deficits created by loss of FMRP in the nervous system, and may provide a novel approach to the treatment of FXS.

Preclinical validation for this hypothesis is found in studies with BK channel openers. The Fmr1 knockout (KO) mouse model recapitulates many of the clinical abnormalities observed in FXS patients and is considered an appropriate model for preclinical assessment of potential therapies against FXS. In this model, a single intraperitoneal (IP) injection of 2 mg/kg BMS-204352 (corresponding to a $C_{max}$ of about 130 ng/mL) reversed dendritic spine abnormalities, rescued hippocampal glutamate homeostasis, and reversed a range of behavioral phenotypes (Hébert B et al., Orphanet J Rare Dis, 2014, 9: 124).

Moreover, BK channel openers have also been proposed for the therapeutic intervention in other diseases, including Williams-Beuren syndrome (Piquemal M et al., bioRxiv, 2020, doi: 10.11 01/2020.12.22.423977).

The compound BMS-204352 (MaxiPost™), i.e. (3S)-(+)-(5-chloro-2-methoxyphenyl)-1,3-dihydro-3-fluoro-6-(trifluoromethyl)-2H-indol-2-one, is a highly potent maxi-K potassium channel opener, which was originally developed by Bristol-Myers Squibb for the treatment of acute ischemic stroke (Jensen BS, CNS Drug Rev, 2002, 8(4): 353-60). However, it has been found that this compound gives rise to a metabolic degradation product that covalently binds to plasma proteins, which poses a major risk of undesirable side effects and thus limits the therapeutic potential of this compound, particularly for long-term administration (Dalvie D et al., "Influence of aromatic rings on ADME properties of drugs", in: Smith DA (ed.), "Metabolism, Pharmacokinetics and Toxicity of Functional Groups", Royal Society of Chemistry, Cambridge (UK), 2010, p. 275-327; Evans DC et al., Chem Res Toxicol, 2004, 17(1): 3-16).

Other oxindoles besides BMS-204352, particularly other 3-fluorooxindoles, have also been proposed as potassium channel modulators but suffer from similar drawbacks (Hewawasam P et al., Bioorg Med Chem Lett, 2002, 12(7): 1023-6; US 5,565,483; US 5,602,169; WO 02/00217; WO 02/32419; WO 02/066426; WO 03/080047; WO 2013/001412).

Moreover, certain indole derivatives have been described as synthetic intermediates or for different therapeutic applications unrelated to potassium channel modulation (Kündig EP et al., Angew Chem Int Ed Engl, 2007, 46(44): 8484-7; Würtz S et al., J Am Chem Soc, 2009, 131(24): 8344-5; Liu L et al., Org Lett, 2011, 13(7): 1666-9; Dey C et al.,

Chem Commun (Camb), 2012, 48(25): 3064-6; Kinthada LK et al., Org Biomol Chem, 2014, 12(41): 8152-73; US 2007/0203184; WO 01/74775; WO 2014/154760).

There is still an urgent and unmet need for novel maxi-K potassium channel openers which can be used in the therapy of fragile X syndrome and which do not suffer from the drawbacks associated with BMS-204352.

The present invention addresses this need and solves the problem of providing novel and improved maxi-K potassium channel openers, which have surprisingly been found to exhibit an advantageously potent stimulating activity on maxi-K channels while, at the same time, they do not give rise to metabolic degradation products that form covalent adducts with proteins (as in the case of BMS-204352) and, thus, provide an improved side effect profile and allow chronic administration. Moreover, they exhibit a beneficial solubility and pharmacokinetic profile. These properties render the compounds of the present invention particularly advantageous for use in therapy, specifically as a chronic medication for the treatment or prevention of fragile X syndrome and related disorders.

In a first aspect, the present invention thus relates to a compound of the following formula (I)

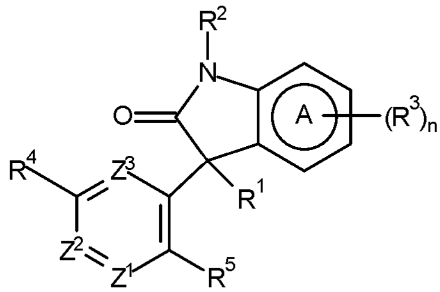

(I)

or a pharmaceutically acceptable salt or solvate thereof, for use in the treatment or prevention of a fragile X associated disorder, particularly fragile X syndrome.

In formula (I), $R^1$ is selected from $C_{1-5}$ alkyl, $C_{2-5}$ alkenyl, $C_{2-5}$ alkynyl, -($C_{0-4}$ alkylene)-CN, -($C_{1-4}$ alkylene)-COOH, -($C_{0-4}$ alkylene)-COO-($C_{1-5}$ alkyl), -($C_{1-4}$ alkylene)-O-CO-($C_{1-5}$ alkyl), -($C_{0-4}$ alkylene)-CO-($C_{1-5}$ alkyl), -($C_{0-4}$ alkylene)-CO-$NH_2$, -($C_{0-4}$ alkylene)-CO-NH($C_{1-5}$ alkyl), and -($C_{0-4}$ alkylene)-CO-N($C_{1-5}$ alkyl)($C_{1-5}$ alkyl), and $R^5$ is a group $R^{5A}$.

$R^2$ is hydrogen.

Ring A is phenyl which is fused to the ring containing -CO-$NR^2$-.

Each $R^3$ is independently halogen or $C_{1-5}$ haloalkyl.

The variable n is an integer of 0 to 4.

One of the ring atoms $Z^1$, $Z^2$ and $Z^3$ is C(-$R^6$) or N, and the other two of said ring atoms are each C(-$R^6$).

$R^4$ is selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), -($C_{0-4}$ alkylene)-SO-($C_{1-5}$ alkyl), -($C_{0-4}$ alkylene)-carbocyclyl, and -($C_{0-4}$ alkylene)-heterocyclyl, wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more groups $R^{Cyc}$.

$R^{5A}$ is selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkyle-

ne)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), -($C_{0-4}$ alkylene)-SO-($C_{1-5}$ alkyl), -($C_{0-4}$ alkylene)-carbocyclyl, and -($C_{0-4}$ alkylene)-heterocyclyl, wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more groups $R^{Cyc}$.

Each $R^6$ is independently selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), -($C_{0-4}$ alkylene)-SO-($C_{1-5}$ alkyl), -($C_{0-4}$ alkylene)-carbocyclyl, and -($C_{0-4}$ alkylene)-heterocyclyl, wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more groups $R^{Cyc}$.

Each $R^{Cyc}$ is independently selected from $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), and -($C_{0-4}$ alkylene)-SO-($C_{1-5}$ alkyl).

In accordance with the above-described first aspect, the present invention also relates to a pharmaceutical composition comprising a compound of formula (I), or a pharmaceutically acceptable salt or solvate thereof, and a pharmaceutically acceptable excipient, for use in the treatment or prevention of a fragile X associated disorder (particularly fragile X syndrome). Accordingly, the invention in particular provides a pharmaceutical composition comprising, as an active ingredient, a compound of formula (I) or a pharmaceutically acceptable salt or solvate thereof, together with a pharmaceutically acceptable excipient, for use in the treatment or prevention of a fragile X associated disorder (such as fragile X syndrome).

The fragile X associated disorder to be treated or prevented in accordance with the present invention may be, in particular, a genetic disease/disorder caused by (or associated with) a change or mutation in the fragile X mental retardation 1 *(FMR1)* gene and/or in the fragile X mental retardation 2 *(FMR2)* gene, such as a pathologically enhanced number of CGG repeats (e.g., the presence of about 45 or more CGG repeats, particularly about 55 or more CGG repeats, especially more than about 200 CGG repeats, or even more than about 230 CGG repeats in the *FMR1* gene), or a disease/disorder caused by (or associated with) a deficiency, absence, functional impairment, or dysfunction of the protein encoded by the *FMR1* gene, i.e. fragile X mental retardation protein (FMRP), and/or of the protein encoded by the *FMR2* gene, particularly a disease/disorder caused by (or associated with) a change or mutation in the *FMR1* gene. The term "fragile X associated disorder" is used herein synonymously and interchangeably with "fragile X spectrum disorder". Corresponding fragile X associated disorders have been described in the literature, e.g., in: Maddalena A et al., Genet Med, 2001, 3(3): 200-5; Monaghan KG et al., Genet Med, 2013, 15(7): 575-86; Mulley JC et al., J Med Genet, 1995, 32(3): 162-9; Gecz J, Ann Hum Genet, 2000, 64(Pt 2): 95-106; Myrick LK et al., Proc Natl Acad Sci USA, 2015, 112(4): 949-56; Hagerman PJ et al., Ann N Y Acad Sci, 2015, 1338(1): 58-70; Jacquemont S et al., Eur J Hum Genet, 2011, 19(9); Hall DA et al., Handb Clin Neurol, 2018, 147: 377-91; Hunter JE et al., "FMR1 Disorders", in: Adam MP et al. (eds), GeneReviews, University of Washington, Seattle (WA), USA, 1998 (updated in November 2019); Lozano R et al., Intractable Rare Dis Res, 2014, 3(4): 134-46; and the references cited in the aforementioned publications.

Preferably, the fragile X associated disorder to be treated or prevented in accordance with the present invention is

selected from fragile X syndrome (FXS), fragile X-associated tremor/ataxia syndrome (FXTAS), fragile X-associated primary ovarian insufficiency (FXPOI), fragile X-associated polycystic ovarian syndrome, fragile XE-associated mental retardation (FRAXE), a clinical syndrome associated with pathogenic mutations in the *FMR1* gene (particularly such mutations that result in altered BK channel function; see, e.g., Myrick LK et al., Proc Natl Acad Sci USA, 2015, 112(4): 949-56), and a clinical syndrome associated with pathogenic variation in the *KCNMA1* gene (such as, e.g., paroxysmal nonkinesigenic dyskinesia (PNKD), particularly PNKD3, with or without generalized epilepsy; see, e.g., Liang L et al., Hum Mol Genet, 2019, 28(17): 2937-51). More preferably, the fragile X associated disorder is selected from fragile X syndrome (FXS), fragile X-associated tremor/ataxia syndrome (FXTAS), fragile X-associated primary ovarian insufficiency (FXPOI), fragile X-associated polycystic ovarian syndrome, and fragile XE-associated mental retardation (FRAXE). Even more preferably, the fragile X associated disorder is selected from fragile X syndrome (FXS), fragile X-associated tremor/ataxia syndrome (FXTAS), and fragile X-associated primary ovarian insufficiency (FXPOI). Yet even more preferably, the fragile X associated disorder is fragile X syndrome.

Accordingly, the present invention relates, in particular, to a compound of formula (I) or a pharmaceutically acceptable salt or solvate thereof (or a pharmaceutical composition comprising any of the aforementioned entities in combination with a pharmaceutically acceptable excipient) for use in the treatment or prevention of fragile X syndrome (FXS), fragile X-associated tremor/ataxia syndrome (FXTAS), or fragile X-associated primary ovarian insufficiency (FXPOI), preferably for use in the treatment or prevention of fragile X syndrome.

In an aspect, the present disclosure furthermore relates to the use of a compound of formula (I) or a pharmaceutically acceptable salt or solvate thereof (as defined in the first aspect of the invention) as a maxi-K potassium channel opener in research, particularly as a research tool compound for stimulating maxi-K potassium channels. Accordingly, in a second aspect, the invention refers to the *in vitro* use of a compound of formula (I) or a pharmaceutically acceptable salt or solvate thereof as a maxi-K potassium channel opener and, in particular, to the *in vitro* use of a compound of formula (I) or a pharmaceutically acceptable salt or solvate thereof as a research tool compound acting as a maxi-K potassium channel opener. The terms "sample", "test sample" and "biological sample" include, without being limited thereto: a cell, a cell culture, or a cellular or subcellular extract; biopsied material obtained from an animal (e.g., a human), or an extract thereof; or blood, serum, plasma, saliva, urine, or any other body fluid, or an extract thereof. It is to be understood that the term *"in vitro"* is used in this specific context in the sense of "outside a living human or animal body", which includes, in particular, experiments performed with cells, cellular or subcellular extracts, and/or biological molecules in an artificial environment such as an aqueous solution or a culture medium which may be provided, e.g., in a flask, a test tube, a Petri dish, a microtiter plate, etc.

In a third aspect, the present invention provides novel compounds, i.e., the invention relates to a compound of the following formula (Ia) or a pharmaceutically acceptable salt or solvate thereof:

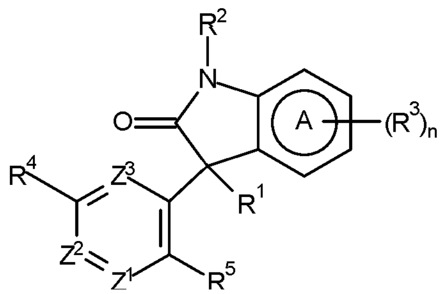

(Ia)

wherein the groups/variables in formula (Ia), including in particular ring A, $Z^1$, $Z^2$, $Z^3$, $R^1$, $R^2$, $R^3$, $R^4$, $R^5$, $R^{5A}$, $R^6$, $R^{Cyc}$ and n, have the same meanings, including the same preferred meanings, as the corresponding groups/variables comprised in the compound of formula (I) according to the first aspect of the invention; and
wherein the following further conditions apply to the compound of formula (Ia):

- n is 1, 2, 3 or 4; and
- if n is 1 or 2, then at least one group $R^3$ is different from halogen.

The compounds of formula (Ia) can be used as active pharmaceutical ingredients, particularly as maxi-K potassium channel openers. The present invention thus also relates to the compound of formula (Ia) or a pharmaceutically acceptable salt or solvate thereof for use in the treatment or prevention of a fragile X associated disorder (such as, e.g., fragile X syndrome (FXS), fragile X-associated tremor/ataxia syndrome (FXTAS), or fragile X-associated primary ovarian insufficiency (FXPOI)) or other diseases/disorders in which maxi-K potassium channels are implicated.

In this third aspect, the present invention further relates to a pharmaceutical composition comprising a compound of formula (Ia), or a pharmaceutically acceptable salt or solvate thereof, in combination with a pharmaceutically acceptable excipient. Accordingly, the invention relates to a compound of formula (Ia) or a pharmaceutically acceptable salt or solvate thereof, or a pharmaceutical composition comprising any of the aforementioned entities and a pharmaceutically acceptable excipient, for use as a medicament.

In particular, the invention refers to a compound of formula (Ia) or a pharmaceutically acceptable salt or solvate thereof, or a pharmaceutical composition comprising any of the aforementioned entities and a pharmaceutically acceptable excipient, for use in the treatment or prevention of a disease/disorder selected from stroke (e.g., ischemic stroke), ischemia reperfusion injury, ischemic heart disease, hypertension (e.g., partial hypertension or arterial hypertension), myocardial infarction, allergic rhinitis, asthma, chronic obstructive pulmonary disease (COPD), multiple sclerosis, spasticity (e.g., muscle spasticity, or spasticity in a subject/patient suffering from multiple sclerosis), tremor, a muscular disorder, dyskinesia, bladder dysfunction, overactive bladder (OAB), urinary incontinence, irritable bowel syndrome (IBS), faecal incontinence, constipation, gastro-oesophageal reflux disorder (GERD), impaired gastrointenstinal passage, detrusor underactivity, erectile dysfunction, opioid-induced respiratory depression, anxiety, an anxiety disorder (e.g., generalized anxiety disorder (GAD) or social anxiety disorder (SAD)), sensory processing disorder (SPD), autism, an autism spectrum disorder, a social (pragmatic) communication disorder, attention deficit hyperactivity disorder (ADHD), social phobia, intellectual disability, pain (e.g., neuropathic pain), epilepsy, an epilepsy and/or seizure disorder (e.g., Dravet syndrome), generalized epilepsy and paroxysmal dyskinesia (GEPD), temporal lobe epilepsy, psychosis, schizophrenia, negative symptoms of schizophrenia, cognitive impairment in schizophrenia, Phelan-McDermid syndrome, Rett syndrome, Pitt-Hopkins syndrome, 16p11.2 deletion syndrome, Williams-Beuren syndrome, open-angle ocular hypertension, glaucoma, tinnitus, diabetic retinopathy, tocolysis (or prevention of preterm labor), migraine, Liang-Wang syndrome, Smith-Lemli-Opitz syndrome, Angelman syndrome, and Hutchinson-Gilford progeria syndrome.

Unless indicated otherwise, the following detailed description relates to each aspect of the present invention, including each one of the above-discussed first aspect, second aspect, and third aspect of the invention.

The compounds of formula (I) as well as the pharmaceutically acceptable salts and solvates thereof will be described in more detail in the following:

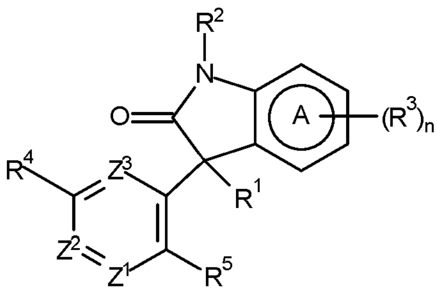

(I)

In formula (I), $R^1$ is selected from $C_{1-5}$ alkyl, $C_{2-5}$ alkenyl, $C_{2-5}$ alkynyl, -($C_{0-4}$ alkylene)-CN (e.g., -$CH_2$-CN), -($C_{1-4}$ alkylene)-COOH (e.g., -$CH_2$-COOH), -($C_{0-4}$ alkylene)-COO-($C_{1-5}$ alkyl), -($C_{1-4}$ alkylene)-O-CO-($C_{1-5}$ alkyl), -($C_{0-4}$ alkylene)-CO-($C_{1-5}$ alkyl), -($C_{0-4}$ alkylene)-CO-$NH_2$, -($C_{0-4}$ alkylene)-CO-NH($C_{1-5}$ alkyl), and -($C_{0-4}$ alkylene)-CO-N($C_{1-5}$ alkyl)($C_{1-5}$ alkyl), and $R^5$ is a group $R^{5A}$.

Preferably, R' is $C_{1-5}$ alkyl, and $R^5$ is $R^{5A}$. More preferably, $R^1$ is methyl or ethyl, and $R^5$ is $R^{5A}$. Even more preferably, $R^1$ is methyl, and $R^5$ is $R^{5A}$.

$R^2$ is hydrogen.

Ring A is phenyl, wherein said phenyl is fused to the ring containing -CO-$NR^2$-. As also depicted in formula (I), ring A is thus fused to the 5-membered ring that contains the ring members C(=O) and N(-$R^2$).

As explained above, ring A is phenyl which is fused to the ring containing -CO-$NR^2$-. Accordingly, the compound of formula (I) has the following structure:

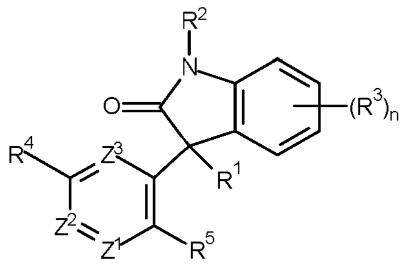

Each $R^3$ is independently halogen (e.g., -F, -Cl or -Br) or $C_{1-5}$ haloalkyl (e.g., $-CF_3$, $-CHF_2$ or $-CH_2F$). Preferably, each $R^3$ is $-CF_3$.

n is an integer of 0 to 4 (i.e., 0, 1, 2, 3 or 4). Preferably, n is 0, 1 or 2. More preferably, n is 1.

It is to be understood that n indicates the number of substituents $R^3$ that are bound to ring A. If n is 0, then ring A is not substituted with any group $R^3$, i.e. is substituted with hydrogen instead of $R^3$.

As explained above, each $R^3$ is independently halogen (e.g., -F, -Cl or -Br) or $C_{1-5}$ haloalkyl (e.g., $-CF_3$, $-CHF_2$ or $-CH_2F$). It is particularly preferred that the group $R^3$ (if n is 1), or at least one group $R^3$ (if n is 2, 3 or 4), is $-CF_3$. Moreover, it is preferred that said halogen or said $C_{1-5}$ haloalkyl (or said $-CF_3$) is attached to ring A (which is phenyl) at a ring carbon atom in the 4-position, the 5-position, the 6-position or the 7-position, more preferably in the 5-position, the 6-position or the 7-position, even more preferably in the 5-position or the 6-position, yet even more preferably in the 6-position, wherein the numbering of ring atoms is as illustrated in the following:

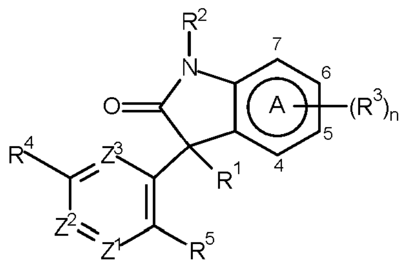

Thus, if n is 1 and $R^3$ is $-CF_3$ which is attached to ring A (which is phenyl) in the 6-position, then the compound of formula (I) has the following structure:

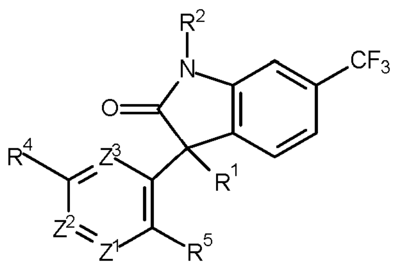

One of the ring atoms $Z^1$, $Z^2$ and $Z^3$ is $C(-R^6)$ or N, and the other two ring atoms are each $C(-R^6)$.

Preferably, one of the ring atoms $Z^1$ and $Z^2$ is $C(-R^6)$ or N, the other one of said ring atoms $Z^1$ and $Z^2$ is $C(-R^6)$, and $Z^3$ is $C(-R^6)$. More preferably, $Z^2$ is $C(-R^6)$ or N, while $Z^1$ and $Z^3$ are each $C(-R^6)$. For example, $Z^2$ may be N, while $Z^1$ and $Z^3$ may each be $C(-R^6)$; in this case, the compound of formula (I) has the following structure:

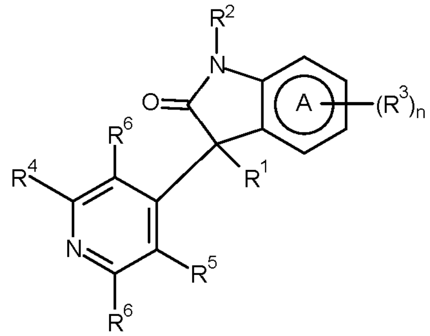

Even more preferably, $Z^1$, $Z^2$ and $Z^3$ are each C(-$R^6$). Accordingly, it is particularly preferred that the compound of formula (I) has the following structure:

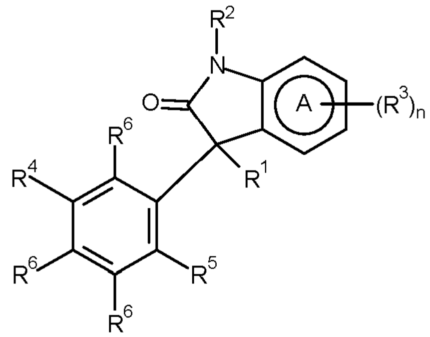

$R^4$ is selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), -($C_{0-4}$ alkylene)-SO-($C_{1-5}$ alkyl), -($C_{0-4}$ alkylene)-carbocyclyl, and -($C_{0-4}$ alkylene)-heterocyclyl, wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more (e.g., one, two or three) groups $R^{Cyc}$.

Preferably, $R^4$ is selected from hydrogen, $C_{1-5}$ alkyl, -O($C_{1-5}$ alkyl), halogen, $C_{1-5}$ haloalkyl, -O($C_{1-5}$ haloalkyl) (e.g., -$OCF_3$), -CN, -$SO_2$-($C_{1-5}$ alkyl), -($C_{0-4}$ alkylene)-carbocyclyl, and -($C_{0-4}$ alkylene)-heterocyclyl (such as -($C_{0-4}$ alkylene)-heteroaryl; e.g., imidazolyl), wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more groups $R^{Cyc}$. More preferably, $R^4$ is selected from hydrogen, $C_{1-5}$ alkyl, -O($C_{1-5}$ alkyl), halogen, $C_{1-5}$ haloalkyl, -O($C_{1-5}$ haloalkyl) (e.g., -$OCF_3$), -CN, -$SO_2$-($C_{1-5}$ alkyl), aryl, and heteroaryl (such as, e.g., imidazolyl), wherein said aryl and said heteroaryl are each optionally substituted with one or more groups $R^{Cyc}$ (for example, $R^4$ may be N-methylimidazolyl, such as 1-methylimidazol-2-yl, 1-methylimidazol-4-yl, or 1-methylimidazol-5-yl). Even more preferably, $R^4$ is halogen (e.g., -F, -Cl, -Br or -I), -$CF_3$, or -CN. It is particularly preferred that $R^4$ is -Cl.

As explained above, $R^5$ is a group $R^{5A}$.

$R^{5A}$ is selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)

($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), -($C_{0-4}$ alkylene)-SO-($C_{1-5}$ alkyl), -($C_{0-4}$ alkylene)-carbocyclyl, and -($C_{0-4}$ alkylene)-heterocyclyl, wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more (e.g., one, two or three) groups $R^{Cyc}$.

Preferably, $R^{5A}$ is selected from hydrogen, -O($C_{1-5}$ alkyl), -OH, halogen, $C_{1-5}$ haloalkyl, -O-($C_{1-5}$ haloalkyl), -CN, -CO-($C_{1-5}$ alkyl), -COOH, -CO-O-($C_{1-5}$ alkyl), -CO-$NH_2$, -CO-NH($C_{1-5}$ alkyl), and -CO-N($C_{1-5}$ alkyl)($C_{1-5}$ alkyl). More preferably, $R^{5A}$ is selected from hydrogen, -O($C_{1-5}$ alkyl), -OH, halogen, $C_{1-5}$ haloalkyl, -CN, -COOH, and -CO-$NH_2$. Even more preferably, $R^{5A}$ is -O($C_{1-5}$ alkyl) or -OH. Yet even more preferably, $R^{5A}$ is -$OCH_3$, -$OCH_2CH_3$ or -OH. Still more preferably, $R^{5A}$ is -$OCH_3$.

Each $R^6$ is independently selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), -($C_{0-4}$ alkylene)-SO-($C_{1-5}$ alkyl), -($C_{0-4}$ alkylene)-carbocyclyl, and -($C_{0-4}$ alkylene)-heterocyclyl, wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more (e.g., one, two or three) groups $R^{Cyc}$.

Preferably, each $R^6$ is independently selected from hydrogen, $C_{1-5}$ alkyl, $C_{2-5}$ alkenyl, $C_{2-5}$ alkynyl, -OH, -O($C_{1-5}$ alkyl), -O($C_{1-5}$ alkylene)-OH, -O($C_{1-5}$ alkylene)-O($C_{1-5}$ alkyl), -SH, -S($C_{1-5}$ alkyl), -$NH_2$, -NH($C_{1-5}$ alkyl), -N($C_{1-5}$ alkyl)($C_{1-5}$ alkyl), -NH-OH, -N($C_{1-5}$ alkyl)-OH, -NH-O($C_{1-5}$ alkyl), -N($C_{1-5}$ alkyl)-O($C_{1-5}$ alkyl), halogen, $C_{1-5}$ haloalkyl, -O-($C_{1-5}$ haloalkyl), -CN, -CHO, -CO-($C_{1-5}$ alkyl), -COOH, -CO-O-($C_{1-5}$ alkyl), -O-CO-($C_{1-5}$ alkyl), -CO-$NH_2$, -CO-NH($C_{1-5}$ alkyl), -CO-N($C_{1-5}$ alkyl)($C_{1-5}$ alkyl), -NH-CO-($C_{1-5}$ alkyl), -N($C_{1-5}$ alkyl)-CO-($C_{1-5}$ alkyl), -NH-CO-O-($C_{1-5}$ alkyl), -N($C_{1-5}$ alkyl)-CO-O-($C_{1-5}$ alkyl), -O-CO-NH-($C_{1-5}$ alkyl), -O-CO-N($C_{1-5}$ alkyl)-($C_{1-5}$ alkyl), -$SO_2$-$NH_2$, -$SO_2$-NH($C_{1-5}$ alkyl), -$SO_2$-N($C_{1-5}$ alkyl)($C_{1-5}$ alkyl), -NH-$SO_2$-($C_{1-5}$ alkyl), -N($C_{1-5}$ alkyl)-$SO_2$-($C_{1-5}$ alkyl), -$SO_2$-($C_{1-5}$ alkyl), and -SO-($C_{1-5}$ alkyl). More preferably, each $R^6$ is independently selected from hydrogen, $C_{1-5}$ alkyl, -O($C_{1-5}$ alkyl), halogen, $C_{1-5}$ haloalkyl, -O-($C_{1-5}$ haloalkyl), and -CN. Even more preferably, each $R^6$ is hydrogen.

Each $R^{Cyc}$ is independently selected from $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), and -($C_{0-4}$ alkylene)-SO-($C_{1-5}$ alkyl).

Preferably, each $R^{Cyc}$ is independently selected from $C_{1-5}$ alkyl, $C_{2-5}$ alkenyl, $C_{2-5}$ alkynyl, -OH, -O($C_{1-5}$ alkyl), -O($C_{1-5}$ alkylene)-OH, -O($C_{1-5}$ alkylene)-O($C_{1-5}$ alkyl), -SH, -S($C_{1-5}$ alkyl), -$NH_2$, -NH($C_{1-5}$ alkyl), -N($C_{1-5}$ alkyl)($C_{1-5}$ alkyl), -NH-OH, -N($C_{1-5}$ alkyl)-OH, -NH-O($C_{1-5}$ alkyl), -N($C_{1-5}$ alkyl)-O($C_{1-5}$ alkyl), halogen, $C_{1-5}$ haloalkyl, -O-($C_{1-5}$ haloalkyl), -CN, -CHO, -CO-($C_{1-5}$ alkyl), -COOH, -CO-O-($C_{1-5}$ alkyl), -O-CO-($C_{1-5}$ alkyl), -CO-$NH_2$, -CO-NH($C_{1-5}$ alkyl), -CO-N($C_{1-5}$ alkyl)($C_{1-5}$ alkyl), -NH-CO-($C_{1-5}$ alkyl), -N($C_{1-5}$ alkyl)-CO-($C_{1-5}$ alkyl), -NH-CO-O-($C_{1-5}$ alkyl), -N($C_{1-5}$ alkyl)-CO-O-($C_{1-5}$ alkyl), -O-CO-NH-($C_{1-5}$ alkyl), -O-CO-N($C_{1-5}$ alkyl)-($C_{1-5}$ alkyl), -$SO_2$-$NH_2$, -$SO_2$-NH($C_{1-5}$ alkyl), -$SO_2$-N($C_{1-5}$ alkyl)($C_{1-5}$ alkyl), -NH-$SO_2$-($C_{1-5}$ alkyl), -N($C_{1-5}$ alkyl)-$SO_2$-($C_{1-5}$ alkyl), -$SO_2$-($C_{1-5}$ alkyl), and -SO-($C_{1-5}$ alkyl). More preferably, each $R^{Cyc}$ is independently selected from $C_{1-5}$ alkyl, -OH, -O($C_{1-5}$ alkyl), -O($C_{1-5}$ alkylene)-OH, -O($C_{1-5}$ alkylene)-O($C_{1-5}$ alkyl), -SH, -S($C_{1-5}$ alkyl), -$NH_2$, -NH($C_{1-5}$ alkyl), -N($C_{1-5}$ alkyl)($C_{1-5}$ alkyl), halogen, $C_{1-5}$ haloalkyl, -O-($C_{1-5}$ haloalkyl), and -CN.

It is particularly preferred that the compound of formula (I) is any one of the specific compounds of formula (I) described in the examples section of this specification, either in non-salt form or as a pharmaceutically acceptable salt or

solvate of the respective compound.

Accordingly, it is particularly preferred that the compound of formula (I) is selected from: 3-(5-chloro-2-methoxyphenyl)-3-methyl-6-(trifluoromethyl)indolin-2-one; 3-(5-chloro-2-hydroxy-phenyl)-3-methyl-6-(trifluoromethyl)indolin-2-one; 3-(5-chloro-2-methoxyphenyl)-3-ethyl-6-(trifluoromethyl)indolin-2-one; 3-(5-chloro-2-methoxyphenyl)-3-propyl-6-(trifluoromethyl)indolin-2-one; 2-(3-(5-chloro-2-methoxyphenyl)-2-oxo-6-(trifluoromethyl)indolin-3-yl)acetonitrile; 3-allyl-3-(5-chloro-2-methoxyphenyl)-6-(trifluoromethyl)indolin-2-one; 2-(3-(5-chloro-2-methoxyphenyl)-2-oxo-6-(trifluoromethyl)indolin-3-yl)acetic acid; 3-(2-chloro-5-methoxy-4-pyridyl)-3-methyl-6-(trifluoromethyl)indolin-2-one; 3-(3-methoxy-4-pyridyl)-3-methyl-6-(trifluoromethyl)indolin-2-one; 3-methyl-3-(4-pyridyl)-6-(trifluoromethyl)indolin-2-one; 3-(2-chloro-4-pyridyl)-3-methyl-6-(trifluoromethyl)indolin-2-one; 3-[2-methoxy-5-(1-methylimidazol-2-yl)phenyl]-3-methyl-6-(trifluoromethyl)indolin-2-one; 3-(5-chloro-2-methoxy-phenyl)-6-(difluoromethyl)-3-methyl-indolin-2-one; 3-(5-chloro-2-methoxy-3-pyridyl)-3-methyl-6-(trifluoromethyl)indolin-2-one; 6-chloro-3-(5-chloro-2-methoxyphenyl)-3-methylindolin-2-one; 6-chloro-3-(2-chloro-5-methoxypyridin-4-yl)-3-methylindolin-2-one; 3-(6-chloro-3-methoxypyridin-2-yl)-3-methyl-6-(trifluoromethyl)indolin-2-one; 6-chloro-3-(5-chloro-2-methoxypyridin-3-yl)-3-methylindolin-2-one; 6-chloro-3-(2-methoxy-5-(1-methyl-1H-imidazol-2-yl)phenyl)-3-methylindolin-2-one; 6-chloro-3-(6-chloro-3-methoxypyridin-2-yl)-3-methylindolin-2-one; 6-chloro-3-(2-methoxy-5-(1-methyl-1H-imidazol-2-yl)pyridin-3-yl)-3-methylindolin-2-one; 6-chloro-3-(5-methoxy-2-(1-methyl-1H-imidazol-2-yl)pyridin-4-yl)-3-methylindolin-2-one; or a pharmaceutically acceptable salt or solvate of any one of the above-mentioned compounds.

The invention furthermore relates specifically and individually to each stereoisomer of the above-mentioned compounds, including in particular to each one of these compounds having the S-configuration at the carbon atom carrying $R^1$, or to each one of these compounds having the R-configuration at the carbon atom carrying $R^1$.

It is thus particularly preferred that the compound of formula (I) is selected from: (3S)-3-(5-chloro-2-methoxy-phenyl)-3-methyl-6-(trifluoromethyl)indolin-2-one; (3R)-3-(5-chloro-2-methoxy-phenyl)-3-methyl-6-(trifluoromethyl)indolin-2-one; (3S)-3-(5-chloro-2-hydroxy-phenyl)-3-methyl-6-(trifluoromethyl)indolin-2-one; (3R)-3-(5-chloro-2-hydroxy-phenyl)-3-methyl-6-(trifluoromethyl)indolin-2-one; (3S)-3-(5-chloro-2-methoxyphenyl)-3-ethyl-6-(trifluoromethyl)indolin-2-one; (3R)-3-(5-chloro-2-methoxyphenyl)-3-ethyl-6-(trifluoromethyl)indolin-2-one; (3S)-3-(5-chloro-2-methoxyphenyl)-3-propyl-6-(trifluoromethyl)indolin-2-one; (3R)-3-(5-chloro-2-methoxyphenyl)-3-propyl-6-(trifluoromethyl)indolin-2-one; (3S)-2-(3-(5-chloro-2-methoxyphenyl)-2-oxo-6-(trifluoromethyl)indolin-3-yl)acetonitrile; (3R)-2-(3-(5-chloro-2-methoxyphenyl)-2-oxo-6-(trifluoromethyl)indolin-3-yl)acetonitrile; (3S)-3-allyl-3-(5-chloro-2-methoxyphenyl)-6-(trifluoromethyl)indolin-2-one; (3R)-3-allyl-3-(5-chloro-2-methoxyphenyl)-6-(trifluoromethyl)indolin-2-one; (3S)-2-(3-(5-chloro-2-methoxyphenyl)-2-oxo-6-(trifluoromethyl)indolin-3-yl)acetic acid; (3R)-2-(3-(5-chloro-2-methoxyphenyl)-2-oxo-6-(trifluoromethyl)indolin-3-yl)acetic acid; (3S)-3-(2-chloro-5-methoxy-4-pyridyl)-3-methyl-6-(trifluoromethyl)indolin-2-one; (3R)-3-(2-chloro-5-methoxy-4-pyridyl)-3-methyl-6-(trifluoromethyl)indolin-2-one; (3S)-3-(3-methoxy-4-pyridyl)-3-methyl-6-(trifluoromethyl)indolin-2-one; (3R)-3-(3-methoxy-4-pyridyl)-3-methyl-6-(trifluoromethyl)indolin-2-one; (3S)-3-methyl-3-(4-pyridyl)-6-(trifluoromethyl)indolin-2-one; (3R)-3-methyl-3-(4-pyridyl)-6-(trifluoromethyl)indolin-2-one; (3S)-3-(2-chloro-4-pyridyl)-3-methyl-6-(trifluoromethyl)indolin-2-one; (3R)-3-(2-chloro-4-pyridyl)-3-methyl-6-(trifluoromethyl)indolin-2-one; (3S)-3-[2-methoxy-5-(1-methylimidazol-2-yl)phenyl]-3-methyl-6-(trifluoromethyl)indolin-2-one; (3R)-3-[2-methoxy-5-(1-methylimidazol-2-yl)phenyl]-3-methyl-6-(trifluoromethyl)indolin-2-one; (3S)-3-(5-chloro-2-methoxy-phenyl)-6-(difluoromethyl)-3-methyl-indolin-2-one; (3R)-3-(5-chloro-2-methoxy-phenyl)-6-(difluoromethyl)-3-methyl-indolin-2-one; (3S)-3-(5-chloro-2-methoxy-3-pyridyl)-3-methyl-6-(trifluoromethyl)indolin-2-one; (3R)-3-(5-chloro-2-methoxy-3-pyridyl)-3-methyl-6-(trifluoromethyl)indolin-2-one; (3S)-6-chloro-3-(5-chloro-2-methoxyphenyl)-3-methylindolin-2-one; (3R)-6-chloro-3-(5-chloro-2-methoxyphenyl)-3-methylindolin-2-one; (3S)-6-chloro-3-(2-chloro-5-methoxypyridin-4-yl)-3-methylindolin-2-one; (3R)-6-chloro-3-(2-chloro-5-methoxypyridin-4-yl)-3-methylindolin-2-one; (3S)-3-(6-chloro-3-methoxypyridin-2-yl)-3-methyl-6-(trifluoromethyl)indolin-2-one; (3R)-3-(6-chloro-3-methoxypyridin-2-yl)-3-methyl-6-(trifluoromethyl)indolin-2-one; (3S)-6-chloro-3-(5-chloro-2-methoxypyridin-3-yl)-3-methylindolin-2-one; (3R)-6-chloro-3-(5-chloro-2-methoxypyridin-3-yl)-3-methylindolin-2-one; (3S)-6-chloro-3-(2-methoxy-5-(1-methyl-1H-imidazol-2-yl)phenyl)-3-methylindolin-2-one; (3R)-6-chloro-3-(2-methoxy-5-(1-methyl-1H-imidazol-2-yl)phenyl)-3-methylindolin-2-one; (3S)-6-chloro-3-(6-chloro-3-methoxypyridin-2-yl)-3-methylindolin-2-one; (3R)-6-chloro-3-(6-chloro-3-methoxypyridin-2-yl)-3-methylindolin-2-one; (3S)-6-chloro-3-(2-methoxy-5-(1-methyl-1H-imidazol-2-yl)pyridin-3-yl)-3-methylindolin-2-one; (3R)-6-chloro-3-(2-methoxy-5-(1-methyl-1H-imidazol-2-yl)pyridin-3-yl)-3-methylindolin-2-one; (3S)-6-chloro-3-(5-methoxy-2-(1-methyl-1H-imidazol-2-yl)pyridin-4-yl)-3-methylindolin-2-one; (3R)-6-chloro-3-(5-methoxy-2-(1-methyl-1H-imidazol-2-yl)pyridin-4-yl)-3-methylindolin-2-one; or a pharmaceutically acceptable salt or solvate of any one of the above-mentioned compounds.

The present invention also provides novel compounds. Accordingly, in the above-discussed third aspect, the invention relates to a compound of the following formula (Ia)

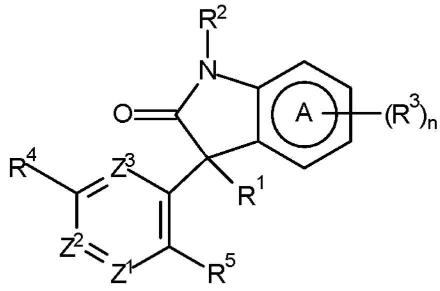

(Ia)

or a pharmaceutically acceptable salt or solvate thereof.

As described above, the compounds of formula (Ia) can be used as active pharmaceutical ingredients, particularly as maxi-K potassium channel openers. The present invention thus also relates to the compound of formula (Ia) or a pharmaceutically acceptable salt or solvate thereof, or a pharmaceutical composition comprising any of the aforementioned entities and a pharmaceutically acceptable excipient, for use in the treatment or prevention of a fragile X associated disorder (such as, e.g., fragile X syndrome (FXS), fragile X-associated tremor/ataxia syndrome (FXTAS), or fragile X-associated primary ovarian insufficiency (FXPOI)) or other diseases/disorders in which maxi-K potassium channels are implicated. In particular, the invention refers to a compound of formula (Ia) or a pharmaceutically acceptable salt or solvate thereof, or a pharmaceutical composition comprising any of the aforementioned entities and a pharmaceutically acceptable excipient, for use in the treatment or prevention of a disease/disorder selected from stroke (e.g., ischemic stroke), ischemia reperfusion injury, ischemic heart disease, hypertension (e.g., partial hypertension or arterial hypertension), myocardial infarction, allergic rhinitis, asthma, chronic obstructive pulmonary disease (COPD), multiple sclerosis, spasticity (e.g., muscle spasticity, or spasticity in a subject/patient suffering from multiple sclerosis), tremor, a muscular disorder, dyskinesia, bladder dysfunction, overactive bladder (OAB), urinary incontinence, irritable bowel syndrome (IBS), faecal incontinence, constipation, gastro-oesophageal reflux disorder (GERD), impaired gastrointenstinal passage, detrusor underactivity, erectile dysfunction, opioid-induced respiratory depression, anxiety, an anxiety disorder (e.g., generalized anxiety disorder (GAD) or social anxiety disorder (SAD)), sensory processing disorder (SPD), autism, an autism spectrum disorder, a social (pragmatic) communication disorder, attention deficit hyperactivity disorder (ADHD), social phobia, intellectual disability, pain (e.g., neuropathic pain), epilepsy, an epilepsy and/or seizure disorder (e.g., Dravet syndrome), generalized epilepsy and paroxysmal dyskinesia (GEPD), temporal lobe epilepsy, psychosis, schizophrenia, negative symptoms of schizophrenia, cognitive impairment in schizophrenia, Phelan-McDermid syndrome, Rett syndrome, Pitt-Hopkins syndrome, 16p11.2 deletion syndrome, Williams-Beuren syndrome, open-angle ocular hypertension, glaucoma, tinnitus, diabetic retinopathy, tocolysis (or prevention of preterm labor), migraine, Liang-Wang syndrome, Smith-Lemli-Opitz syndrome, Angelman syndrome, and Hutchinson-Gilford progeria syndrome.

The groups/variables comprised in formula (Ia), including in particular ring A, $Z^1$, $Z^2$, $Z^3$, $R^1$, $R^2$, $R^3$, $R^4$, $R^5$, $R^{5A}$, $R^6$, $R^{Cyc}$ and n, have the same meanings, including the same preferred meanings, as described and defined herein above in relation with the compound of formula (I), whereby the following further conditions apply to the compounds of formula (Ia):

- n is 1, 2, 3 or 4; and
- if n is 1 or 2, then at least one group $R^3$ is different from halogen.

Moreover, in formula (Ia), if n is 1, 2 or 3, and a group $R^3$ is attached to ring A in 5-position (wherein the numbering of ring atoms is as illustrated below), then it is preferred that said group $R^3$ which is attached in 5-position is not -Br; more preferably, said group $R^3$ which is attached in 5-position is not halogen (i.e., is different from halogen):

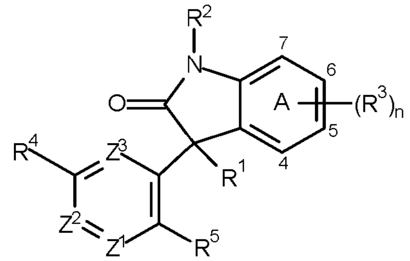

As explained above, ring A, $Z^1$, $Z^2$, $Z^3$, $R^1$, $R^2$, $R^3$, $R^4$, $R^5$, $R^{5A}$ , $R^6$, $R^{CYc}$, and n comprised in the compound of formula (Ia) according to the third aspect of the invention have the same preferred meanings as described and defined herein above in relation to the compound of formula (I) according to the first aspect of the invention.

It is particularly preferred that the compound of formula (Ia) is any one of the corresponding specific compounds described in the examples section of this specification, either in non-salt form or as a pharmaceutically acceptable salt or solvate of the respective compound.

Accordingly, it is particularly preferred that the compound of formula (Ia) is selected from: 3-(5-chloro-2-methoxyphenyl)-3-methyl-6-(trifluoromethyl)indolin-2-one; 3-(5-chloro-2-hydroxy-phenyl)-3-methyl-6-(trifluoromethyl) indolin-2-one; 3-(5-chloro-2-methoxyphenyl)-3-ethyl-6-(trifluoromethyl)indolin-2-one; 3-(5-chloro-2-methoxyphenyl)-3-propyl-6-(trifluoromethyl)indolin-2-one; 2-(3-(5-chloro-2-methoxyphenyl)-2-oxo-6-(trifluoromethyl)indolin-3-yl)acetonitrile; 3-allyl-3-(5-chloro-2-methoxyphenyl)-6-(trifluoromethyl)indolin-2-one; 2-(3-(5-chloro-2-methoxyphenyl)-2-oxo-6-(trifluoromethyl)indolin-3-yl)acetic acid; 3-(2-chloro-5-methoxy-4-pyridyl)-3-methyl-6-(trifluoromethyl)indolin-2-one; 3-(3-methoxy-4-pyridyl)-3-methyl-6-(trifluoromethyl)indolin-2-one; 3-methyl-3-(4-pyridyl)-6-(trifluoromethyl)indolin-2-one; 3-(2-chloro-4-pyridyl)-3-methyl-6-(trifluoromethyl)indolin-2-one; 3-[2-methoxy-5-(1-methylimidazol-2-yl)phenyl]-3-methyl-6-(trifluoromethyl)indolin-2-one; 3-(5-chloro-2-methoxy-phenyl)-6-(difluoromethyl)-3-methyl-indolin-2-one; 3-(5-chloro-2-methoxy-3-pyridyl)-3-methyl-6-(trifluoromethyl)indolin-2-one; 3-(6-chloro-3-methoxypyridin-2-yl)-3-methyl-6-(trifluoromethyl)indolin-2-one; or a pharmaceutically acceptable salt or solvate of any one of the above-mentioned compounds.

Moreover, the invention also relates specifically and individually to each stereoisomer of the above-mentioned compounds, including in particular to each one of these compounds having the S-configuration at the carbon atom carrying $R^1$, or to each one of these compounds having the R-configuration at the carbon atom carrying $R^1$.

Thus, it is particularly preferred that the compound of formula (Ia) is selected from: (3S)-3-(5-chloro-2-methoxy-phenyl)-3-methyl-6-(trifluoromethyl)indolin-2-one; (3R)-3-(5-chloro-2-methoxy-phenyl)-3-methyl-6-(trifluoromethyl)indolin-2-one; (3S)-3-(5-chloro-2-hydroxy-phenyl)-3-methyl-6-(trifluoromethyl)indolin-2-one; (3R)-3-(5-chloro-2-hydroxy-phenyl)-3-methyl-6-(trifluoromethyl)indolin-2-one; (3S)-3-(5-chloro-2-methoxyphenyl)-3-ethyl-6-(trifluoromethyl)indolin-2-one; (3R)-3-(5-chloro-2-methoxyphenyl)-3-ethyl-6-(trifluoromethyl)indolin-2-one; (3S)-3-(5-chloro-2-methoxyphenyl)-3-propyl-6-(trifluoromethyl)indolin-2-one; (3R)-3-(5-chloro-2-methoxyphenyl)-3-propyl-6-(trifluoromethyl)indolin-2-one; (3S)-2-(3-(5-chloro-2-methoxyphenyl)-2-oxo-6-(trifluoromethyl)indolin-3-yl)acetonitrile; (3R)-2-(3-(5-chloro-2-methoxyphenyl)-2-oxo-6-(trifluoromethyl)indolin-3-yl)acetonitrile; (3S)-3-allyl-3-(5-chloro-2-methoxyphenyl)-6-(trifluoromethyl)indolin-2-one; (3R)-3-allyl-3-(5-chloro-2-methoxyphenyl)-6-(trifluoromethyl)indolin-2-one; (3S)-2-(3-(5-chloro-2-methoxyphenyl)-2-oxo-6-(trifluoromethyl)indolin-3-yl)acetic acid; (3R)-2-(3-(5-chloro-2-methoxyphenyl)-2-oxo-6-(trifluoromethyl)indolin-3-yl)acetic acid; (3S)-3-(2-chloro-5-methoxy-4-pyridyl)-3-methyl-6-(trifluoromethyl)indolin-2-one; (3R)-3-(2-chloro-5-methoxy-4-pyridyl)-3-methyl-6-(trifluoromethyl)indolin-2-one; (3S)-3-(3-methoxy-4-pyridyl)-3-methyl-6-(trifluoromethyl)indolin-2-one; (3R)-3-(3-methoxy-4-pyridyl)-3-methyl-6-(trifluoromethyl)indolin-2-one; (3S)-3-methyl-3-(4-pyridyl)-6-(trifluoromethyl)indolin-2-one; (3R)-3-methyl-3-(4-pyridyl)-6-(trifluoromethyl)indolin-2-one; (3S)-3-(2-chloro-4-pyridyl)-3-methyl-6-(trifluoromethyl)indolin-2-one; (3R)-3-(2-chloro-4-pyridyl)-3-methyl-6-(trifluoromethyl)indolin-2-one; (3S)-3-[2-methoxy-5-(1-methylimidazol-2-yl)phenyl]-3-methyl-6-(trifluoromethyl)indolin-2-one; (3R)-3-[2-methoxy-5-(1-methylimidazol-2-yl)phenyl]-3-methyl-6-(trifluoromethyl)indolin-2-one; (3S)-3-(5-chloro-2-methoxy-phenyl)-6-(difluoromethyl)-3-methyl-indolin-2-one; (3R)-3-(5-chloro-2-methoxy-phenyl)-6-(difluoromethyl)-3-methyl-indolin-2-one; (3S)-3-(5-chloro-2-methoxy-3-pyridyl)-3-methyl-6-(trifluoromethyl)indolin-2-one; (3R)-3-(5-chloro-2-methoxy-3-pyridyl)-3-methyl-6-(trifluoromethyl)indolin-2-one; (3S)-3-(6-chloro-3-methoxypyridin-2-yl)-3-methyl-6-(trifluoromethyl)indolin-2-one; (3R)-3-(6-chloro-3-methoxypyridin-2-yl)-3-methyl-6-(trifluoromethyl)indolin-2-one; or a pharmaceutically acceptable salt or solvate of any one of the above-mentioned compounds.

For a person skilled in the field of synthetic chemistry, various ways for the preparation of the compounds of formula (I) or (Ia) and their pharmaceutically acceptable salts or solvates will be readily apparent. For example, the compounds of the invention can be prepared in accordance with, or in analogy to, the synthetic routes described herein below in the examples section. In particular, the compounds of formula (I) or (Ia) can be synthesized in accordance with the general methods described in the following Schemes 1, 2, 7 and 8 (the preparation of reference compounds is described in Schemes 3 to 6).

**Scheme 1:** General method for the preparation of 3-aryl-3-alkyl-indolin-2-ones.

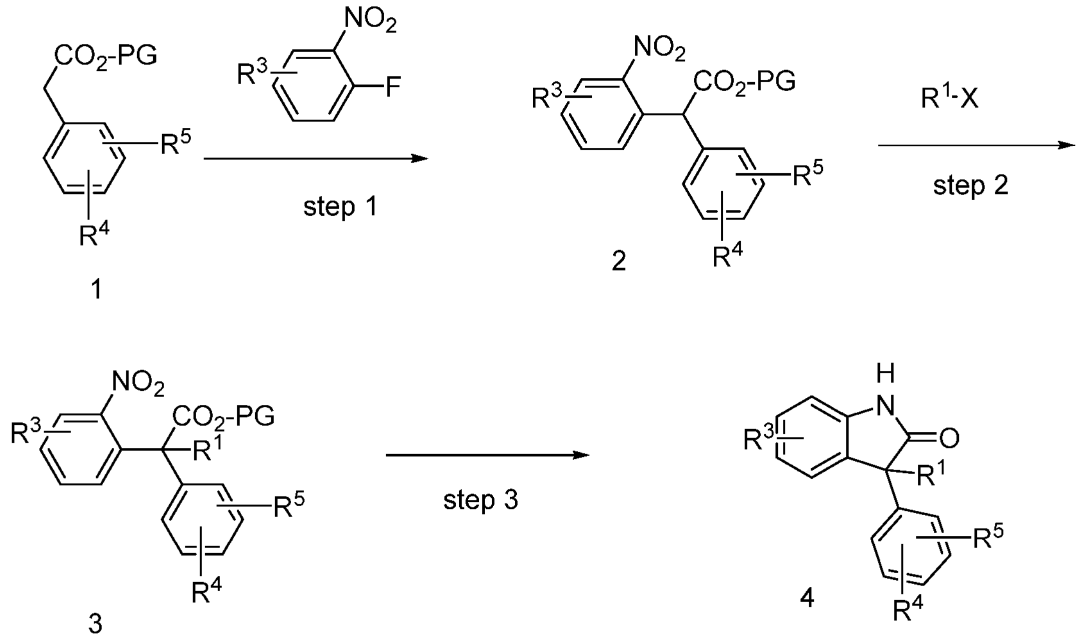


**Step 1:** To a mixture of phenylacetate ester (**1**, 5 mmol, 1 *eq*) and the 1-fluoro-2-nitrobenzene derivative (1.2 *eq*) in an inert solvent such as THF (40 mL) cooled to -40°C is added a base such as LiHMDS (1.3 *eq*). The mixture is warmed to ambient temperature and stirred until the reaction is complete. The reaction is quenched by addition of saturated aqueous ammonium chloride and extracted with ethyl acetate. The organic phase is washed with saturated brine, dried over anhydrous sodium sulfate, filtered and evaporated. Chromatography of the residue over silica gel (eluent petroleum ether / ethyl acetate gradient) gives the product **2.**

**Step 2:** To a stirred solution of **2** (1 mmol, 1 *eq*) and alkyl or aryl halide, $R_1$-X (6 *eq*) in an inert solvent such as DMF (5 mL), cooled on ice, is added a deprotonating agent such as NaH (1.5 *eq*). The mixture is stirred at on ice for 0.5 hr, then warmed to 50 °C and stirred until the reaction is complete. The reaction is cooled on ice, quenched with water and extracted with EtOAc. The organic phase is washed with saturated brine, dried over anhydrous sodium sulfate, filtered and evaporated. Chromatography of the residue over silica gel (eluent petroleum ether / ethyl acetate gradient) gives the product **3.**

**Step 3:** To a solution of **3** (0.5 mmol, 1 *eq*) in mixture of ethanol / water is added a reducing agent such as sodium dithionite (10 *eq*) and the mixture is stirred at 80 °C until the reaction is complete. The reaction mixture is filtered and concentrated under reduced pressure and the residue is purified by chromatography over silica gel (eluent petroleum ether / ethyl acetate gradient) to give the product **4.**

**Scheme 2:** General method for the preparation of 3-heteroaryl-3-alkyl-indolin-2-ones.

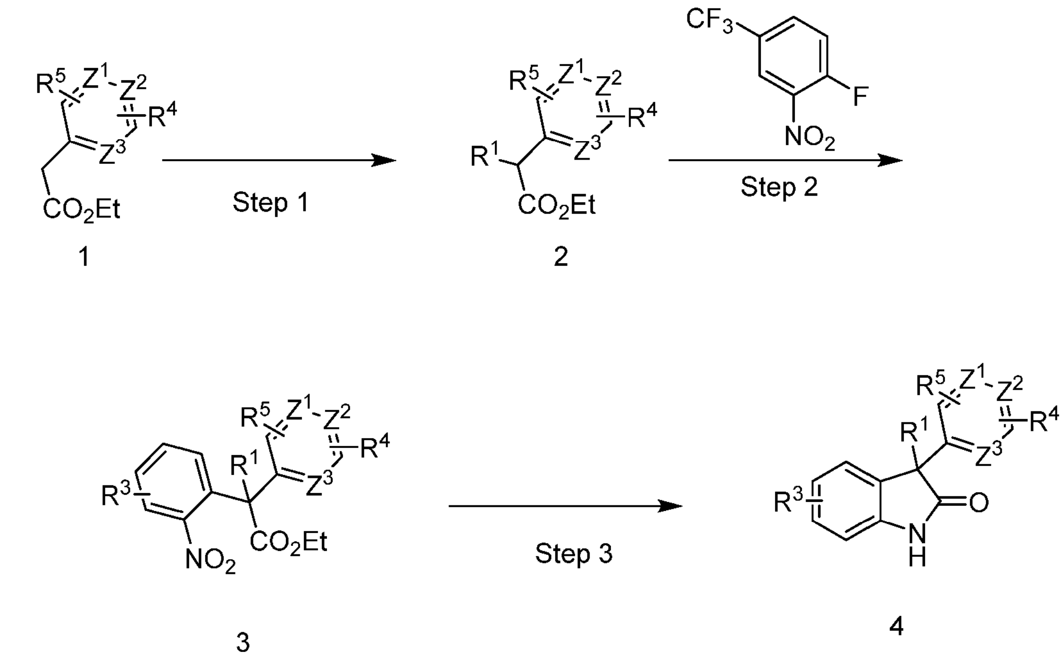


1 2 3 4

**Step 1:** To a solution of ethyl 2-(heteroaryl)acetate **1** (10 mmol, 1 eq) in THF (10 mL) is added dropwise a deprotonating agent such as NaH (1 eq) at -50 °C under $N_2$. The mixture is stirred for 1 h. The alkyl iodide, R'-X, (5 eq) is added slowly and the reaction mixture is warmed to -10 °C, and stirred until the reaction is complete. Water (20 mL) is added at 0°C, and the mixture is extracted with EtOAc. The organic phase is washed saturated brine, dried over $Na_2SO_4$, filtered and concentrated under reduced pressure. The residue is purified by chromatography to give the product **2.**

**Step 2:** To a solution of the ethyl 2-(heteroaryl)acetate 2 (5 mmol, 1 eq) and the 1-fluoro-2-nitrobenzene derivative (1.02 eq) in THF (5 mL) is added a deprotonating agent such as KOtBu (1.5 eq). The mixture is stirred at 25 °C until the reaction is complete. The reaction is quenched by addition saturated $NH_4Cl$ at 0°C and extracted with EtOAc. The organic phase is washed with saturated brine, dried over $Na_2SO_4$, filtered and concentrated under reduced pressure. The residue is purified by chromatography to give product **3.**

**Step 3:** To a solution of **3** (3 mmol, 1 eq) in AcOH (10 mL) is added Fe (10 eq) and the mixture is stirred at 50 °C for until the reaction is complete. The mixture is filtered, concentrated under reduced pressure and the residue is purified by chromatography to give the product **4.**

**Scheme 3:** General method for the preparation of pyrrolopyridin-2-ones.

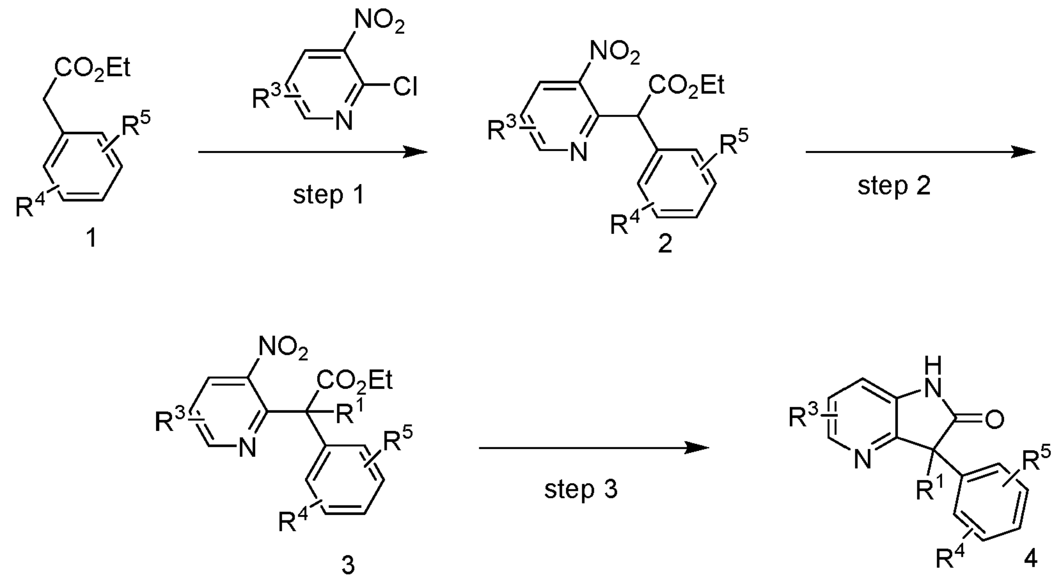


1 2 3 4

**Step 1:** To a mixture of ethyl phenylacetate derivative **1** (5 mmol, 1 eq) and 2-chloro-3-nitropyridine derivative (1.02 eq) in THF (10 mL) is added a deprotonating agent such as t-BuOK (1.5 eq) at 0°C under $N_2$. The mixture is stirred at 25°C until the reaction is complete. The mixture is filtered and concentrated under reduced pressure and the residue is purified by chromatography to give product **2.**

**Step 2:** To a mixture of **2** (1 mmol, 1 eq) and MeI (6 eq) in DMF (5 mL) is added a deprotonating agent such as NaH (1.5 eq) at 0°C under $N_2$. The mixture is stirred at 0 °C for 0.5 h and then heated to 50 °C and stirred until the reaction is complete. The reaction is quenched with water, extracted with EtOAc, dried over $Na_2SO_4$, filtered and concentrated under reduced pressure. The residue is purified by chromatography to give product **3.**

**Step 3:** To a mixture of **3** (1 mmol, 1 eq) in AcOH (5 mL) is added Fe (10 eq) in one portion at 25°C under $N_2$. The mixture is stirred at 60 °C until the reaction is complete. The mixture is filtered and concentrated under reduced pressure. The residue is purified by chromatography to give the product **4.**

**Scheme 4:** General method for the preparation of 2H-spiro[benzofuran-3,3'-indolin]-2'-ones.

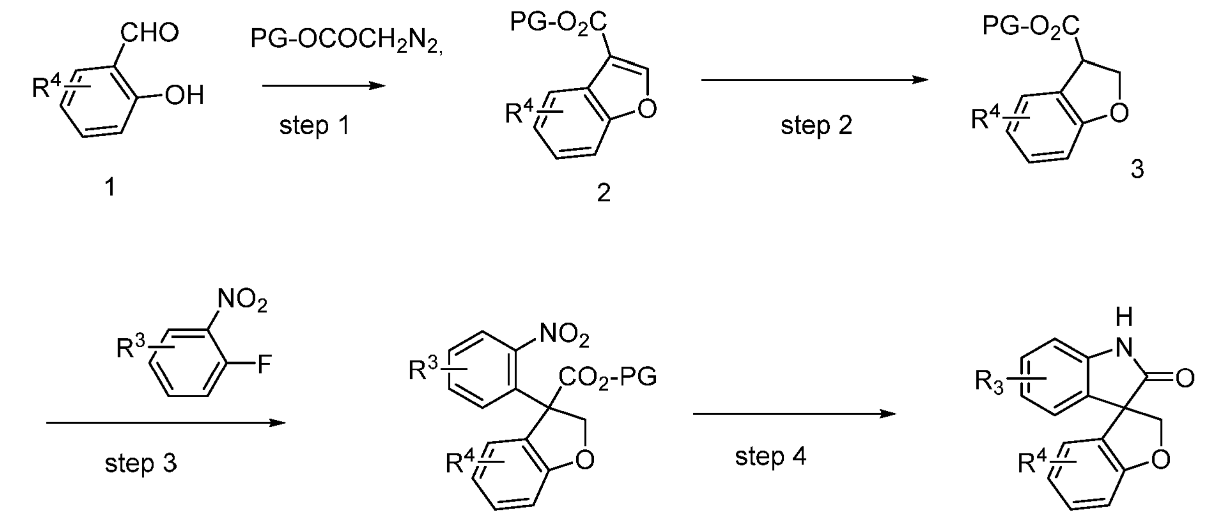


1 2 3 4 5

**Step 1:** To a solution of hydroxybenzaldehyde derivative **1** (50 mmol, 1 *eq*) in DCM (150 mL) is added $BF_3.Et_2O$ (0.1 *eq*) and the mixture is stirred at 15 °C for 0.5 hr. To the mixture is added dropwise a 2-diazoacetate ester (1.7 *eq*) and the mixture is stirred at 15 °C until the reaction is complete. The mixture is concentrated under reduced pressure. The residue is added dropwise to concentrated $H_2SO_4$ and stirred at 15 °C until the reaction is complete. The mixture is added to saturated $NaHCO_3$ solution to adjust the pH to 8 and extracted with EtOAc. The organic phase is washed with saturated brine, dried over $Na_2SO_4$, filtered and evaporated. The residue is purified by chromatography over silica gel (eluent, petroleum ether / ethyl acetate, gradient) to give product **2.**

**Step 2:** To a solution of the protected benzofuran-3-carboxylate **2** (10 mmol, 1 *eq*) in MeOH (50 mL) is added Mg (5 *eq*) and the mixture is stirred at 15°C until the reaction is complete. Aqueous HCl (1 N, 100 mL) is added at 0 °C, and the mixture is extracted with EtOAc. The organic phase is washed with saturated brine, dried over anhydrous $Na_2SO_4$, filtered and concentrated under reduced pressure. The residue is purified by chromatography over silica gel (eluent petroleum ether / ethyl acetate gradient) to give the product **3.**

**Step 3:** To a solution of the protected 2,3-dihydrobenzofuran-3-carboxylate **3** (1 mmol, 1 *eq*) cooled to 0 °C in an inert solvent such as DMF (5 mL) is added a deprotonating agent such as NaH (1.5 *eq*) slowly at 0 °C, and the mixture is stirred at 0 °C for 0.5 hr. To the mixture is added dropwise the 1-fluoro-2-nitrobenzene reagent (1.2 *eq*) at 0 °C and the mixture is stirred at 15 °C until the reaction is complete. The mixture is quenched by addition water (50 mL) at 0°C and extracted with EtOAc. The organic phase is washed with saturated brine, dried over anhydrous $Na_2SO_4$, filtered and concentrated under reduced pressure. The residue is purified by chromatography over silica gel (eluent petroleum ether / ethyl acetate gradient) to give product 4.

**Step 4:** A mixture of 2,3-dihydrobenzofuran-3-carboxylate **4** (1 mmol, 1 eq) and $Na_2S_2O_4$ (10 eq) in EtOH (10 mL) and $H_2O$ (2 mL) is stirred at 80 °C until the reaction is complete. The mixture is filtered and concentrated under reduced pressure. The residue is purified by chromatography over silica gel (eluent petroleum ether / ethyl acetate gradient) to give

product 5.

**Scheme 5:** General method for the preparation of spiro[indane-1,3'-indoline]-2'-ones.

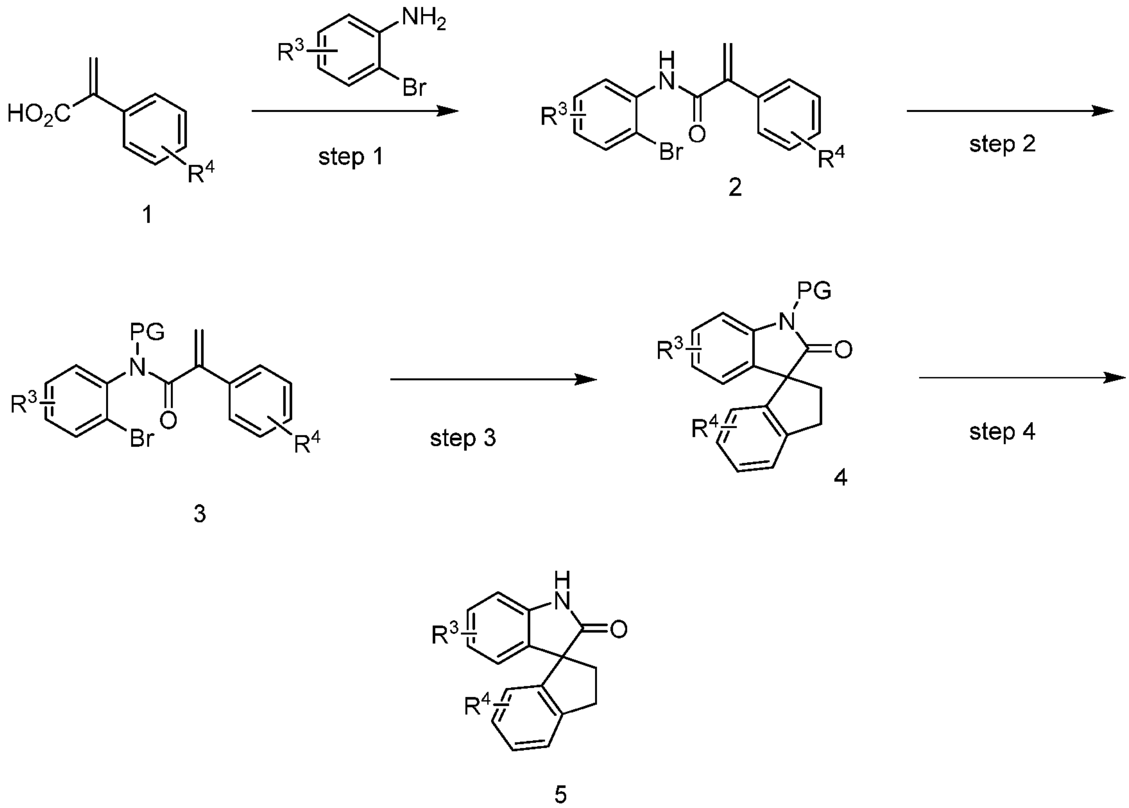


1 2 3 4 5

**Step 1:** To a stirred mixture of arylprop-2-enoic acid 1 (40 mmol, 1 eq) and $(COCl)_2$ (5 eq) in DCM (70 mL) cooled on ice is added DMF (0.3 eq) and the mixture is stirred at 0°C for 1 h. The solvent is evaporated and the residue is dissolved in DCM and added to the 2-bromoaniline derivative (1.2 eq) and TEA (2 eq) at 0 °C. Then the mixture is stirred until the reaction is complete. The mixture is diluted with water and extracted with EtOAc. The organic phase is washed with brine, dried over $Na_2SO_4$, filtered and concentrated under reduced pressure. The residue is purified by chromatography over silica gel (eluent petroleum ether / ethyl acetate gradient) to give product **2.**

**Step 2:** To a stirred solution of **2** (30 mmol, 1 eq) in an inert solvent such as THF (140 mL) is added a deprotonating agent such as NaH (3 eq) at 0 °C. A benzylic halide such a paramethoxybenzyl chloride (2.5 eq) is added and the mixture is warmed to 15°C and stirred until th reaction is complete. The mixture is diluted with water and extracted with EtOAc. The organic phase is washed with brine, dried over $Na_2SO_4$, filtered and concentrated under reduced pressure. The residue is purified by chromatography over silica gel (eluent petroleum ether / ethyl acetate gradient) to give product **3.**

**Step 3:** A mixture of **3** (6 mmol, 1 eq), $K_2CO_3$ (3 eq), $CH_2Br_2$ (8 eq) and a suitable catalyst such as [2-(2-aminophenyl)phenyl]-chloro-palladium;tritert-butylphosphane (0.15 eq) in DMA (50 mL) is degassed and purged with $N_2$. The mixture is then stirred at 130 °C under $N_2$ atmosphere until the reaction is complete. The reaction mixture is diluted with water and extracted times with EtOAc. The organic phase is washed with brine, dried over $Na_2SO_4$, filtered and concentrated under reduced pressure. The residue is purified by chromatography to give product **4.**

**Step 4:** To a solution of **4** (1 mmol, 1 eq) in DCM (5 mL) is added TFA (100 eq) and TfOH (20 eq). The mixture is stirred at 50 °C until the reaction is complete. The mixture is diluted with water and extracted with EtOAc. The organic phase is washed with brine, dried over $Na_2SO_4$, filtered and concentrated under reduced pressure. The residue is purified by chromatography over silica gel (eluent petroleum ether / ethyl acetate gradient) to give product **5.**

**Scheme 6:** General method for the preparation of 3'H-spiro[indoline-3,1'-isobenzofuran]-2,3'-diones.

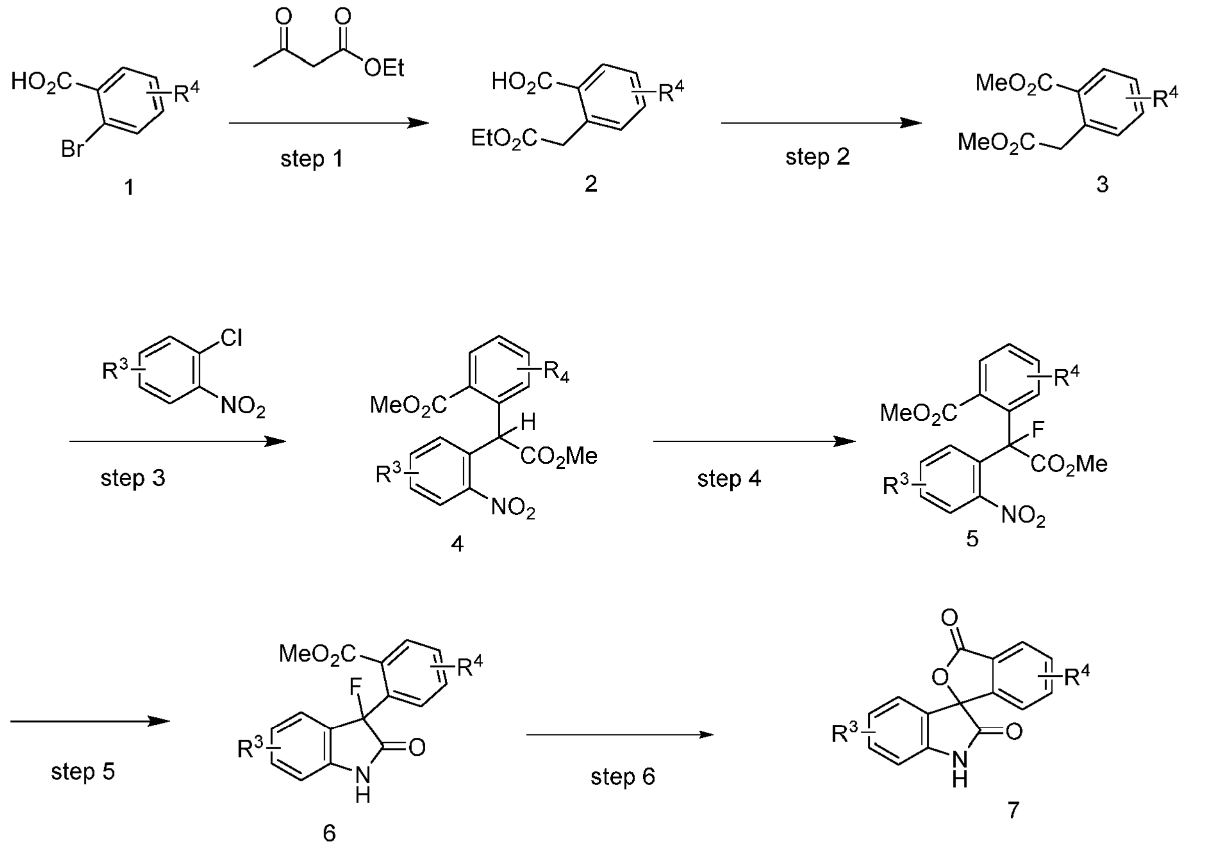


**Step 1:** To a mixture of the 2-bromobenzoic acid derivative **1** (50 mmol, 1 eq) and CuBr (0.056 eq) in ethyl 3-oxobutanoate (13.42 eq) is added NaH (2.4 eq) in one portion at 25°C under $N_2$. The mixture is stirred at 80 °C until the reaction is complete. The mixture is poured into water and washed with EtOAc. The aqueous phase is acidified to pH 1 with concentrated HCl and extracted with EtOAc. The organic phase is washed with brine, dried over $Na_2SO_4$, filtered and concentrated under reduced pressure. Purification of the residue by trituration or chromatography gives product **2.**

**Step 2:** To a solution of benzoic acid derivative **2** (25 mmol, 1 eq) in MeOH (40 mL) is added dropwise $SOCl_2$ (5 eq). The mixture is stirred at 60 °C until the reaction is complete. The reaction mixture is concentrated under reduced pressure and the residue is purified by chromatography to give product **3.**

**Step 3:** To a mixture of diester derivative **3** (10 mmol, 1 eq) and the 1-chloro-2-nitrobenzene reagent (1.02 eq) in THF (30 mL) is added t-BuOK (2 eq) in one portion at 25 °C under $N_2$. The mixture is stirred at 25°C until the reaction is complete. The mixture is diluted with water and extracted with EtOAc. The organic phase is dried over $Na_2SO_4$, filtered and concentrated under reduced pressure. The residue is purified by column to give product **4.**

**Step 4:** To a mixture of ester **4** (8 mmol, 1 eq) in THF (30 mL) is added LiHMDS (1.3 eq) at -78°C under $N_2$. The mixture is stirred at -78 °C for 30 min. NFSI (1.02 eq) is added and the mixture is stirred until the reaction is complete. The mixture is diluted with water, extracted with EtOAc, dried over $Na_2SO_4$, filtered and concentrated under reduced pressure. The residue is purified by chromatography to give product **5.**

**Step 5:** To a mixture of the alphafluoro ester **5** (1 mmol, 1 eq) in AcOH (3 mL) is added Fe (10 eq) in one portion at 25°C under $N_2$. The mixture is stirred at 60 °C until the reaction is complete. The mixture is filtered and concentrated under reduced pressure and the residue is purified by chromatography to give product **6.**

**Step 6:** A solution of the 3-fluoroindolinone **6** (0.5 mmol, 1 *eq*) in AcOH (2 mL) and HCl (1 mL) is stirred at 100 °C for 1.5 hours. The mixture is filtered and concentrated under reduced pressure and the residue is purified by chromatography to give the product 7.

**Scheme 7:** General method for the preparation of 3-heteroaryl-3-alkyl-indolin-2-ones.

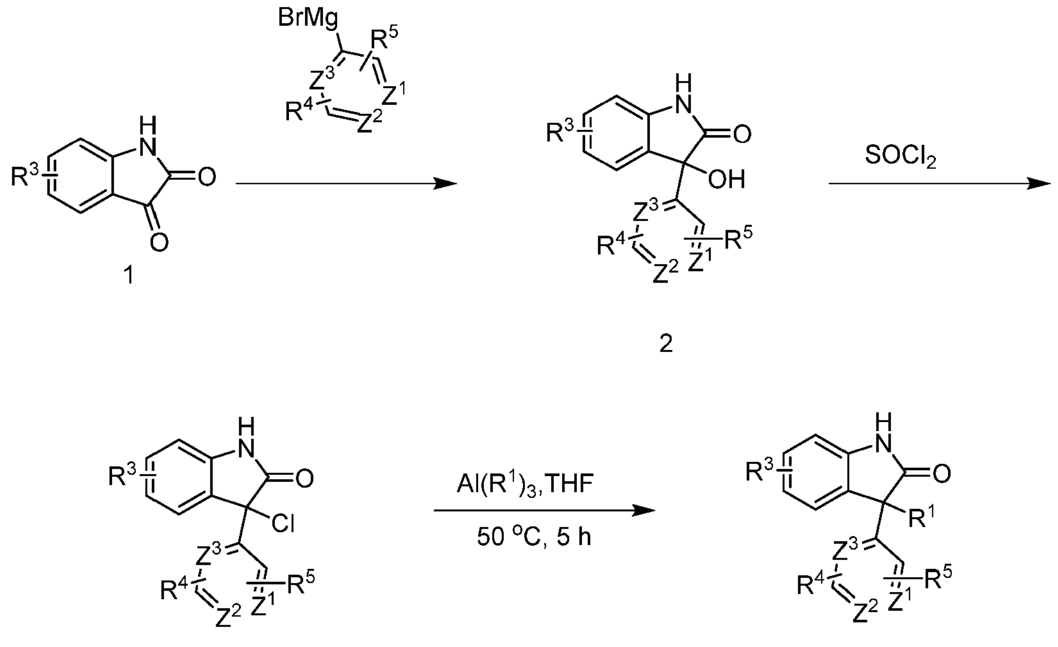


1 2 3 4

**Step 1:** To a solution of indoline-2,3-dione derivative 1 (5 mmol, 1 eq) in THF (10 mL) is added a deprotonating agent such as NaH (1.5 eq) slowly at 0 °C, and the mixture is stirred for 1 h. A solution of the heteroaryl Grignard reagent, prepared under standard conditions, is added under $N_2$ and the mixture is stirred at 15 °C until the reaction is complete. The mixture is diluted with water and extracted with EtOAc. The organic phase is washed with brine, dried over $Na_2SO_4$, filtered and concentrated under reduced pressure. The residue is purified by chromatography over silica gel (eluent petroleum ether / ethyl acetate gradient) to give product **2.**

**Step 2:** To a solution of indolin-2-one **2** (0.5 mmol, 1 eq) in DCM (5 mL) is added $SOCl_2$ (5 eq). The mixture is stirred at 50 °C under $N_2$ until the reaction is complete. The reaction is quenched with water and extracted with EtOAc. The organic phase is washed with brine, dried over $Na_2SO_4$, filtered and concentrated under reduced pressure. The residue is purified by chromatography over silica gel (eluent petroleum ether / ethyl acetate gradient) to give product **3.**

**Step 3:** To a solution of 3-chloro-indolin-2-one **3** (0.5 mmol, 1 eq) in THF cooled to 0 °C is added dropwise a trialkyl aluminum, in an inert solvent (8 eq). The mixture is warmed to 50 °C and stirred for $N_2$ until the reaction is complete. The reaction is quenched with water and extracted with EtOAc. The organic phase is washed with brine (10 mL), dried over $Na_2SO_4$, filtered and concentrated under reduced pressure. The residue is purified by chromatography to give product **4.**

**Scheme 8:** General method for functionalization of 3-alkyl-indolin-2-ones.

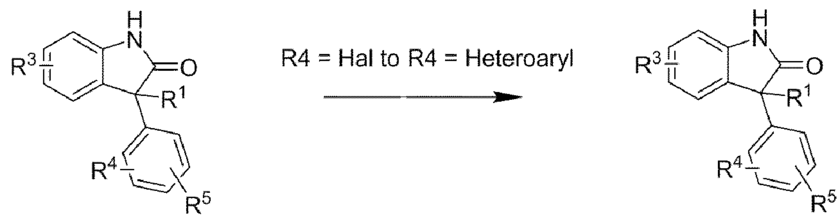


1 2

To a solution of **1** (0.1 mmol, 1 eq) in DMF (1 mL) is added a catalyst such as palladium bis(tri-tert-butylphosphane) (1 eq) and tributylstannyl heteroaryl derivative (1.5 eq). The mixture is stirred at 110 °C under $N_2$ until the reaction is compete. The reaction is quenched with saturated $NH_4Cl$, and extracted with EtOAc. The organic phase is washed with saturated brine, dried over $Na_2SO_4$, filtered and concentrated under reduced pressure. The residue is purified by chromatography to give product **2.**

The following definitions apply throughout the present specification and the claims, unless specifically indicated otherwise.

The term "hydrocarbon group" refers to a group consisting of carbon atoms and hydrogen atoms.

The term "alicyclic" is used in connection with cyclic groups and denotes that the corresponding cyclic group is non-aromatic.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "$C_{1-5}$ alkyl" denotes an alkyl group having 1 to 5 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to $C_{1-4}$ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "$C_{2-5}$ alkenyl" denotes an alkenyl group having 2 to 5 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to $C_{2-4}$ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more (e.g., one or two) carbon-to-carbon double bonds. The term "$C_{2-5}$ alkynyl" denotes an alkynyl group having 2 to 5 carbon atoms. Preferred exemplary alkynyl groups are ethynyl, propynyl (e.g., propargyl), or butynyl. Unless defined otherwise, the term "alkynyl" preferably refers to $C_{2-4}$ alkynyl.

As used herein, the term "alkylene" refers to an alkanediyl group, i.e. a divalent saturated acyclic hydrocarbon group which may be linear or branched. A "$C_{1-5}$ alkylene" denotes an alkylene group having 1 to 5 carbon atoms, and the term "$C_{0-4}$ alkylene" indicates that a covalent bond (corresponding to the option "$C_0$ alkylene") or a $C_{1-4}$ alkylene is present. Preferred exemplary alkylene groups are methylene ($-CH_2-$), ethylene (e.g., $-CH_2-CH_2-$ or $-CH(-CH_3)-$), propylene (e.g., $-CH_2-CH_2-CH_2-$, $-CH(-CH_2-CH_3)-$, $-CH_2-CH(-CH_3)-$, or $-CH(-CH_3)-CH_2-$), or butylene (e.g., $-CH_2-CH_2-CH_2-CH_2-$). Unless defined otherwise, the term "alkylene" preferably refers to $C_{1-4}$ alkylene (including, in particular, linear $C_{1-4}$ alkylene), more preferably to methylene or ethylene, and even more preferably to methylene.

As used herein, the term "alkenylene" refers to an alkenediyl group, i.e. a divalent unsaturated acyclic hydrocarbon group which may be linear or branched and comprises one or more (e.g., one or two) carbon-to-carbon double bonds while it does not comprise any carbon-to-carbon triple bond. A "$C_{2-5}$ alkenylene" denotes an alkenylene group having 2 to 5 carbon atoms. A "$C_{2-3}$ alkenylene" refers to an alkenylene group having 2 or 3 carbon atoms, such as, e.g., $-CH=CH-$, $-CH_2-CH=CH-$ or $-CH=CH-CH_2-$.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). If the aryl is a bridged and/or fused ring system which contains, besides one or more aromatic rings, at least one non-aromatic ring (e.g., a saturated ring or an unsaturated alicyclic ring), then one or more carbon ring atoms in each non-aromatic ring may optionally be oxidized (i.e., to form an oxo group). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "heteroaryl" refers to an aromatic ring group, including monocyclic aromatic rings as well

as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, particularly preferred examples of a "heteroaryl" include pyridinyl (e.g., 2-pyridyl, 3-pyridyl, or 4-pyridyl), imidazolyl, thiazolyl, 1H-tetrazolyl, 2H-tetrazolyl, thienyl (i.e., thiophenyl), or pyrimidinyl.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a $C_{3-11}$ cycloalkyl, and more preferably refers to a $C_{3-7}$ cycloalkyl. A particularly preferred "cycloalkyl" is a monocyclic saturated hydrocarbon ring having 3 to 7 ring members. Moreover, unless defined otherwise, particularly preferred examples of a "cycloalkyl" include cyclohexyl or cyclopropyl, particularly cyclohexyl.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic

ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, particularly preferred examples of a "heterocycloalkyl" include tetrahydropyranyl, piperidinyl, piperazinyl, morpholinyl, pyrrolidinyl, or tetrahydrofuranyl.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a $C_{3-11}$ cycloalkenyl, and more preferably refers to a $C_{3-7}$ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-Cl), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to $-CF_3$, $-CHF_2$, $-CH_2F$, $-CF_2-CH_3$, $-CH_2-CF_3$, $-CH_2-CHF_2$, $-CH_2-CF_2-CH_3$, $-CH_2-CF_2-CF_3$, or $-CH(CF_3)_2$. A particularly preferred "haloalkyl" group is $-CF_3$.

The terms "bond" and "covalent bond" are used herein synonymously, unless explicitly indicated otherwise or contradicted by context.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either

substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

A skilled person will appreciate that the substituent groups comprised in the compounds of the present invention may be attached to the remainder of the respective compound via a number of different positions of the corresponding specific substituent group. Unless defined otherwise, the preferred attachment positions for the various specific substituent groups are as illustrated in the examples.

As used herein, unless explicitly indicated otherwise or contradicted by context, the terms "a", "an" and "the" are used interchangeably with "one or more" and "at least one". Thus, for example, a composition comprising "a" compound of formula (I) can be interpreted as referring to a composition comprising "one or more" compounds of formula (I).

As used herein, the term "about" preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint. If the term "about" is used in connection with the endpoint of an open-ended range, it preferably refers to the corresponding range starting from the lower endpoint -10% or from the upper endpoint +10%, more preferably to the range starting from the lower endpoint -5% or from the upper endpoint +5%, and even more preferably to the open-ended range defined by the exact numerical value of the corresponding endpoint. If the term "about" is used in connection with a parameter that is quantified in integers, such as the number of CGG repeats in the *FMR1* gene or the *FMR2* gene, then the numbers corresponding to ±10% or ±5% of the indicated numerical value are to be rounded to the nearest integer (using the tie-breaking rule "round half up").

It is to be understood that wherever numerical ranges are provided/disclosed herein, all values and subranges encompassed by the respective numerical range are meant to be encompassed within the scope of the invention. Accordingly, the present invention specifically and individually relates to each value that falls within a numerical range disclosed herein, as well as each subrange encompassed by a numerical range disclosed herein.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

The scope of the present invention embraces all pharmaceutically acceptable salt forms of the compounds of formula (I) or (Ia) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogen phosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate),

benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred pharmaceutically acceptable salts of the compounds of formula (I) or (Ia) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (I) or (Ia) is a hydrochloride salt. Accordingly, it is preferred that the compound of formula (I) or (Ia), including any one of the specific compounds of formula (I) or (Ia) described herein, is in the form of a hydrochloride salt, a hydrobromide salt, a mesylate salt, a sulfate salt, a tartrate salt, a fumarate salt, an acetate salt, a citrate salt, or a phosphate salt, and it is particularly preferred that the compound of formula (I) or (Ia) is in the form of a hydrochloride salt.

The present invention also specifically relates to the compound of formula (I) or (Ia), including any one of the specific compounds of formula (I) or (Ia) described herein (including in the examples section), in non-salt form. In accordance with the invention, it is particularly preferred that the compound of formula (I) or (Ia) is employed in non-salt form.

Moreover, the scope of the invention embraces the compounds of formula (I) or (Ia) in any solvated form, including, e.g., solvates with water (i.e., as a hydrate) or solvates with organic solvents such as, e.g., methanol, ethanol, isopropanol, acetic acid, ethyl acetate, ethanolamine, DMSO, or acetonitrile. All physical forms, including any amorphous or crystalline forms (i.e., polymorphs), of the compounds of formula (I) or (Ia) are also encompassed within the scope of the invention. It is to be understood that such solvates and physical forms of pharmaceutically acceptable salts of the compounds of the formula (I) or (Ia) are likewise embraced by the invention.

Furthermore, the compounds of formula (I) or (Ia) may exist in the form of different isomers, in particular stereoisomers (including, e.g., geometric isomers (or cis/trans isomers), atropisomers, enantiomers and diastereomers) or tautomers (including, in particular, prototropic tautomers, such as keto/enol tautomers or thione/thiol tautomers). All such isomers of the compounds of formula (I) or (Ia) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds of formula (I) or (Ia). It will be understood that some compounds may exhibit tautomerism. In such cases, the formulae provided herein expressly depict only one of the possible tautomeric forms. The formulae and chemical names as provided herein are intended to encompass any tautomeric form of the corresponding compound and not to be limited merely to the specific tautomeric form depicted by the drawing or identified by the name of the compound.

In particular, the compounds of formula (I) or (Ia) may have the (S)-configuration or the (R)-configuration at the carbon atom carrying the group $R^1$, as illustrated in the following (see also Examples 2 and 3):

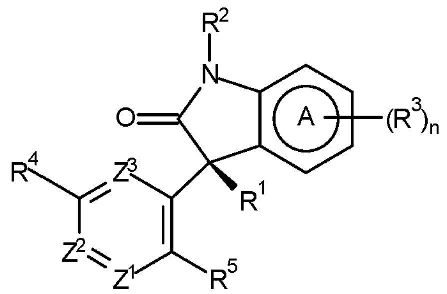

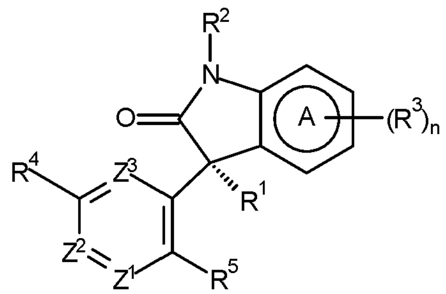

The present invention specifically relates to an optical isomer (or optically active isomer) of the compound of formula (I) or (Ia), wherein the carbon atom carrying $R^1$ is in (S)-configuration, and further specifically relates to an optical isomer (or optically active isomer) of the compound of formula (I) or (Ia), wherein the carbon atom carrying $R^1$ is in (R)-configuration. The invention also relates to any mixtures of such isomers, including a corresponding racemic mixture.

The scope of the invention further embraces compounds of formula (I) or (Ia), in which one or more atoms are replaced by a specific isotope of the corresponding atom. For example, the invention encompasses compounds of formula (I) or (Ia), in which one or more hydrogen atoms (or, e.g., all hydrogen atoms) are replaced by deuterium atoms (i.e., $^2H$; also referred to as "D"). Accordingly, the invention also embraces compounds of formula (I) or (Ia) which are enriched in deuterium. Naturally occurring hydrogen is an isotopic mixture comprising about 99.98 mol-% hydrogen-1 ($^1H$) and about 0.0156 mol-% deuterium ($^2H$ or D). The content of deuterium in one or more hydrogen positions in the compounds of formula (I) or (Ia) can be increased using deuteration techniques known in the art. For example, a compound of formula (I) or (Ia), or a reactant or precursor to be used in the synthesis of the compound of formula (I) or (Ia), can be subjected to an

H/D exchange reaction using, e.g., heavy water ($D_2O$). Further suitable deuteration techniques are described in: Atzrodt J et al., Bioorg Med Chem, 2012, 20(18): 5658-67; William JS et al., Journal of Labelled Compounds and Radiopharmaceuticals, 2010, 53(11-12): 635-44; Modvig A et al., J Org Chem, 2014, 79: 5861-8. The content of deuterium can be determined, e.g., using mass spectrometry or NMR spectroscopy. Unless specifically indicated otherwise, it is preferred that the compound of formula (I) or (Ia) is not enriched in deuterium. Accordingly, the presence of naturally occurring hydrogen atoms or $^{1}H$ hydrogen atoms in the compounds of formula (I) or (Ia) is preferred.

The present invention also embraces compounds of formula (I) or (Ia), in which one or more atoms are replaced by a positron-emitting isotope of the corresponding atom, such as, e.g., $^{18}F$, $^{11}C$, $^{13}N$, $^{15}O$, $^{76}Br$, $^{77}Br$, $^{120}I$ and/or $^{124}I$. Such compounds can be used as tracers, trackers or imaging probes in positron emission tomography (PET). The invention thus includes (i) compounds of formula (I) or (Ia), in which one or more fluorine atoms (or, e.g., all fluorine atoms) are replaced by $^{18}F$ atoms, (ii) compounds of formula (I) or (Ia), in which one or more carbon atoms (or, e.g., all carbon atoms) are replaced by $^{11}C$ atoms, (iii) compounds of formula (I) or (Ia), in which one or more nitrogen atoms (or, e.g., all nitrogen atoms) are replaced by $^{13}N$ atoms, (iv) compounds of formula (I) or (Ia), in which one or more oxygen atoms (or, e.g., all oxygen atoms) are replaced by $^{15}O$ atoms, (v) compounds of formula (I) or (Ia), in which one or more bromine atoms (or, e.g., all bromine atoms) are replaced by $^{76}Br$ atoms, (vi) compounds of formula (I) or (Ia), in which one or more bromine atoms (or, e.g., all bromine atoms) are replaced by $^{77}Br$ atoms, (vii) compounds of formula (I) or (Ia), in which one or more iodine atoms (or, e.g., all iodine atoms) are replaced by $^{120}I$ atoms, and (viii) compounds of formula (I) or (Ia), in which one or more iodine atoms (or, e.g., all iodine atoms) are replaced by $^{124}I$ atoms. In general, it is preferred that none of the atoms in the compounds of formula (I) or (Ia) are replaced by specific isotopes.

In the following, where reference is made to the compounds of the present invention, this is intended to refer to the compounds of formula (I) according to the first or second aspect of the invention (or a pharmaceutically acceptable salt or solvate thereof) as well as the compounds of formula (Ia) according to the third aspect of the invention (or a pharmaceutically acceptable salt or solvate thereof). While the reference to pharmaceutically acceptable salts and solvates is omitted for ease of legibility at some instances, it will be understood that a pharmaceutically acceptable salt or solvate of the compound of formula (I) or (Ia) can likewise be employed in accordance with the present invention.

The compounds of the present invention may be administered as compounds *per se* or may be formulated as medicaments/pharmaceutical compositions. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers.

The pharmaceutical compositions may comprise one or more solubility enhancers, such as, e.g., poly(ethylene glycol), including poly(ethylene glycol) having a molecular weight in the range of about 200 to about 5,000 Da (e.g., PEG 200, PEG 300, PEG 400, or PEG 600), ethylene glycol, propylene glycol, glycerol, a non-ionic surfactant, tyloxapol, polysorbate 80, macrogol-15-hydroxystearate (e.g., Kolliphor® HS 15, CAS 70142-34-6), a phospholipid, lecithin, dimyristoyl phosphatidylcholine, dipalmitoyl phosphatidylcholine, distearoyl phosphatidylcholine, a cyclodextrin, α-cyclodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, a carboxyalkyl thioether, hydroxypropyl methylcellulose, hydroxypropylcellulose, polyvinylpyrrolidone, a vinyl acetate copolymer, vinyl pyrrolidone, sodium lauryl sulfate, dioctyl sodium sulfosuccinate, or any combination thereof.

The pharmaceutical compositions may also comprise one or more preservatives, particularly one or more antimicrobial preservatives, such as, e.g., benzyl alcohol, chlorobutanol, 2-ethoxyethanol, m-cresol, chlorocresol (e.g., 2-chloro-3-methyl-phenol or 4-chloro-3-methylphenol), benzalkonium chloride, benzethonium chloride, benzoic acid (or a pharmaceutically acceptable salt thereof), sorbic acid (or a pharmaceutically acceptable salt thereof), chlorhexidine, thimerosal, or any combination thereof.

The pharmaceutical compositions can be formulated by techniques known to the person skilled in the art, such as the techniques published in "Remington: The Science and Practice of Pharmacy", Pharmaceutical Press, 22nd edition. The pharmaceutical compositions can be formulated as dosage forms for oral, parenteral, such as intramuscular, intravenous, subcutaneous, intradermal, intraarterial, intracardial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds of formula (I) or (Ia), or the above described pharmaceutical compositions comprising a compound of formula (I) or (Ia), may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, or vaginal administration. It is preferred that said compounds or pharmaceutical compositions are administered orally.

If said compounds or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the compounds or pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Preferably, said compounds or pharmaceutical compositions are administered orally, e.g., in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

For oral administration, the compounds or pharmaceutical compositions are preferably administered by oral ingestion, particularly by swallowing. The compounds or pharmaceutical compositions can thus be administered to pass through the mouth into the gastrointestinal tract, which can also be referred to as "oral-gastrointestinal" administration.

Alternatively, said compounds or pharmaceutical compositions can be administered in the form of a suppository or pessary, or may be applied topically in the form of a gel, hydrogel, lotion, solution, cream, ointment or dusting powder. The compounds of the present invention may also be dermally or transdermally administered, for example, by the use of a skin patch.

Said compounds or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides, copolymers of L-glutamic acid and gamma-ethyl-L-glutamate, poly(2-hydroxyethyl methacrylate), ethylene vinyl acetate, or poly-D-(-)-3-hydroxybutyric acid. Sustained-release pharmaceutical compositions also include liposomally entrapped compounds. The present invention thus also relates to liposomes containing a compound of the invention.

Said compounds or pharmaceutical compositions may also be administered by the pulmonary route, the rectal route, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

It is also envisaged to prepare dry powder formulations of the compounds of formula (I) for pulmonary administration, particularly inhalation. Such dry powders may be prepared by spray drying under conditions which result in a substantially amorphous glassy or a substantially crystalline bioactive powder. Accordingly, dry powders of the compounds of the present invention can be made according to an emulsification/spray drying process.

For topical application to the skin, said compounds or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

The present invention thus relates to the compounds or the pharmaceutical compositions provided herein, wherein the corresponding compound or pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route. A preferred route of administration is oral administration. Thus, for each of the compounds of formula (I) or (Ia) or the corresponding pharmaceutical compositions provided herein, it is particularly preferred that the respective compound or pharmaceutical composition is to be administered orally (particularly by oral ingestion).

Typically, a physician will determine the actual dosage which will be most suitable for an individual subject. The specific dose level and frequency of dosage for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

A proposed, yet non-limiting dose of the compounds according to the invention for oral administration to a human (of approximately 70 kg body weight) may be 0.05 to 2000 mg, preferably 0.1 mg to 1000 mg, of the active ingredient per unit dose. The unit dose may be administered, e.g., 1 to 3 times per day. The unit dose may also be administered 1 to 7 times per week, e.g., with not more than one administration per day. It will be appreciated that it may be necessary to make routine variations to the dosage depending on the age and weight of the patient/subject as well as the severity of the condition to be treated. The precise dose and also the route of administration will ultimately be at the discretion of the attendant physician or veterinarian.

The compound of formula (I) or (Ia), or a pharmaceutical composition comprising the compound of formula (I) or (Ia), can be administered in monotherapy (e.g., without concomitantly administering any further therapeutic agents, or without concomitantly administering any further therapeutic agents against the same disease that is to be treated or prevented with the compound of formula (I) or (Ia)). Thus, the present invention relates to the compound of formula (I) or (Ia), or a corresponding pharmaceutical composition, for use in the monotherapeutic treatment or prevention of a fragile X associated disorder (such as fragile X syndrome). In particular, the invention relates to the monotherapeutic administration of the compound of formula (I) or (Ia), or a corresponding pharmaceutical composition, without concomitantly administering any further therapeutic agents against a fragile X associated disorder (or any further therapeutic agents against fragile X syndrome).

However, the compound of formula (I) or (Ia), or a pharmaceutical composition comprising the compound of formula (I) or (Ia), can also be administered in combination with one or more further therapeutic agents. If the compound of formula (I) or (Ia) is used in combination with a second therapeutic agent active against the same disease or condition, the dose of each compound may differ from that when the corresponding compound is used alone, in particular, a lower dose of each compound may be used. The combination of the compound of formula (I) or (Ia) with one or more further therapeutic agents may comprise the simultaneous/concomitant administration of the compound of formula (I) or (Ia) and the further therapeutic agent(s) (either in a single pharmaceutical formulation or in separate pharmaceutical formulations), or the sequential/separate administration of the compound of formula (I) or (Ia) and the further therapeutic agent(s). If administration is sequential, either the compound of formula (I) or (Ia) according to the invention or the one or more further therapeutic agents may be administered first. If administration is simultaneous, the one or more further therapeutic agents may be included in the same pharmaceutical formulation as the compound of formula (I) or (Ia), or they may be administered in two or more different (separate) pharmaceutical formulations. Such different pharmaceutical formulations may be administered via the same route or via different routes of administration.

The subject or patient to be treated in accordance with the present invention may be an animal (e.g., a non-human animal). Preferably, the subject/patient is a mammal. More preferably, the subject/patient is a human (e.g., a male human or a female human) or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig). Most preferably, the subject/patient to be treated in accordance with the invention is a human.

The term "treatment" of a disease or disorder, as used herein, is well known in the art. "Treatment" of a disease or disorder implies that the corresponding disease or disorder is suspected or has been diagnosed in a patient/subject. A patient/subject suspected of suffering from a disease or disorder typically shows specific clinical and/or pathological symptoms which a skilled person can easily attribute to a specific pathological condition (i.e., diagnose a disease or disorder).

The "treatment" of a disease or disorder may, for example, lead to a halt in the progression of the disease or disorder (e.g., no deterioration of symptoms) or a delay in the progression of the disease or disorder (in case the halt in progression is of a transient nature only). The "treatment" of a disease or disorder may also lead to a partial response (e.g.,

amelioration of symptoms) or complete response (e.g., disappearance of symptoms) of the subject/patient suffering from the disease or disorder. Accordingly, the "treatment" of a disease or disorder may also refer to an amelioration of the disease or disorder, which may, e.g., lead to a halt in the progression of the disease or disorder or a delay in the progression of the disease or disorder. Such a partial or complete response may be followed by a relapse. It is to be understood that a subject/patient may experience a broad range of responses to a treatment (such as the exemplary responses as described herein above). The treatment of a disease or disorder may, *inter alia,* comprise curative treatment (preferably leading to a complete response and eventually to healing of the disease or disorder) or palliative treatment (including symptomatic relief).

The term "prevention" of a disease or disorder, as used herein, is also well known in the art. For example, a patient/subject suspected of being prone to suffer from a disease or disorder may particularly benefit from a prevention of the disease or disorder. The subject/patient may have a susceptibility or predisposition for a disease or disorder, including but not limited to hereditary predisposition. Such a predisposition can be determined by standard methods or assays, using, e.g., genetic markers or phenotypic indicators. It is to be understood that a disease or disorder to be prevented in accordance with the present invention has not been diagnosed or cannot be diagnosed in the patient/subject (for example, the patient/subject does not show any clinical or pathological symptoms). Thus, the term "prevention" comprises the use of a compound of the present invention before any clinical and/or pathological symptoms are diagnosed or determined or can be diagnosed or determined by the attending physician.

It is to be understood that the present invention specifically relates to each and every combination of features and embodiments described herein, including any combination of general and/or preferred features/embodiments. In particular, the invention specifically relates to each combination of meanings (including general and/or preferred meanings) for the various groups and variables comprised in formula (I) or (Ia).

The reference in this specification to any prior publication (or information derived therefrom) is not and should not be taken as an acknowledgment or admission or any form of suggestion that the corresponding prior publication (or the information derived therefrom) forms part of the common general knowledge in the technical field to which the present specification relates.

The invention will now be described by reference to the following examples which are merely illustrative and are not to be construed as a limitation of the scope of the present invention, which is as defined in the claims.

EXAMPLES

The compounds of formula (I) or (Ia) described in this section, including in particular the compounds of Examples 1 to 40, are defined by their chemical formulae and their corresponding chemical names. In case of conflict between any chemical formula and the corresponding chemical name indicated herein, the present invention relates to both the compound defined by the chemical formula and the compound defined by the chemical name, and particularly relates to the compound defined by the chemical formula.

**Example 1: 3-(5-chloro-2-methoxyphenyl)-3-methyl-6-(trifluoromethyl)indolin-2-one**

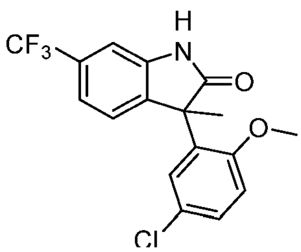

**Synthetic scheme:**

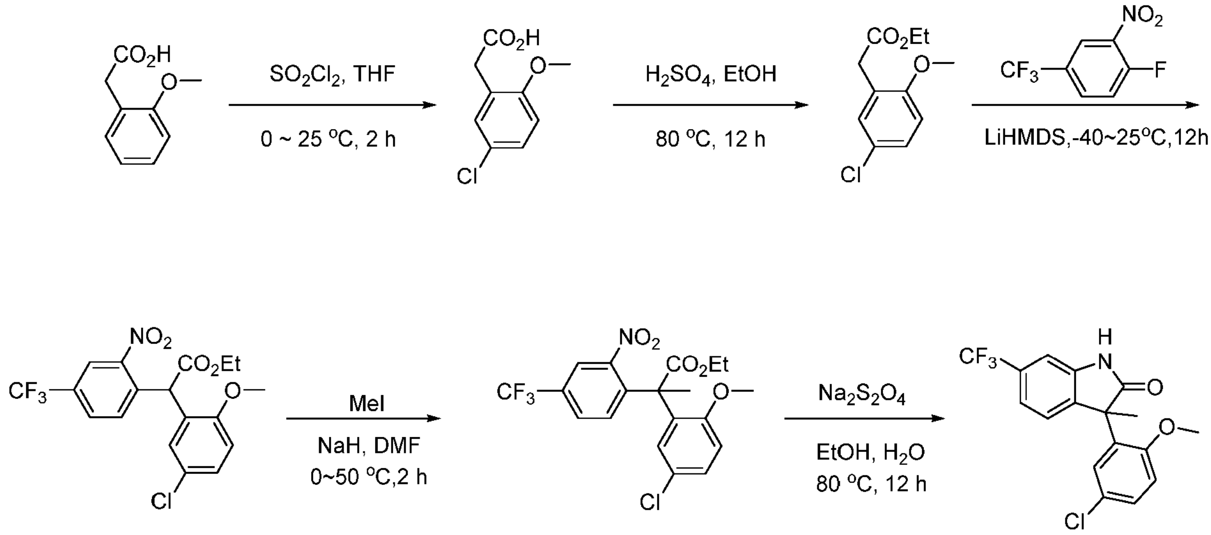


**Step 1.** Preparation of 2-(5-chloro-2-methoxyphenyl)acetic acid.

To a solution of 2-(2-methoxyphenyl)acetic acid (35 g, 210.6 mmol, 1 eq) in THF (400 mL) was added dropwise $SO_2Cl_2$ (42.64 g, 315.9 mmol, 31.59 mL, 1.5 eq) at 0 °C. After addition, the mixture was stirred at 0 °C for 30 min. Then the mixture was warmed to 25 °C and stirred at 25 °C for 1.5 hr. The reaction was quenched by addition of water (800 mL) at 0 °C, and then extracted four times with EtOAc (600 mL). The combined organic layers were washed twice with saturated brine (800 mL), dried over $Na_2SO_4$, filtered and concentrated under reduced pressure to give 2-(5-chloro-2-methoxyphenyl)acetic acid (58 g) as a white solid which was used into next step without further purification. LCMS: 201.0 ($M+H^+$)

**Step 2**. Preparation of ethyl 2-(5-chloro-2-methoxyphenyl)acetate.

To a solution of 2-(5-chloro-2-methoxyphenyl)acetic acid (58 g, 289 mmol, 1 eq) in EtOH (600 mL) was added $H_2SO_4$ (12 M, 3.61 mL, 0.15 eq) and then the mixture was stirred at 80°C for 12 hr. The reaction mixture was concentrated under reduced pressure and the residue was purified by column chromatography ($SiO_2$, petroleum ether / ethyl acetate, 1:0 to 1:0 gradient). Ethyl 2-(5-chloro-2-methoxyphenyl)acetate (24.3 g, 27%) was obtained as a yellow oil. LCMS: 229.0 ($M+H^+$)

**Step 3**. Preparation of ethyl 2-(5-chloro-2-methoxyphenyl)-2-(2-nitro-4-(trifluoromethyl)phenyl) acetate.

To a mixture of ethyl 2-(5-chloro-2-methoxyphenyl)acetate (4 g, 17.5 mmol, 1 eq) and 1-fluoro-2-nitro-4-(trifluoromethyl)benzene (4.39 g, 20.1 mmol, 2.95 mL, 1.2 eq) in THF (40 mL) at -40°C was added LiHMDS (1 M, 22.74 mL, 1.3 eq). The mixture was warmed to 25 °C and stirred at 25 °C for 12 hr. The reaction mixture was quenched by addition of saturated $NH_4Cl$ (100 mL) at 0°C, and then extracted three times with EtOAc (150 mL). The combined organic layers were washed twice with saturated brine (150 mL), dried over $Na_2SO_4$, filtered and concentrated under reduced pressure. The residue was purified by column chromatography ($SiO_2$, petroleum ether / ethyl acetate, 1:0 to 10:1 gradient) to give ethyl 2-(5-chloro-2-methoxyphenyl)-2-(2-nitro-4-(trifluoromethyl)phenyl)acetate (3.2 g, 43.79% yield) as a yellow solid. LCMS: 418.0 ($M+H^+$)

**Step 4**. Preparation of ethyl 2-(5-chloro-2-methoxyphenyl)-2-(2-nitro-4-(trifluoromethyl)phenyl) propanoate.

To a solution of ethyl 2-(5-chloro-2-methoxyphenyl)-2-(2-nitro-4-(trifluoromethyl)phenyl) acetate (400 mg, 0.957 mmol, 1 eq) and MeI (815 mg, 5.74 mmol, 357.6 uL, 6 eq) in DMF (5 mL) was added NaH (60%, 57.45 mg, 1.44 mmol, 1.5 eq) at 0 °C. The mixture was stirred at 0 °C for 0.5 hr, then warmed to 50 °C and stirred at 50 °C for 1.5 hr. The reaction mixture was quenched by addition of water (50 mL) at 0 °C, and then extracted three times with EtOAc (50 mL). The combined organic layers were washed twice with saturated brine (50 mL), dried over $Na_2SO_4$, filtered and concentrated under reduced pressure. The residue was purified by column chromatography ($SiO_2$, petroleum ether / ethyl acetate, 1:0 to 5:1 gradient) to give ethyl 2-(5-chloro-2-methoxyphenyl)-2-(2-nitro-4-(trifluoromethyl)phenyl)propanoate (244 mg, 59%) as a yellow oil.
LCMS: 432.0 ($M+H^+$)

**Step 5**. 3-(5-chloro-2-methoxyphenyl)-3-methyl-6-(trifluoromethyl)indolin-2-one

To a solution of ethyl 2-(5-chloro-2-methoxyphenyl)-2-(2-nitro-4-(trifluoromethyl)phenyl) propanoate (224 mg, 518.8 umol, 1 eq) in EtOH (5 mL) and $H_2O$ (1 mL) was added $Na_2S_2O_4$ (903.22 mg, 5.19 mmol, 1.13 mL, 10 eq) and the mixture was stirred at 80 °C for 2 hr. The reaction mixture was filtered and concentrated under reduced pressure to give a residue which was purified by column chromatography ($SiO_2$, petroleum ether / ethyl acetate, 20:1 to 2:1 gradient) to give 3-(5-chloro-2-methoxyphenyl)-3-methyl-6-(trifluoromethyl)indolin-2-one (81 mg, 43.5%, 99.2% purity) as white solid.

LCMS: 356.0 ($M+H^+$)
$^1H$ NMR (400 MHz, DMSO-$d_6$): 10.737 (1H, s), 7.587 (1H, d), 7.370 (1H, dd), 7.197 (1H, d), 7.086 (1H, s), 7.030 (1H, d), 6.937 (1H, s), 3.397 (3H, s), 1.586 (3H, s).

<u>Examples 2 and 3:</u> **(3S)-3-(5-chloro-2-methoxy-phenyl)-3-methyl-6-(trifluoromethyl)indolin-2-one (Example 2) and (3R)-3-(5-chloro-2-methoxy-phenyl)-3-methyl-6-(trifluoromethyl)indolin-2-one (Example 3)**

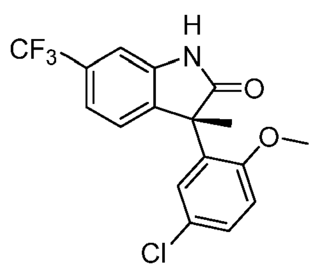

Example 2
(3S)-3-(5-chloro-2-methoxyphenyl)-
3-methyl-6-(trifluoromethyl)indolin-2-one

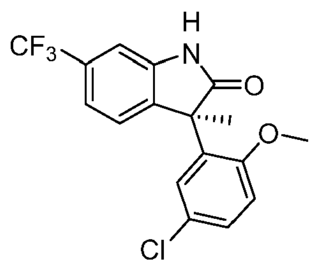

Example 3
(3R)-3-(5-chloro-2-methoxyphenyl)-
3-methyl-6-(trifluoromethyl)indolin-2-one

Separation of 3-(5-chloro-2-methoxyphenyl)-3-methyl-6-(trifluoromethyl)indolin-2-one (110 mg; example 1) by SFC (column: DAICEL CHIRALPAK AD-H (250mm*30mm, 5um); mobile phase, $CO_2$:MeOH (0.1% $NH_3H_2O$) 8:2 isocratic, gave compound **2,** i.e. (3S)-3-(5-chloro-2-methoxyphenyl)-3-methyl-6-(trifluoromethyl)indolin-2-one (34 mg) as a white solid, and compound 3, i.e. (3R)-3-(5-chloro-2-methoxy-phenyl)-3-methyl-6-(trifluoromethyl) indolin-2-one (37 mg) as a white solid.

(3S)-3-(5-chloro-2-methoxy-phenyl)-3-methyl-6-(trifluoromethyl)indolin-2-one LCMS: 356.0 ($M+H^+$)
$^1H$ NMR (400 MHz, DMSO-$d_6$): 10.732, (1H, s), 7.582 (1H, d), 7.368 (1H, dd), 7.192 (1H, dd), 7.080 (1H, s), 7.028 (1H, d), 6.935 (1H, d), 3.394 (3H, s), 1.582 (3H, s)
(3R)-3-(5-chloro-2-methoxy-phenyl)-3-methyl-6-(trifluoromethyl)indolin-2-one LCMS: 356.0 ($M+H^+$)
$^1H$ NMR (400 MHz, DMSO-$d_6$): 10.737 (1H, s), 7.587 (1H, d), 7.367 (1H, dd), 7.197 (1H, d), 7.085 (1H, s), 7.032 (1H, d), 6.937 (1H, s), 3.398 (3H, s), 1.586 (3H, s).

<u>Example 4:</u> **3-(5-chloro-2-hydroxy-phenyl)-3-methyl-6-(trifluoromethyl)indolin-2-one**

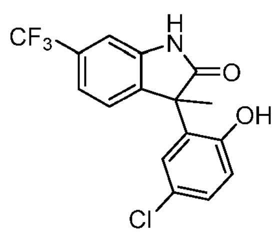

To a mixture of 3-(5-chloro-2-methoxy-phenyl)-3-methyl-6-(trifluoromethyl)indolin-2-one (example 1, 150 mg, 421.66 umol, 1 eq) in DCM (10 mL) was added $BBr_3$ (316.91 mg, 1.26 mmol, 121.89 uL, 3 eq) in one portion at 0°C under

$N_2$, and the mixture was stirred at 25°C for 12h. The reaction mixture was concentrated under reduced pressure. The residue was combined with material from another reaction and purified by prep-HPLC [water (0.1%TFA)-ACN] to give 3-(5-chloro-2-hydroxy-phenyl)-3-methyl-6-(trifluoromethyl)indolin-2-one (74 mg) as a white solid.

LCMS: $(M+H)^+$: 342.0
$^1H$ NMR (400 MHz, DMSO): 7.527 (1H, d), 7.221 (1H, d), 7.156 - 7.123 (2H), 7.015 (1H, d), 6.646 (1H, d), 1.680 (3H, s).

**Example 5: 3-(5-chloro-2-methoxyphenyl)-3-ethyl-6-(trifluoromethyl)indolin-2-one**

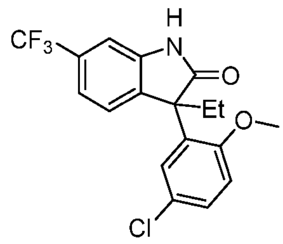

**Step 1.** Preparation of ethyl 2-(5-chloro-2-methoxyphenyl)-2-(2-nitro-4-(trifluoromethyl)phenyl) butanoate.

To a mixture of ethyl 2-(5-chloro-2-methoxyphenyl)-2-(2-nitro-4-(trifluoromethyl)phenyl)acetate (400 mg, 957.48 umol, 1 eq) in DMF (5 mL) was added NaH (60%, 57.45 mg, 1.44 mmol, 1.5 eq) slowly at 0 °C and the mixture was stirred at 0 °C for 0.5 hr. Iodoethane (597.33 mg, 3.83 mmol, 306.32 uL, 4 eq) was added and the mixture was stirred at 45°C for 1 hr under $N_2$ atmosphere. The reaction was quenched by addition of water (100 mL) at 25°C, and then extracted twice with ethyl acetate (50 mL). The combined organic layers were washed twice with saturated brine (40 mL), dried over $Na_2SO_4$, filtered and concentrated under reduced. The residue was purified by column chromatography ($SiO_2$, petroleum ether / ethyl acetate, 1:0 to 10:1 gradient) to give ethyl 2-(5-chloro-2-methoxyphenyl)-2-(2-nitro-4-(trifluoromethyl)phenyl)butanoate (217 mg, 50.8%) as a yellow oil.

**Step 2**. Preparation of 3-(5-chloro-2-methoxyphenyl)-3-ethyl-6-(trifluoromethyl)indolin-2-one.

To a solution of ethyl 2-(5-chloro-2-methoxyphenyl)-2-(2-nitro-4-(trifluoromethyl)phenyl) butanoate (197 mg, 441.89 umol, 1 eq) in EtOH (3 mL) and $H_2O$ (1 mL) was added $Na_2S_2O_4$ (769.36 mg, 4.42 mmol, 961.69 uL, 10 eq) and the mixture was stirred at 80 °C for 12 hr. The reaction mixture was filtered and concentrated under reduced pressure. The residue was purified by column chromatography ($SiO_2$, petroleum ether / ethyl acetate, 10:1 to 3:1 gradient) to give 3-(5-chloro-2-methoxyphenyl)-3-ethyl-6-(trifluoromethyl)indolin-2-one (65 mg, 38%, 96% purity) as a white solid.

LCMS: 370.0 $(M+H^+)$
$^1H$ NMR (400 MHz, DMSO-$d_6$): 10.758 (1H, s), 7.577 (1H, d), 7.354 (1H, dd), 7.202, 1H, dd), 7.062 (1H, s), 7.026 (1H, d), 7.939 (1H, d), 3.406 (3H, s), 2.276 (1H, m), 2.094 (1H, m), 0.578 (3H, t).

**Example 6: 3-(5-chloro-2-methoxyphenyl)-3-propyl-6-(trifluoromethyl)indolin-2-one**

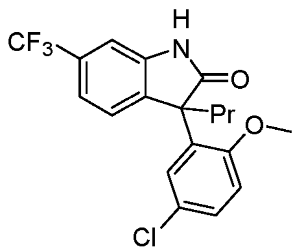

Example 6 was prepared following the procedure described in example 5, using propyl iodide in step 1, to give 3-(5-chloro-2-methoxyphenyl)-3-propyl-6-(trifluoromethyl)indolin-2-one (27 mg, 25.6% yield, 95% purity) as a pale yellow

solid.

LCMS: 384.1 (M+H+)
$^1$H NMR (400 MHz, $CDCl_3$): 8.075 (1H, s), 7.489 (1H, d), 7.197 - 7.128 (2H, m), 7.047, 1H, s), 6.842 (1H, d), 6.646 (1H, d), 3.386 (3H, s), 2.183 (1H, m), 2.053 (1H, m), 1.242 (2H, m), 0.836 (3H, m).

**Example 7: 3-benzyl-3-(5-chloro-2-methoxyphenyl)-6-(trifluoromethyl)indolin-2-one (reference)**

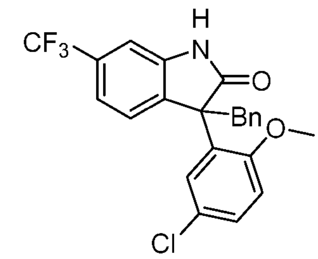

Example 7 was prepared following the procedure described in example 5, using benzyl bromide in step 1, to give 3-benzyl-3-(5-chloro-2-methoxyphenyl)-6-(trifluoromethyl)indolin-2-one (106 mg, 38.7% yield, 98.3% purity) a white solid.

LCMS: 432.1 (M+H+)
$^1$H NMR (400 MHz, $CDCl_3$): 7.678 (1H, d), 7.256 (1H, dd), 7.105, (2H, m), 7.036 (2H, m), 6.744 (4H, m), 3.505 (2H, m), 3.435 (3H, s).

**Example 8: 2-(3-(5-chloro-2-methoxyphenyl)-2-oxo-6-(trifluoromethyl)indolin-3-yl) acetonitrile**

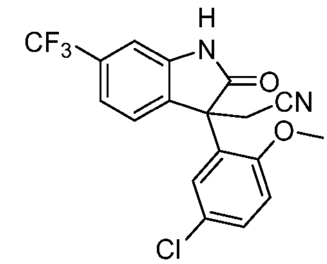

Example 8 was prepared following the procedure described in example 5, using bromoacetonitrile in step 1, to give 2-(3-(5-chloro-2-methoxyphenyl)-2-oxo-6-(trifluoromethyl)indolin-3-yl)acetonitrile (127 mg, 33.8% yield, 93% purity) as a white solid.

LCMS: 398.0 $(M+NH_4)^+$
$^1$H NMR (400 MHz, DMSO-d6): 11.082 (1H, s), 7.612 (1H, d), 7.425 (1H, dd), 7.285 (1H, d), 7.181 - 7.134 (2H, m), 6.998 (1H, d), 3.723 (1H, d), 3.509 (1H, d), 3.330 (3H, s).

**Example 9: 3-allyl-3-(5-chloro-2-methoxyphenyl)-6-(trifluoromethyl)indolin-2-one**

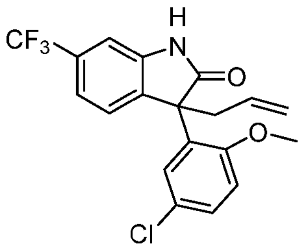

Example 9 was prepared following the procedure described in example 5, using allyl bromide in step 1, to give 3-allyl-3-(5-chloro-2-methoxyphenyl)-6-(trifluoromethyl)indolin-2-one (0.6 g, 28.7% yield, 95.8% purity) as a white solid.

LCMS: 382.0 $(M+H)^+$
$^1H$ NMR (400 MHz, DMSO-$d_6$): 10.737 (1H, s), 7.616 (1H, d), 7.366 (1H, d), 7.206 (1H, d), 7.074 (1H, d), 7.030 (1H, s), 6.944 (1H, d), 5.298 (1H, m), 5.009 - 4.918 (2H, m), 3.403 (3H, s), 3.072 (1H, m), 2.807 (1H, m).

**Example 10: 2-(3-(5-chloro-2-methoxyphenyl)-2-oxo-6-(trifluoromethyl)indolin-3-yl)acetic acid**

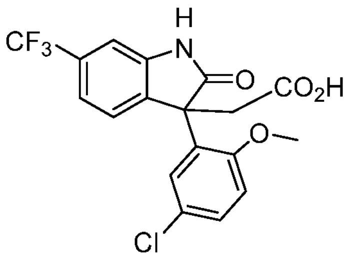

To a solution of 3-allyl-3-(5-chloro-2-methoxyphenyl)-6-(trifluoromethyl)indolin-2-one (example 9, 600 mg, 1.57 mmol, 1 *eq*) in ACN (10 mL) and $H_2O$ (5 mL) was added $NaIO_4$ (1.34 g, 6.29 mmol, 348.34 uL, 4 eq), 4-methyl-4-oxido-morpholin-4-ium (202.52 mg, 1.73 mmol, 182.45 uL, *1.1* eq) and $OsO_4$ (799.10 mg, 3.14 mmol, 163.08 uL, 2 eq). The mixture was degassed and purged with $N_2$ three times, then stirred at 14°C for 12 hr under $N_2$ atmosphere. The reaction mixture was filtered and concentrated under reduced pressure and the residue was purified by prep-TLC ($SiO_2$, DCM: MeOH, 10:1) followed by prep-HPLC (column: Luna C18 100*30 5u; mobile phase: [water (0.1%TFA)-ACN]; B%: 40%-52%, 14min) to give 2-(3-(5-chloro-2-methoxyphenyl)-2-oxo-6-(trifluoromethyl)indolin-3-yl)acetic acid (11 mg, 1.7% yield, 95% purity) as a white solid.

LCMS: 400.0 $(M+H)^+$
$^1H$ NMR (400 MHz, DMSO-$d_6$): 12.241 (1H, br s), 10.829 (1H, s), 7.463 (1H, s), 7.331 (2H, m), 7.211 (1H, d), 7.029 (1H, s), 6.977 (1H, d), 3.558 (1H, d), 3.505 (3H, s), 3.007 (1 H, d).

**Example 11: 5-chloro-6'-(trifluoromethyl)-2H-spiro[benzofuran-3,3'-indolin]-2'-one (reference)**

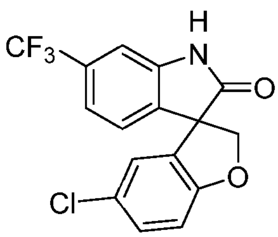

**Synthetic scheme:**

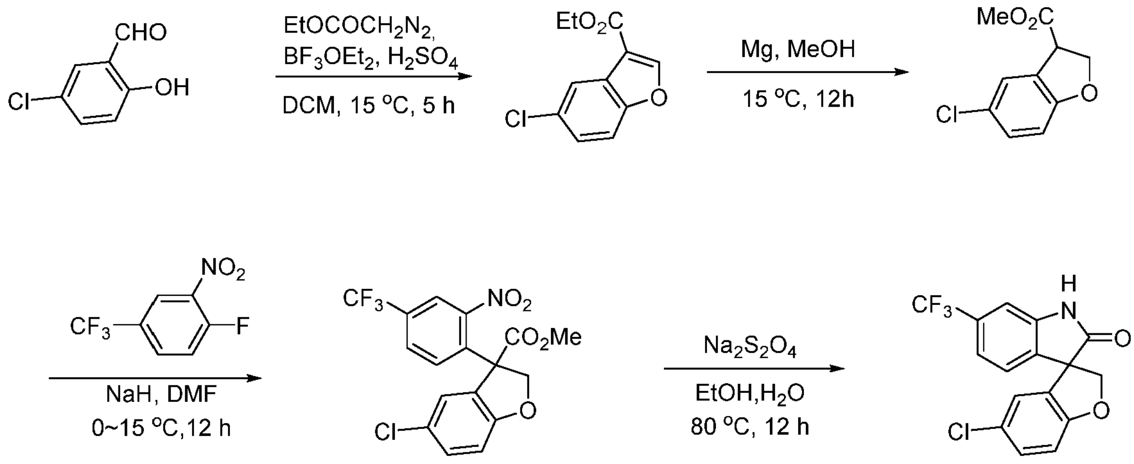


**Step 1:** Preparation of ethyl 5-chlorobenzofuran-3-carboxylate.

To a solution of 5-chloro-2-hydroxybenzaldehyde (10 g, 63.87 mmol, 1 *eq*) in DCM (150 mL) was added $BF_3.Et_2O$ (906.52 mg, 6.39 mmol, 788.28 uL, 0.1 *eq*) and the mixture was stirred at 15 °C for 0.5 hr. To the mixture was then added dropwise ethyl 2-diazoacetate (12.39 g, 108.58 mmol, 1.7 *eq*). After addition, the mixture was stirred at 15 °C for 2 hr. The reaction mixture was concentrated under reduced pressure to give a brown oil which was added dropwise to $H_2SO_4$ (12 M, 5 mL). After addition, the mixture was stirred at 15 °C for 2.5 hr. The mixture was added saturated $NaHCO_3$ solution until pH = 8, then the mixture was extracted three times with EtOAc (250 mL). The combined organic layers were washed twice with saturated brine (750 mL), dried over $Na_2SO_4$, filtered and concentrated under reduced pressure. The residue was purified by column chromatography ($SiO_2$, petroleum ether / ethyl acetate, 0:1 to 10:1 gradient) to give ethyl 5-chlorobenzofuran-3-carboxylate (10 g, 69.70%) was as a white solid.
LCMS: $(M+H^+)$: 225.0

**Step 2:** Preparation of methyl 5-chloro-2,3-dihydrobenzofuran-3-carboxylate.

To a solution of ethyl 5-chlorobenzofuran-3-carboxylate (2 g, 8.90 mmol, 1 *eq*) in MeOH (50 mL) was added Mg (1.08 g, 44.52 mmol, 5 eq) and the mixture was stirred at 15°C for 12hr. To the reaction mixture was added HCl (1 N, 100 mL) at 0 °C, and the mixture was extracted twice with EtOAc (100 mL). The combined organic layers were washed with saturated brine (200 mL), dried over $Na_2SO_4$, filtered and concentrated under reduced pressure. The residue was purified by column chromatography ($SiO_2$, petroleum ether / ethyl acetate, 0:1 to 10:1 gradient) to give methyl 5-chloro-2,3-dihydrobenzofuran-3-carboxylate (1.3 g, 68.67% yield) as an orange oil.

$^1H$ NMR (400 MHz, DMSO-$d_6$): 7.340 (1H, d), 7.137 (1H, dd), 6.734 (1H, d), 4.949 (1H, m), 4.688 (1H, m), 4.323 (1H, m), 3.799 (3H, s).

**Step 3:** Preparation of methyl 5-chloro-3-(2-nitro-4-(trifluoromethyl)phenyl)-2,3-dihydrobenzofuran-3-carboxylate.

To a solution of methyl 5-chloro-2,3-dihydrobenzofuran-3-carboxylate (200 mg, 940.61 umol, 1 *eq)* in DMF (5 mL) was added NaH (56.44 mg, 1.41 mmol, 60% purity, 1.5 *eq*) slowly at 0 °C, then the mixture was stirred at 0 °C for 0.5 hr. To the mixture was added dropwise 1-fluoro-2-nitro-4-(trifluoromethyl)benzene (236.01 mg, 1.13 mmol, 158.40 uL, 1.2 *eq*) at 0 °C and the mixture was stirred at 15 °C for 11.5 hr. The reaction mixture was quenched by addition water (50 mL) at 0°C, and the mixture was extracted twice with EtOAc (50 mL). The combined organic layers were washed with saturated brine (100 mL), dried over $Na_2SO_4$, filtered and concentrated under reduced pressure. The residue was combined with another batch and purified by column chromatography ($SiO_2$, petroleum ether / ethyl acetate 0:1 to 20:1 gradient) to give 5-chloro-3-(2-nitro-4-(trifluoromethyl)phenyl)-2,3- dihydrobenzofuran-3-carboxylate as a yellow solid.

**Step 4:** Preparation of 5-chloro-6'-(trifluoromethyl)-2H-spiro[benzofuran-3,3'-indolin]-2'-one.

A mixture of 5-chloro-3-(2-nitro-4-(trifluoromethyl)phenyl)-2,3-dihydrobenzofuran-3-carboxylate (380 mg, 945.93 umol, 1 eq) and $Na_2S_2O_4$ (1.65 g, 9.46 mmol, 2.06 mL, 10 eq) in EtOH (10 mL) and $H_2O$ (2 mL) was stirred at 80 °C for 12hr. The reaction mixture was filtered and concentrated under reduced pressure and the residue was purified by column chromatography ($SiO_2$, petroleum ether / ethyl acetate, 10:1 to 3:1 gradient) to give 5-chloro-6'-(trifluoromethyl)-2H-spiro[benzofuran-3,3'-indolin]-2'-one (67 mg, 20.4% yield, 98% purity) as a white solid.

LCMS: 357.0 ($M+NH_4^+$)
$^1$H NMR (400 MHz, DMSO-$d_6$): 10.974 (1H, s), 7.383 (2H, m), 7.282 (1H, m), 7.164 (1H, s), 7.033 (1H, d), 6.917 (1H, d), 4.879 (1H, d), 4.803 (1H, d)

**Example 12: 7-methoxy-6'-(trifluoromethyl)-2,3-dihydrospiro[indene-1,3'-indolin]-2'-one (reference)**

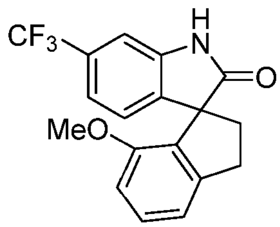

Synthetic scheme:

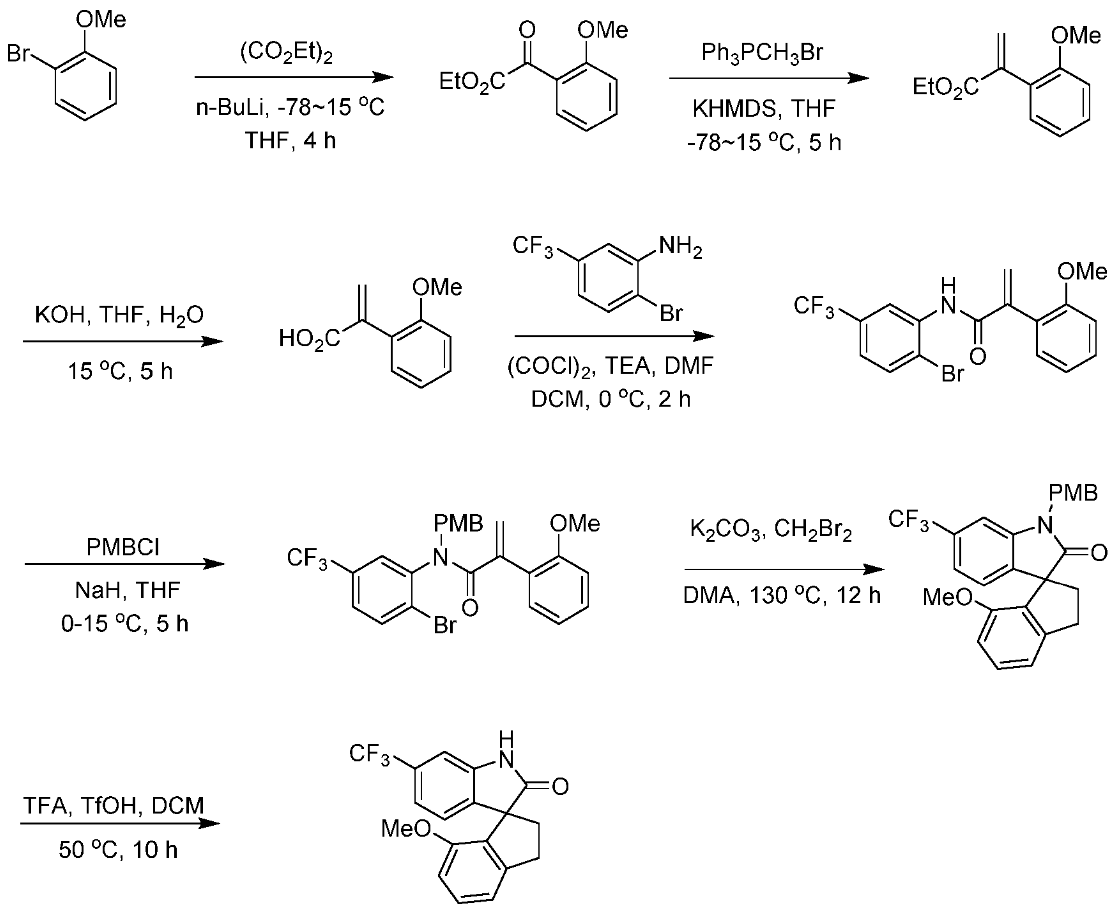


**Step 1:** Preparation of ethyl 2-(2-methoxyphenyl)-2-oxoacetate.

A solution of 1-bromo-2-methoxy-benzene (50 g, 267.33 mmol, 33.33 mL, 1 eq) in THF (500 mL) was cooled to -78 °C, and n-BuLi (2.5 M, 106.93 mL, 1 eq) was added by syringe, and stirred for 1 h. Diethyl oxalate (155.15 g, 1.06 mol, 145.00 mL, 3.97 eq) was added rapidly with stirring and the mixture was stirred at 15 °C for 4 h under $N_2$. The reaction mixture was diluted with water (100 mL) and extracted four times with EtOAc (50 mL). The combined organic layers were washed with brine (90 mL), dried over $Na_2SO_4$, filtered and concentrated under reduced pressure. The residue was

purified by column chromatography ($SiO_2$, petroleum ether / ethyl acetate, 20:1 to 10:1 gradient) to give ethyl 2-(2-methoxyphenyl)-2-oxoacetate (45 g, 80.8% yield) as a light yellow liquid.

**Step 2:** Preparation of ethyl 2-(2-methoxyphenyl)prop-2-enoate.

To a solution of ethyl 2-(2-methoxyphenyl)-2-oxoacetate (45 g, 216.13 mmol, 1 eq) and methyltriphenylphosphonium bromide (92.08 g, 259.35 mmol, 1.2 eq) in THF (400 mL) was added KHMDS (1M, 259.35 mL, 1.2 eq) at 0 °C. The mixture was stirred at 15 °C for 5 hr under $N_2$. The reaction mixture was diluted with water (15 mL) and extracted four times with EtOAc (10 mL). The combined organic layers were washed with brine (20 mL), dried over $Na_2SO_4$, filtered and concentrated under reduced pressure. The residue was purified by column chromatography ($SiO_2$, petroleum ether / ethyl acetate, 20:1 to 5:1 gradient) to give ethyl 2-(2-methoxyphenyl)prop-2-enoate (18 g, 87.28 mmol, 40.38% yield) as a white oil.

**Step 3:** Preparation of 2-(2-methoxyphenyl)prop-2-enoic acid.

To a solution of ethyl 2-(2-methoxyphenyl)prop-2-enoate (18 g, 87.28 mmol, 1 eq) in THF (200 mL) and $H_2O$ (50 mL) was added KOH (58.76 g, 1.05 mol, 12 eq). The mixture was stirred at 15 °C for 15 hr. HCl (2 N) was added to adjust to pH 3 and the mixture was concentrated under reduced pressure to a volume of 45 mL. The solid precipitate was collected by filtration to give 2-(2-methoxyphenyl)prop-2-enoic acid (14 g, 90% yield) as a white solid.

**Step 4:** N-[2-bromo-5-(trifluoromethyl)phenyl]-2-(2-methoxyphenyl)prop-2-enamide.

To a mixture of 2-(2-methoxyphenyl)prop-2-enoic acid (7 g, 39.29 mmol, 1 eq) and $(COCl)_2$ (24.93 g, 196.43 mmol, 17.19 mL, 5 eq) in DCM (70 mL) was added DMF (861.41 mg, 11.79 mmol, 906.74 uL, 0.3 eq) and the mixture was stirred at 0°C for 1 h. The solvent was removed and the residue was taken up in DCM (70 mL) and added to a mixture of 2-bromo-5-(trifluoromethyl)aniline (11.32 g, 47.14 mmol, 6.74 mL, 1.2 eq) and TEA (7.95 g, 78.57 mmol, 10.94 mL, 2 eq) at 0 °C. Then the mixture was stirred for 1 h. The reaction mixture was diluted with water (20 mL) and extracted four times with EtOAc (10 mL). The combined organic layers were washed with brine (35 mL), dried over $Na_2SO_4$, filtered and concentrated under reduced pressure. The residue was purified by column chromatography ($SiO_2$, petroleum ether / ethyl acetate, 30:1 to 10:1 gradient) to give N-[2-bromo-5-(trifluoromethyl)phenyl]-2-(2-methoxyphenyl)prop-2-enamide (12 g,) as a light yellow oil.

**Step 5:** Preparation of N-[2-bromo-5-(trifluoromethyl)phenyl]-2-(2-methoxyphenyl)-N-[(4-methoxyphenyl)methyl] prop-2-enamide.

To a stirred solution of N-[2-bromo-5-(trifluoromethyl)phenyl]-2-(2-methoxyphenyl)prop-2-enamide (11.95 g, 29.86 mmol, 1 eq) in THF (140 mL) was added NaH (60%, 3.58 g, 89.58 mmol, 3 eq) at 0 °C. PMB-Cl (11.69 g, 74.65 mmol, 10.17 mL, 2.5 eq) was then added and the mixture was warmed to 15°C and stirred for 5 hr under $N_2$. The reaction mixture was diluted with water (20 mL) and extracted four times with EtOAc (15 mL). The combined organic layers were washed with brine (30 mL), dried over $Na_2SO_4$, filtered and concentrated under reduced pressure. The residue was purified by column chromatography ($SiO_2$, petroleum ether / ethyl acetate, 20:1 to 5:1 gradient) to give N-[2-bromo-5-(trifluoromethyl) phenyl]-2-(2-methoxyphenyl)-N-[(4-methoxyphenyl)methyl]prop-2-enamide (7 g) as a white solid.

**Step 6:** Preparation of 7-methoxy-1'-[(4-methoxyphenyl)methyl]-6'-(trifluoromethyl)spiro[indane-1,3'-indoline]-2'-one

A mixture of N-[2-bromo-5-(trifluoromethyl)phenyl]-2-(2-methoxyphenyl)-N-[(4-methoxyphenyl)methyl]prop-2-enamide (3 g, 5.77 mmol, 1 eq), $K_2CO_3$ (2.39 g, 17.30 mmol, 3 eq), [2-(2-aminophenyl)phenyl]-chloro-palladium;tritert-butylphosphane (443.14 mg, 864.82 umol, 0.15 eq), $CH_2Br_2$ (7.93 g, 46.12 mmol, 8 eq) in DMA (50 mL) was degassed and purged with $N_2$ five times. The mixture was then stirred at 130 °C for 12 hr under $N_2$ atmosphere. The reaction mixture was diluted with water (20 mL) and extracted four times with EtOAc (8 mL). The combined organic layers were washed with brine (25 mL), dried over $Na_2SO_4$, filtered and concentrated under reduced pressure. The residue was purified by prep-HPLC (column: Xtimate C18 10μ 250mm * 50mm; mobile phase: [water(10mM $NH_4HCO_3$)-ACN]; B%: 60%-70%, 20min) to give 7-methoxy-1'-[(4-methoxyphenyl)methyl]-6'-(trifluoromethyl)spiro[indane-1,3'-indoline]-2'-one (1.3 g, 2.87 mmol, 49.7% yield) as a white solid.

**Step 7:** Preparation of 7-methoxy-6'-(trifluoromethyl)spiro[indane-1,3'-indoline]-2'-one.

To a solution of 7-methoxy-1'-[(4-methoxyphenyl)methyl]-6'-(trifluoromethyl)spiro[indane-1,3'-indoline]-2'-one

(500 mg, 1.10 mmol, 1 eq) in DCM (5 mL) was added TFA (12.57 g, 110.27 mmol, 8.16 mL, 100 eq) and TfOH (3.31 g, 22.05 mmol, 1.95 mL, 20 eq). The mixture was stirred at 50 °C for 10 hr. The reaction mixture was diluted with water (5 mL) and extracted four times with EtOAc (5 mL). The combined organic layers were washed with brine (10 mL), dried over $Na_2SO_4$, filtered and concentrated under reduced pressure. The residue was purified by column chromatography ($SiO_2$, petroleum ether / ethyl acetate, 20:1 to 5/1 gradient) to give 7-methoxy-6'-(trifluoromethyl)spiro[indane-1,3'-indoline]-2'-one (150 mg, 40.8% yield) as a white solid.

LCMS: 334.0 $(M+H)^+$
$^1$H NMR (400 MHz, $CDCl_3$): 7.635 (1H, s), 7.269 - 7.218 (2H, m), 7.150 (1H, s), 7.044 (1H, d), 6.980 (1H, d), 6.646 (1H, d), 3.559 (3H, s), 3.304 (2H, m), 2.768 (1H, m), 2.349 (1H, m).

**<u>Examples 13 and 14:</u> 4-chloro-7-methoxy-6'-(trifluoromethyl)spiro[indane-1,3'-indoline]-2'-one (Example 13; reference) and 4,6-dichloro-7-methoxy-6'-(trifluoromethyl)spiro[indane-1,3'-indoline]-2'-one (Example 14; reference)**

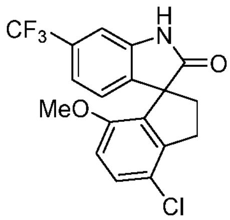

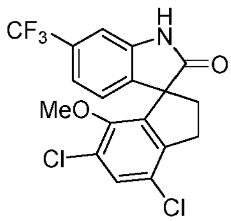

To a solution of 7-methoxy-6'-(trifluoromethyl)spiro[indane-1,3'-indoline]-2'-one (example 12, 190 mg, 570.05 umol, 1 eq) in AcOH (5 mL) was added NCS (114.18 mg, 855.08 umol, 1.5 eq). The mixture was stirred at 50 °C for 5 h. The reaction mixture was diluted with water (15 mL) and extracted four times with EtOAc (10 mL). The combined organic layers were washed with brine (10 mL), dried over $Na_2SO_4$, filtered and concentrated under reduced pressure. The residue was fractionated by prep-HPLC (column: Waters Xbridge Prep OBD C18 150*40mm*10um; mobile phase: [water(0.04% $NH_3H_2O$ + 10mM $NH_4HCO_3$)-ACN]; B%: 30%-60%, 10min) to give 4-chloro-7-methoxy-6'-(trifluoromethyl)spiro[indane-1,3'-indoline]-2'-one (19 mg, 8.95% yield, 98.8% purity) as a white solid and 4,6-dichloro-7-methoxy-6'-(trifluoromethyl)spiro[indane-1,3'-indoline]-2'-one (60 mg, 24.4% yield, 93.3% purity) as a white solid.

4-chloro-7-methoxy-6'-(trifluoromethyl)spiro[indane-1,3'-indoline]-2'-one LCMS: 368.1 $(M+H)^+$
$^1$H NMR (400 MHz, $CDCl_3$): 7.694 (1H, s), 7.270 - 7.225 (2H, m), 7.143 (1H, s), 7.052 (1H, d), 6.601 (1H, d), 3.547 (3H, s), 3.320 (2H, m), 2.782 (1H, m), 2.362 (1H, m).
4,6-dichloro-7-methoxy-6'-(trifluoromethyl)spiro[indane-1,3'-indoline]-2'-one LCMS: $(M+H)^+$: 401.9 $(M+H)^+$
$^1$H NMR (400 MHz, $CDCl_3$): 7.840 (1H, br s), 7.330 (1H, s), 7.279 (1H, m), 7.199 (1H, s), 7.100 (1H, d), 3.429 (3H, s), 3.313 (2H, m), 2.815 (1H, m), 2.413 (1H, m).

**<u>Example 15:</u> 3-(2-chloro-5-methoxy-4-pyridyl)-3-methyl-6-(trifluoromethyl)indolin-2-one**

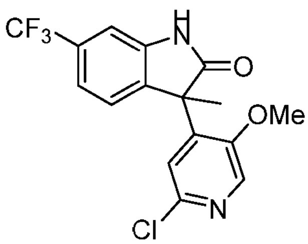

**Synthetic scheme:**

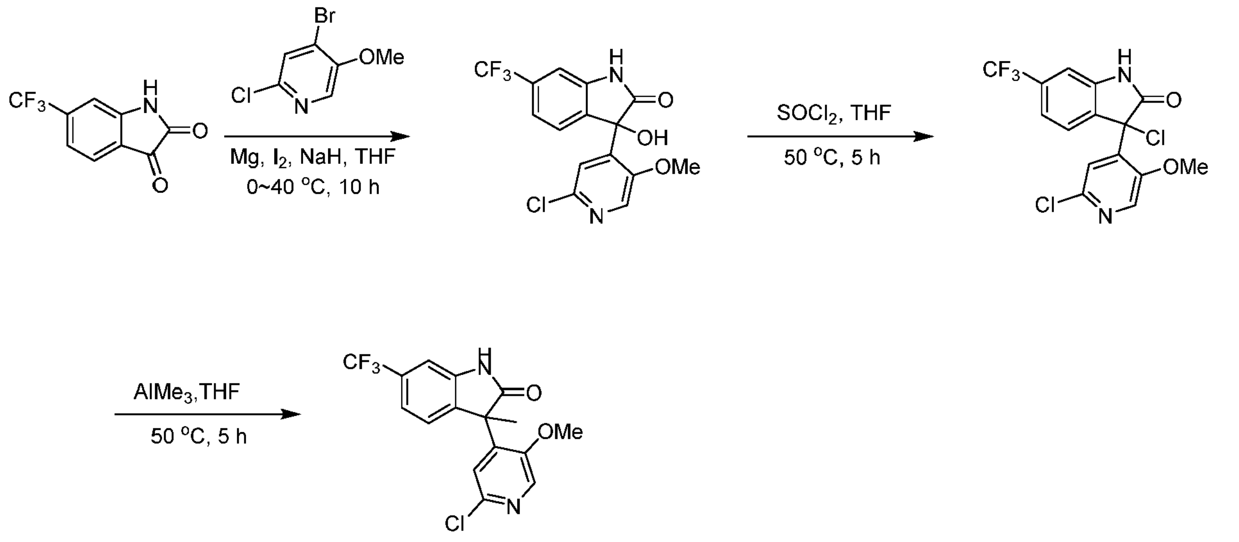


**Step 1:** Preparation of 3-(2-chloro-5-methoxy-4-pyridyl)-3-hydroxy-6- (trifluoromethyl)indolin-2-one

(a) To a mixture of Mg (564.89 mg, 23.24 mmol, 5 eq) and $I_2$ (117.98 mg, 464.84 umol, 93.63 uL, 0.1 eq) was added dropwise a solution of 4-bromo-2-chloro-5-methoxy-pyridine (2.07 g, 9.30 mmol, 2 eq) in THF (10 mL) at 15 °C. The mixture was stirred at 40 °C for 2 hr under $N_2$.

(b) To a solution of 6-(trifluoromethyl)indoline-2,3-dione (1 g, 4.65 mmol, 1 eq) in THF (10 mL) was added NaH (278.88 mg, 6.97 mmol, 60% purity, 1.5 eq) slowly at 0 °C, and the mixture was stirred for 1 h. The solution from (a) above was added under $N_2$ and the mixture was stirred at 15 °C for 7 hr under $N_2$. The reaction mixture was diluted with water (15 mL) and extracted four times with EtOAc (10 mL). The combined organic layers were washed with brine (10 mL), dried over $Na_2SO_4$, filtered and concentrated under reduced pressure. The residue was purified by column chromatography ($SiO_2$, petroleum ether / ethyl acetate, 10:1 to 1:1 gradient) to give 3-(2-chloro-5-methoxy-4-pyridyl)-3-hydroxy-6-(trifluoromethyl)indolin-2-one (1.5 g, 89.9% yield) as a yellow solid.

**Step 2**: Preparation of 3-chloro-3-(2-chloro-5-methoxy-4-pyridyl)-6-(trifluoromethyl)indolin -2-one

To a solution of 3-(2-chloro-5-methoxy-4-pyridyl)-3-hydroxy-6-(trifluoromethyl)indolin-2-one (200 mg, 557.57 umol, 1 eq) in DCM (5 mL) was added $SOCl_2$ (331.67 mg, 2.79 mmol, 202.24 uL, 5 eq). The mixture was stirred at 50 °C for 5 hr under $N_2$. The reaction mixture was diluted with water (2 mL) and extracted four times with EtOAc (2 mL). The combined organic layers were washed with brine (3 mL), dried over $Na_2SO_4$, filtered and concentrated under reduced pressure. The residue was purified by column chromatography ($SiO_2$, petroleum ether / ethyl acetate, 5:1 to 2:1 gradient) to give 3-chloro-3-(2-chloro-5-methoxy-4-pyridyl)-6-(trifluoromethyl)indolin-2-one (190 mg, 90.4% yield) as a white solid.

**Step 3**: Preparation of 3-(2-chloro-5-methoxy-4-pyridyl)-3-methyl-6-(trifluoromethyl)indolin-2-one

To a solution of 3-chloro-3-(2-chloro-5-methoxy-4-pyridyl)-6-(trifluoromethyl)indolin-2-one (180 mg, 477.27 umol, 1 eq) in THF (3 mL) was added dropwise $AlMe_3$ (2 M, 1.91 mL, 8 eq) at 0 °C. The mixture was warmed to 50 °C and stirred for 5 hr under $N_2$. The reaction mixture was diluted with water (10 mL) and extracted four times with EtOAc (10 mL). The combined organic layers were washed with brine (10 mL), dried over $Na_2SO_4$, filtered and concentrated under reduced pressure. The residue was purified by prep-HPLC (column: Waters Xbridge BEH C18 100*30mm*10um; mobile phase: [water(0.04%$NH_3H_2O$)-ACN]; B%: 25%-55%, 10min) to give 3-(2-chloro-5-methoxy-4-pyridyl)-3-methyl-6-(trifluoromethyl)indolin-2-one (19 mg, 5.6% yield, 99.8% purity) as a white solid.

LCMS: 356.9 $(M+H)^+$

$^1H$ NMR (400 MHz, $CDCl_3$): 7.924 (1H, s), 7.555 (1H, br s), 7.247 (1H), 7.178 (1H, s), 6.953 (1H, d), 3.613 (3H, s), 1.754 (3H, s).

**Example 16: 3-(3-methoxy-4-pyridyl)-3-methyl-6-(trifluoromethyl)indolin-2-one**

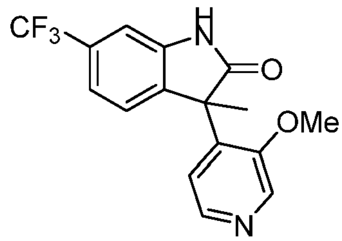

To a solution of 3-(2-chloro-5-methoxy-4-pyridyl)-3-methyl-6-(trifluoromethyl)indolin-2-one (example 15, 7 mg, 19.6 umol, 1 eq) in THF (4 mL) was added Pd/C (10%, 0.02 g) under $H_2$ atmosphere. The suspension was degassed and purged with $H_2$ three times. The mixture was stirred under $H_2$ (15 Psi) at 15 °C for 5 hr. The reaction mixture was filtered and filtrate was concentrated under reduced pressure. The residue was purified by prep-TLC ($SiO_2$, petroleum ether / ethyl acetate, 0:1) to give 3-(3-methoxy-4-pyridyl)-3-methyl-6-(trifluoromethyl)indolin-2-one (2 mg, 95% purity) as a white solid.

LCMS: 323.1 $(M+H)^+$
$^1H$ NMR (400 MHz, $CDCl_3$): 8.394 (1H, d), 8.204 (1H, s), 7.789 (1H, br s), 7.507 (1H, d), 7.232 (1H, d), 7.177 (1H, s), 6.922 (1H, d), 3.627 (3H, s), 1.773 (3H, s).

**Example 17: 3-methyl-3-(4-pyridyl)-6-(trifluoromethyl)indolin-2-one**

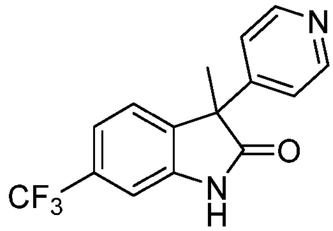

**Synthetic scheme:**

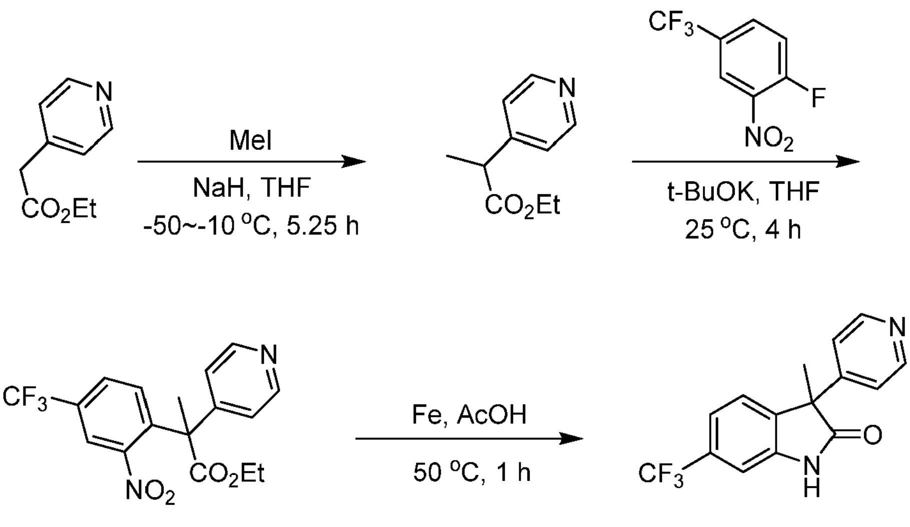

**Step 1:** Preparation of ethyl 2-(4-pyridyl)propanoate

To a solution of ethyl 2-(4-pyridyl)acetate (1.5 g, 9.08 mmol, 1.39 mL, 1 eq) in THF (10 mL) was added dropwise NaH (363.19 mg, 9.08 mmol, 60% purity, 1 eq) at -50 °C under $N_2$. The mixture was stirred for 1 h. MeI (7.15 g, 50.34 mmol, 3.13 mL, 5.54 eq) was added slowly at -50 °C, over 15 min, then the reaction mixture was warmed to -10 °C, and stirred for another 4 h. The reaction mixture was quenched by addition water (20 mL) at 0°C, and then extracted three times with EtOAc 10 mL). The combined organic layers were washed three times with saturated brine (10 mL), dried over $Na_2SO_4$, filtered and concentrated under reduced pressure. The residue was purified by column chromatography ($SiO_2$, petroleum ether / ethyl acetate 20:1 to 5:1 gradient) to give ethyl 2-(4-pyridyl)propanoate (800 mg, 49.2% yield) as a yellow oil.

**Step 2**: Preparation of ethyl 2-[2-nitro-4-(trifluoromethyl)phenyl]-2-(4-pyridyl)propanoate

To a solution of ethyl 2-(4-pyridyl)propanoate (800 mg, 4.46 mmol, 462.96 uL, 1 eq) and 1-fluoro-2-nitro-4-(trifluoromethyl)benzene (952.06 mg, 4.55 mmol, 638.97 uL, 1.02 eq) in THF (5 mL) was added KOtBu (751.36 mg, 6.70 mmol, 1.5 eq). The mixture was stirred at 25 °C for 4 hr. The reaction mixture was quenched by addition saturated $NH_4Cl$ (30 mL) at 0°C, and extracted three times with EtOAc (10 mL). The combined organic layers were washed twice with saturated brine (10 mL), dried over $Na_2SO_4$, filtered and concentrated under reduced pressure. The residue was purified by column chromatography ($SiO_2$, petroleum ether / ethyl acetate, 10:0 to 5:1 gradient) to give ethyl 2-[2-nitro-4-(trifluoromethyl)phenyl]-2-(4-pyridyl)propanoate (1 g, 2.72 mmol, 60.8% yield) as a yellow oil.

**Step 3**: Preparation of 3-methyl-3-(4-pyridyl)-6-(trifluoromethyl)indolin-2-one

To a solution of ethyl 2-[2-nitro-4-(trifluoromethyl)phenyl]-2-(4-pyridyl)propanoate (1 g, 2.72 mmol, 1 eq) in AcOH (10 mL) was added Fe (1.52 g, 27.15 mmol, 10 eq) and the mixture was stirred at 50 °C for 1 hr. The reaction mixture was filtered and concentrated under reduced pressure. The residue was purified by prep-HPLC (column: Waters Xbridge Prep OBD C18 150*40mm*10um; mobile phase: [water(0.04% $NH_3H_2O$ + 10mM $NH_4HCO_3$)-ACN]; B%: 10%-40%, 10min) to give 3-methyl-3-(4-pyridyl)-6-(trifluoromethyl)indolin-2-one (412 mg, 51.92 yield, 100% purity) as a white solid.

LCMS: 293.0 $(M+H)^+$
$^1H$ NMR (400 MHz, DMSO): 10.968 (1H, s), 8.523 (2H, d), 7.478 (1H, d), 7.386 (1H, d), 7.264 (2H, d), 7.180 (1H, s), 1.725 (3H, s).

**Example 18: 3-(2-chloro-4-pyridyl)-3-methyl-6-(trifluoromethyl)indolin-2-one**

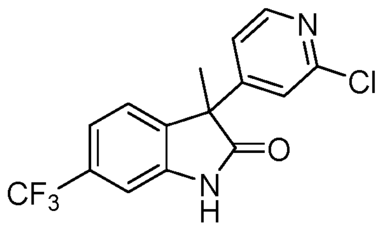

**Synthetic scheme:**

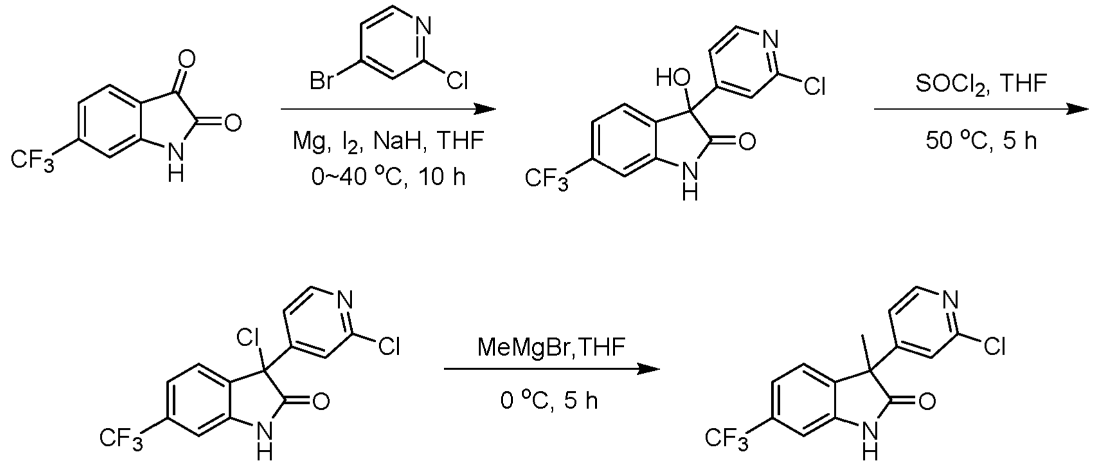


**Step 1:** Preparation of 3-(2-chloro-4-pyridyl)-3-hydroxy-6-(trifluoromethyl)indolin-2-one.

(a) To a mixture of Mg (282.45 mg, 11.62 mmol, 5 eq) and $I_2$ (58.99 mg, 232.42 umol, 46.82 uL, 0.1 eq) was added dropwise a solution of 4-bromo-2-chloro-pyridine (894.54 mg, 4.65 mmol, 2 eq) in THF (10 mL) at 15 °C. The mixture was stirred at 40 °C for 2 hr under $N_2$.

(b) To a solution of 6-(trifluoromethyl)indoline-2,3-dione (500 mg, 2.32 mmol, 1 eq) in THF (10 mL) was added NaH (60%, 139.44 mg, 3.49 mmol, 1.5 eq) slowly at 0 °C, and stirred for 1 h. The solution of from (a) above was added under $N_2$. The mixture was stirred at 15 °C for 7 hr under $N_2$. The reaction mixture was diluted with water (15 mL) and extracted four times with EtOAc (10 mL). The combined organic layers were washed with brine (10 mL), dried over $Na_2SO_4$, filtered and concentrated under reduced pressure. The residue was purified by column chromatography ($SiO_2$, petroleum ether / ethyl acetate, 10:1 to 1:1 gradient) to give 3-(2-chloro-4-pyridyl)-3-hydroxy-6-(trifluoromethyl) indolin-2-one (300 mg, 39.3% yield) as a yellow solid.

**Step 2:** Preparation of 3-chloro-3-(2-chloro-4-pyridyl)-6-(trifluoromethyl)indolin-2-one.

To a solution of 3-(2-chloro-4-pyridyl)-3-hydroxy-6-(trifluoromethyl)indolin-2-one (300 mg, 912.76 umol, 1 eq) in DCM (5 mL) was added $SOCl_2$ (1.09 g, 9.13 mmol, 662.14 uL, 10 eq). The mixture was stirred at 50 °C for 5 hr under $N_2$. The reaction mixture was diluted with water (2 mL) and extracted four times with EtOAc (2 mL). The combined organic layers were washed with brine (3 mL), dried over $Na_2SO_4$, filtered and concentrated under reduced pressure. The residue was purified by column chromatography ($SiO_2$, petroleum ether / ethyl acetate, 5:1 to 2:1) to give 3-chloro-3-(2-chloro-4-pyridyl)-6-(trifluoromethyl)indolin-2-one (200 mg, 63.1% yield) as a white solid.

**Step 3**: Preparation of 3-(2-chloro-4-pyridyl)-3-methyl-6-(trifluoromethyl)indolin-2-one.

To a solution of 3-chloro-3-(2-chloro-4-pyridyl)-6-(trifluoromethyl)indolin-2-one (150 mg, 432.13 umol, 1 eq) in THF (3 mL) was added dropwise MeMgBr (3M, 864.26 uL, 6 eq) at 0 °C. The mixture was stirred at 0 °C for 5 hr under $N_2$. The reaction mixture was diluted with water (2 mL) and extracted four times with EtOAc (2 mL). The combined organic layers were washed with brine (3 mL), dried over $Na_2SO_4$, filtered and concentrated under reduced pressure. The residue was purified by prep-HPLC (column: Waters Xbridge BEH C18 100*30mm*10um; mobile phase: [water(0.04% $NH_3H_2O$)-ACN]; B%: 20%-80%, 10min) to give 3-(2-chloro-4-pyridyl)-3-methyl-6-(trifluoromethyl)indolin-2-one (8 mg, 5.2% yield, 92% purity) as a white solid.

LCMS: 327.0 $(M+H)^+$
$^1H$ NMR (400 MHz, $CDCl_3$): 8.281 (1H, d), 7.490 (1H, br s), 7.356 (1H, d), 7.226 - 7.100 (4H, m), 1.836 (3H, s).

**Example 19: 3-[2-methoxy-5-(1-methylimidazol-2-yl)phenyl]-3-methyl-6-(trifluoromethyl) indolin-2-one**

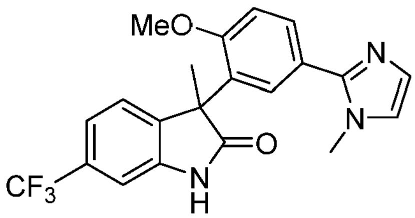

**Synthetic scheme:**

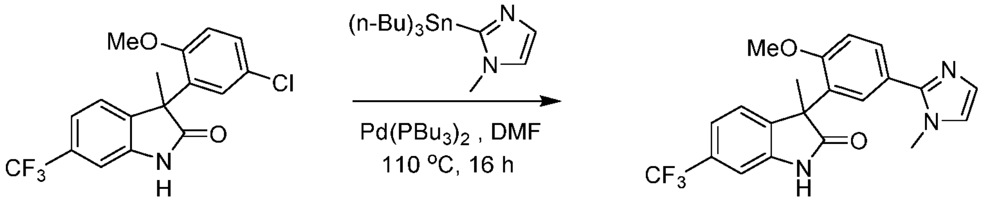


To a solution of 3-(5-chloro-2-methoxy-phenyl)-3-methyl-6-(trifluoromethyl)indolin-2-one (example 1, 50 mg, 140.55 umol, 1 eq) in DMF (1 mL) was added palladium bis(tri-tert-butylphosphane) (71.83 mg, 140.55 umol, 1 eq) and tributyl-(1-methylimidazol-2-yl)stannane (78.25 mg, 210.83 umol, 1.5 eq). The mixture was stirred at 110 °C for 16 hr under $N_2$. The reaction mixture was quenched by addition saturated $NH_4Cl$ 10 mL at 0°C, and then extracted three times with EtOAc (5 mL). The combined organic layers were washed twice with saturated brine (5 mL), dried over $Na_2SO_4$, filtered and concentrated under reduced pressure. The residue was purified by prep-HPLC (column: Waters Xbridge BEH C18 100*25mm*5um; mobile phase: [water(0.04% $NH_3H_2O$ + 10mM $NH_4HCO_3$)-ACN]; B%: 25%-55%, 12min) to give 3-[2-methoxy-5-(1-methylimidazol-2-yl)phenyl]-3-methyl-6-(trifluoromethyl)indolin-2-one (15 mg, 13.3% yield, 100% purity) as a white solid.

LCMS: 402.0 $(M+H)^+$
$^1H$ NMR (400 MHz, DMSO): 10.705 (1H, s), 7.712 (1H, s), 7.635 (1H, s), 7.420 (1H, d), 7.201 (1H, d), 7.045 - 6.982 (4H, m), 3.712 (3H, s), 3.447 (3H, s).

**Example 20: 3-(5-chloro-2-methoxy-phenyl)-3-methyl-6-(trifluoromethyl)-1H-pyrrolo[3,2-b]pyridin-2-one (reference)**

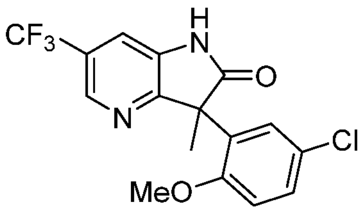

**Synthetic scheme:**

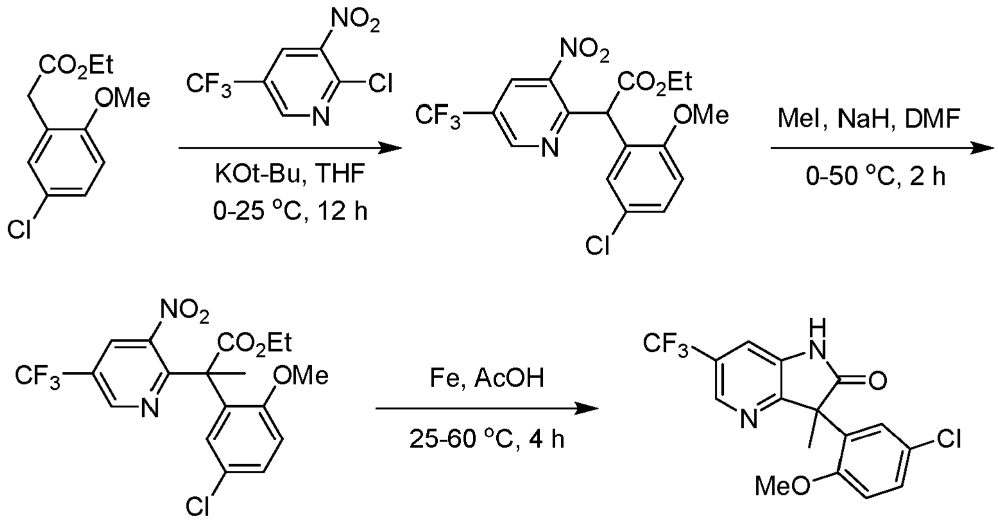


**Step 1:** Preparation of ethyl 2-(5-chloro-2-methoxy-phenyl)-2-[3-nitro-5-(trifluoromethyl)-2-pyridyl]acetate.

To a mixture of ethyl 2-(5-chloro-2-methoxy-phenyl)acetate (1.5 g, 6.56 mmol, 1 eq) and 2-chloro-3-nitro-5-(trifluoromethyl)pyridine (1.52 g, 6.69 mmol, 1.02 eq) in THF (10 mL) was added t-BuOK (1.10 g, 9.84 mmol, 1.5 eq) in one portion at 0°C under $N_2$. The mixture was stirred at 25°C for 12 hours. The reaction mixture was filtered and concentrated under reduced pressure. The residue was purified by prep-HPLC [water(10mM $NH_4HCO_3$)-ACN] to give ethyl 2-(5-chloro-2-methoxy-phenyl)-2-[3-nitro-5-(trifluoromethyl)-2-pyridyl]acetate (350 mg, 12.7% yield) as yellow oil.
LCMS: 419.0 $(M+H)^+$

**Step 2:** Preparation of ethyl 2-(5-chloro-2-methoxy-phenyl)-2-[3-nitro-5-(trifluoromethyl)-2-pyridyl]propanoate.

To a mixture of ethyl 2-(5-chloro-2-methoxy-phenyl)-2-[3-nitro-5-(trifluoromethyl)-2-pyridyl]acetate (300 mg, 716.42 umol, 1 eq) and MeI (610.12 mg, 4.30 mmol, 267.60 uL, 6 eq) in DMF (5 mL) was added NaH (60%, 42.98 mg, 1.07 mmol, 1.5 eq) in one portion at 0°C under $N_2$. The mixture was stirred at 0 °C for 0.5 h and then heated to 50 °C and stirred for 1.5 hours. The reaction was quenched by addition of water (9 mL), extracted three times with EtOAc (9 mL), dried over $Na_2SO_4$, filtered and concentrated under reduced pressure. The residue was purified by prep-TLC ($SiO_2$, petroleum ether / ethyl acetate, 3:1) to give ethyl 2-(5-chloro-2-methoxy-phenyl)-2-[3-nitro-5-(trifluoromethyl)-2-pyridyl]propanoate (0.2 g) as a yellow oil.
LCMS: 433.1 $(M+H)^+$

**Step 3:** Preparation of 3-(5-chloro-2-methoxy-phenyl)-3-methyl-6-(trifluoromethyl)-1H-pyrrolo[3,2-b]pyridin-2-one.

To a mixture of ethyl 2-(5-chloro-2-methoxy-phenyl)-2-[3-nitro-5-(trifluoromethyl)-2-pyridyl]propanoate (0.2 g, 462.13 umol, 1 eq) in AcOH (5 mL) was added Fe (258.08 mg, 4.62 mmol, 10 eq) in one portion at 25°C under $N_2$. The mixture was stirred at 60 °C for 4 hours. The reaction mixture was filtered and the filtrate was concentrated under reduced pressure. The residue was purified by column chromatography ($SiO_2$, petroleum ether / ethyl acetate, 30:1 to 2:1 gradient) to give 3-(5-chloro-2-methoxy-phenyl)-3-methyl-6-(trifluoromethyl) -1H-pyrrolo[3,2-b]pyridin-2-one (29.5 mg, 97% purity) as a white solid.

LCMS: 355.0 $(M-H)^-$
$^1H$ NMR (400 MHz, $CDCl_3$): 8.380 (1H, s), 7.847 (1H, br s), 7.581 (1H, d), 7.372 (1H, s), 7.298 (1H, dd), 6.731 (1H, d), 3.475 (3H, s), 1.795 (3H, s).

**Example 21: 6'-chloro-6-(trifluoromethyl)-3'H-spiro[indoline-3,1'-isobenzofuran]-2,3'-dione (reference)**

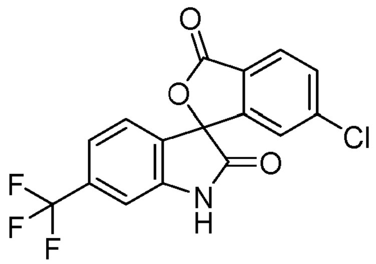

**Synthetic scheme:**

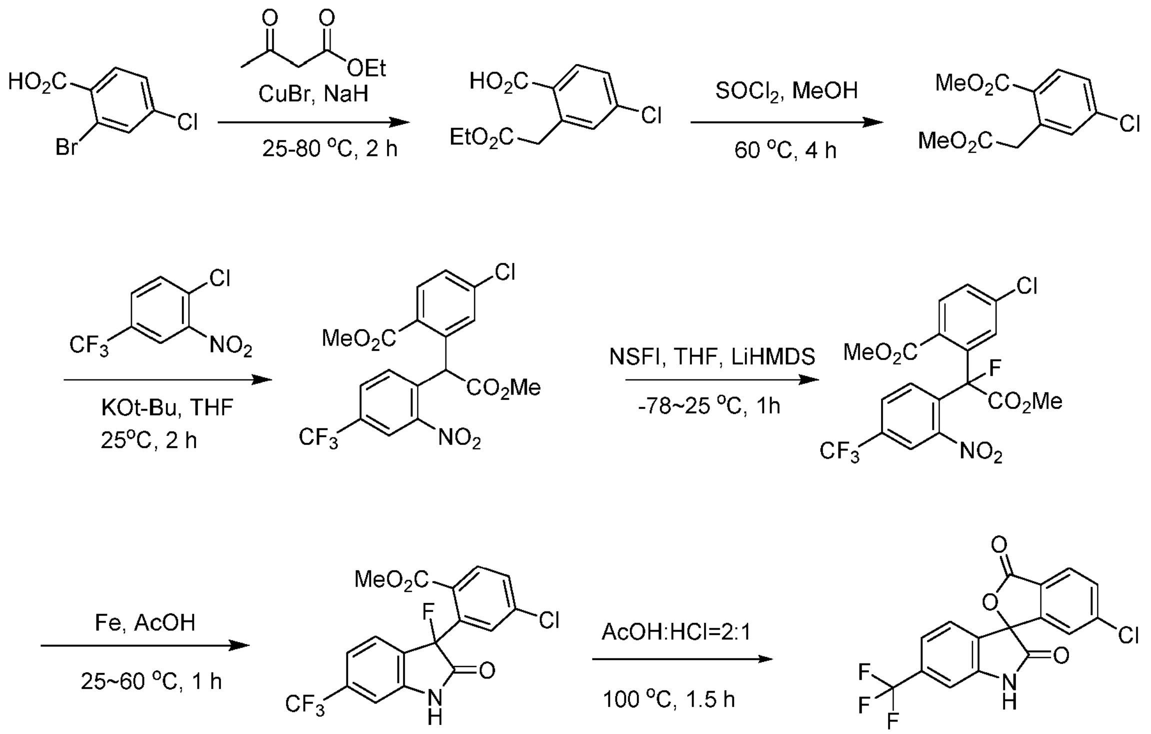


**Step 1:** Preparation of 4-chloro-2-(2-ethoxy-2-oxo-ethyl)benzoic acid.

To a mixture of 2-bromo-4-chloro-benzoic acid (10 g, 42.47 mmol, 1 eq) and CuBr (341.17 mg, 2.38 mmol, 72.43 uL, 0.056 eq) in ethyl 3-oxobutanoate (74.17 g, 569.94 mmol, 72.01 mL, 13.42 eq) was added NaH (4.08 g, 101.93 mmol, 60% purity, 2.4 eq) in one portion at 25°C under $N_2$. The mixture was stirred at 80 °C for 2 hours. The reaction was poured into water (50 mL), then was washed three times with EtOAc (20 mL) and these organic fractions were discarded. The aqueous layer was acidified to pH 1 using concentrated HCl and extracted three times with EtOAc (20 mL). The combined organic layers were washed with brine (10 mL), dried over $Na_2SO_4$, filtered and concentrated under reduced pressure. The residue was triturated with petroleum ether / ethyl acetate (10:1, 20 mL) at 25 °C for 10 min to give 4-chloro-2-(2-ethoxy-2-oxo-ethyl)benzoic acid (5 g, 48.5% yield) as a white solid.

**Step 2:** Preparation of methyl 4-chloro-2-(2-methoxy-2-oxo-ethyl)benzoate.

To a solution of 4-chloro-2-(2-ethoxy-2-oxo-ethyl)benzoic acid (5 g, 23.30 mmol, 1 eq) in MeOH (40 mL) was added $SOCl_2$ (13.86 g, 116.49 mmol, 8.45 mL, 5 eq). The mixture was stirred at 60 °C for 4 hr. The reaction mixture was concentrated under reduced pressure and the residue was purified by column chromatography ($SiO_2$, petroleum ether / ethyl acetate, 10:1 to 5:1 gradient) to give methyl 4-chloro-2-(2-methoxy-2-oxo-ethyl)benzoate (3.5 g, 61.9% yield) as a colorless oil.

**Step 3:** Preparation of methyl 4-chloro-2-[2-methoxy-1-[2-nitro-4-(trifluoromethyl)phenyl]-2-oxo-ethyl]benzoate.

To a mixture of methyl 4-chloro-2-(2-methoxy-2-oxo-ethyl)benzoate (3.1 g, 12.78 mmol, 1 eq) and 1-chloro-2-nitro-4-(trifluoromethyl)benzene (2.94 g, 13.03 mmol, 1.95 mL, 1.02 eq) in THF (30 mL) was added t-BuOK (2.87 g, 25.55 mmol, 2 eq) in one portion at 25°C under $N_2$. The mixture was stirred at 25°C for 2h. The reaction mixture was diluted with water (30 mL) and extracted three times with EtOAc (40 mL). The combined organic layers were dried over $Na_2SO_4$, filtered and concentrated under reduced pressure. The residue was purified by column chromatography ($SiO_2$, petroleum ether / ethyl acetate, 50:1 to 10:1 gradient) to give methyl 4-chloro-2-[2-methoxy-1-[2-nitro-4-(trifluoromethyl)phenyl]-2-oxo-ethyl]benzoate (3.2 g) as a white solid.

**Step 4:** Preparation of methyl 4-chloro-2-[1-fluoro-2-methoxy-1-[2-nitro-4-(trifl uoromethyl) phenyl]- 2-oxo-ethyl] benzoate.

To a mixture of methyl 4-chloro-2-[2-methoxy-1-[2-nitro-4-(trifluoromethyl)phenyl]-2-oxoethyl]benzoate (3.2 g, 7.41 mmol, 1 eq) in THF (30 mL) was added LiHMDS (1 M, 9.64 mL, 1.3 eq) in one portion at -78°C under $N_2$. The mixture was stirred at -78 °C for 30 min. NFSI (2.38 g, 7.56 mmol, 1.02 eq) was added and the mixture was stirred for 30 min. The reaction mixture was diluted with water (30 mL) and extracted three times with EtOAc (40 mL), dried over $Na_2SO_4$, filtered and concentrated under reduced pressure. The residue was purified by column chromatography ($SiO_2$, petroleum ether / ethyl acetate, 30:1 to 1:1 gradient) to give methyl 4-chloro-2-[1-fluoro-2-methoxy-1-[2-nitro-4-(trifluoromethyl)phenyl]-2-oxoethyl]benzoate (2 g) as a white solid.

**Step 5:** Preparation of methyl 4-chloro-2-[3-fluoro-2-oxo-6-(trifluoromethyl)indolin-3-yl] benzoate.

To a mixture of methyl 4-chloro-2-[1-fluoro-2-methoxy-1-[2-nitro-4-(trifluoromethyl)phenyl]-2-oxo-ethyl]benzoate (0.43 g, 956.11 umol, 1 eq) in AcOH (3 mL) was added Fe (533.94 mg, 9.56 mmol, 10 eq) in one portion at 25°C under $N_2$. The mixture was stirred at 60 °C for 1 hour. The reaction mixture was filtered and concentrated under reduced pressure to give a residue. The residue was purified by column chromatography ($SiO_2$, petroleum ether / ethyl acetate, 30:1 to 3:1 gradient) followed by prep-HPLC [water (0.04%$NH_3H_2O$+10mM $NH_4HCO_3$)-ACN] to give methyl 4-chloro-2-[3-fluoro-2-oxo-6-(trifluoromethyl)indolin-3-yl]benzoate (0.138 g, 36.5% yield, 98% purity) as white solid.
LCMS: $(M-H)^-$: 386.0

**Step 6.** Preparation of 6'-chloro-6-(trifluoromethyl)-3'H-spiro[indoline-3,1'-isobenzofuran]-2,3'-dione.

Methyl 4-chloro-2-[3-fluoro-2-oxo-6-(trifluoromethyl)indolin-3-yl]benzoate (0.2 g, 515.85 umol, 1 eq) in AcOH (2 mL) and HCl (1 mL) was stirred at 100 °C for 1.5 hours. The reaction mixture was filtered and concentrated under reduced pressure and the residue was purified by prep-HPLC [water(0.04% $NH_3H_2O$+10mM $NH_4HCO_3$)-ACN] to give 6'-chloro-6-(trifluoromethyl)-3'H-spiro[indoline-3,1'-isobenzofuran]-2,3'-dione (110 mg, 54.15% yield, 94.9% purity) as a white solid.

LCMS: $(M-H)^-$: 352.0
$^1H$ NMR (400 MHz, MeOD): 8.003 (1H, d), 7.735 (1H, dd), 7.412 (2H), 7.341 (1H, s), 7.297 (1H, d)

__Example 22:__ **3-(5-chloro-2-methoxy-phenyl)-6-(difluoromethyl)-3-methyl-indolin-2-one**

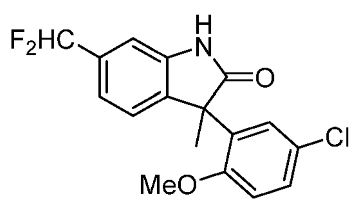

**Synthetic scheme:**

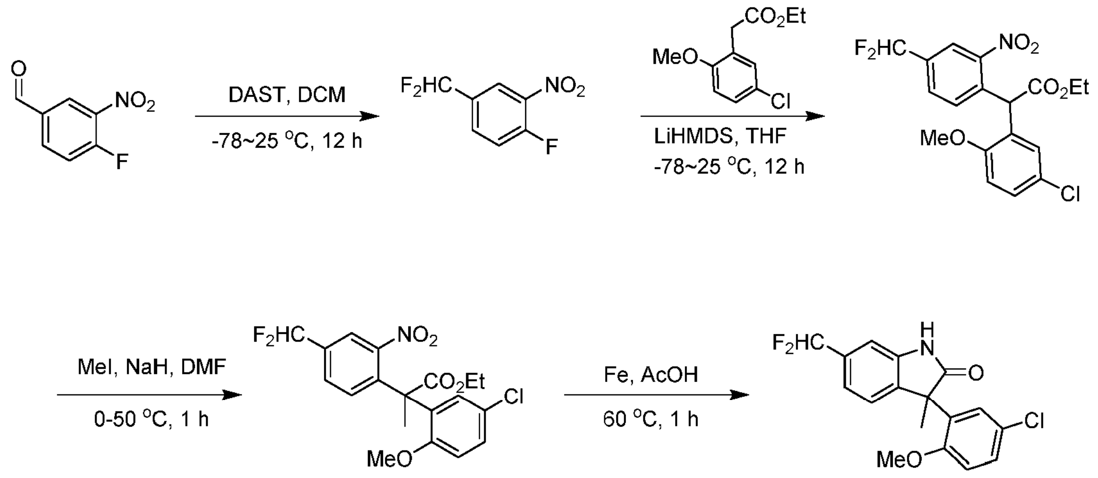


**Step 1:** Preparation of 4-(difluoromethyl)-1-fluoro-2-nitro-benzene.

To a mixture of 4-fluoro-3-nitro-benzaldehyde (2 g, 11.83 mmol, 1 eq) in DCM (20 mL) was added DAST (3.81 g, 23.65 mmol, 3.13 mL, 2 eq) in one portion at -78°C under $N_2$. The mixture was stirred at -78 °C for 3 h, then heated to 25 °C and stirred for 9 hours. The reaction mixture was concentrated under reduced pressure and the residue was purified by column chromatography ($SiO_2$, etroleum ether / ethyl acetate, 30:1 to 3:1) to give 4-(difluoromethyl)-1-fluoro-2-nitro-benzene (1.7 g, 75.2% yield) as a yellow oil.

**Step 2:** Preparation of ethyl 2-(5-chloro-2-methoxy-phenyl)-2-[4-(difluoromethyl)-2-nitrophenyl]acetate.

To a mixture of ethyl 2-(5-chloro-2-methoxy-phenyl)acetate (1 g, 4.37 mmol, 1 eq) and 4-(difluoromethyl)-1-fluoro-2-nitro-benzene (852.44 mg, 4.46 mmol, 1.02 eq) in THF (10 mL) was added LiHMDS (1 M, 9.62 mL, 2.2 eq) in one portion at -78°C under $N_2$. The mixture was stirred at 25 °C for 12 hours. The reaction mixture was quenched by addition water (10 mL) at 25°C and extracted three times with EtOAc (10 mL), dried over $Na_2SO_4$, filtered and concentrated under reduced pressure. The residue was purified by prep-HPLC [water (0.04%$NH_3H_2O$+10mM $NH_4HCO_3$)-ACN] to give ethyl 2-(5-chloro-2-methoxy-phenyl)-2-[4-(difluoromethyl)-2-nitro-phenyl]acetate (0.11 g, 6.29% yield) as a yellow oil.

**Step 3:** Preparation of ethyl 2-(5-chloro-2-methoxy-phenyl)-2-[4-(difluoromethyl)-2-nitrophenyl] propanoate.

To a mixture of ethyl 2-(5-chloro-2-methoxy-phenyl)-2-[4-(difluoromethyl)-2-nitro-phenyl] acetate (0.11 g, 275.16 umol, 1 eq) and MeI (234.33 mg, 1.65 mmol, 102.78 uL, 6 eq) in DMF (3 mL) was added NaH (60%, 16.51 mg, 412.73 umol, 1.5 eq) in one portion at 0°C under $N_2$. The mixture was stirred at 0 °C for 0.5 hours, then heated to 50 °C and stirred for 0.5 hours. The reaction mixture was quenched by addition of water (3 mL) at 25°C and extracted three times with EtOAc (3 mL). The combined organic layers were dried over $Na_2SO_4$, filtered and concentrated under reduced pressure to give ethyl 2-(5-chloro-2-methoxy-phenyl)-2-[4-(difluoromethyl)-2-nitro-phenyl]propanoate (100 mg, crude) as yellow oil which was used without further purification.

**Step 4:** Preparation of 3-(5-chloro-2-methoxy-phenyl)-6-(difluoromethyl)-3-methyl-indolin-2-one.

To a mixture of ethyl 2-(5-chloro-2-methoxy-phenyl)-2-[4-(difluoromethyl)-2-nitrophenyl]propanoate (0.1 g, 241.66 umol, 1 eq) in AcOH (3 mL) was added Fe (134.96 mg, 2.42 mmol, 10 eq) and the mixture was stirred at 60 °C for 1 hour. The reaction mixture was filtered and concentrated under reduced pressure. The residue was purified by prep-TLC ($SiO_2$, petroleum ether / ethyl acetate, 2:1) to give 3-(5-chloro-2-methoxy-phenyl)-6-(difluoromethyl)-3-methyl-indolin-2-one (0.041 g, 48.7% yield, 97% purity) as a white solid.

LCMS: $(M-H)^-$: 336.0
$^1H$ NMR (400 MHz, $CDCl_3$): 7.831 (1H, br s), 7.543 (1H, d), 7.281 (1H), 7.081 (1H, s), 7.069 (1H, d), 6.915 (1H, d), 6.728 (1H, d), 6.606 (1H, t), 3.477 (3H, s), 1.729 (3H, s).

**Example 23: 3-(5-chloro-2-methoxy-3-pyridyl)-3-methyl-6-(trifluoromethyl)indolin-2-one**

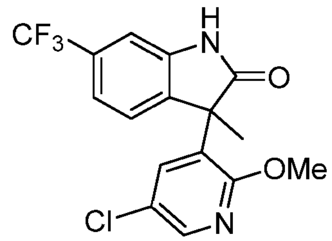

Synthetic scheme:

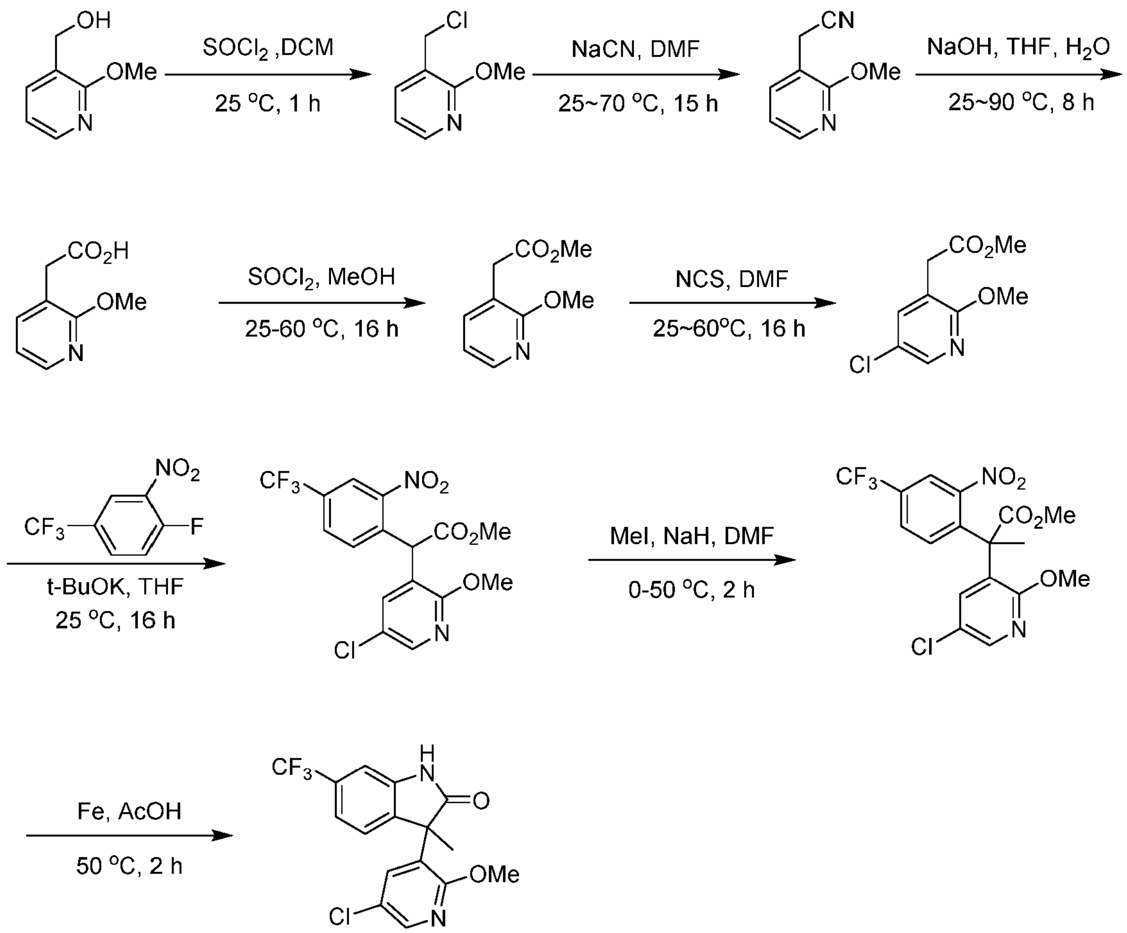


**Step** 1: Preparation of 3-(chloromethyl)-2-methoxy-pyridine.

To a mixture of (2-methoxy-3-pyridyl)methanol (8.0 g, 57.5 mmol, 1.0 eq) in dichloromethane (80.0 mL) was added $SOCl_2$ (17.1 g, 143.7 mmol, 10.4 mL, 2.5 eq) in one portion at 25°C under $N_2$. The mixture was stirred at 25 °C for 1 hour. The reaction was concentrated in vacuum. The residue was re-suspended in dichloromethane (50.0 mL) and saturated sodium bicarbonate solution was added carefully. The mixture was stirred for 10 min and the organic phase was separated, dried over magnesium sulphate, filtered and concentrated in vacuum to give 3-(chloromethyl)-2-methoxy-pyridine (8.1 g) as a yellow oil.

Step 2: Preparation of 2-(2-methoxy-3-pyridyl) acetonitrile.

To a mixture of 3-(chloromethyl)-2-methoxy-pyridine (8.0 g, 50.8 mmol, 1.0 eq) in DMF (50.0 mL) was added NaCN (5.00 g, 101.5 mmol, 2.0 eq) in one portion at 25°C under $N_2$. The mixture was stirred at 50°C for 3 hours, then heated to 70°C and stirred for 12 hours. The mixture was cooled to 25°C and combined with another batch for work-up. The reaction was poured into ice-saturated sodium bicarbonate solution (w/w = 1/1, 50.0 mL) and stirred for 15 min. The aqueous phase was extracted three times with ethyl acetate (50.0 mL). The combined organic phase was washed twice with brine (50.0 mL), dried over anhydrous $Na_2SO_4$, filtered and concentrated in vacuum. The crude product was purified by silica gel chromatography (petroleum ether / ethyl acetate, 10:1 to 5:1 gradient) to give 2-(2-methoxy-3-pyridyl) acetonitrile as a yellow oil.

**Step 3:** Preparation of 2-(2-methoxy-3-pyridyl)acetic acid.

To a mixture of 2-(2-methoxy-3-pyridyl)acetonitrile (3.0 g, 20.2 mmol, 1.0 eq) in water (12.0 mL) and THF (4.0 mL) was added NaOH (3.2 g, 81.0 mmol, 4.0 eq) in one portion at 25°C under $N_2$. The mixture was stirred 90°C for 8 hours. The pH of the mixture was adjusted to 7 with 1N HCl (aq). The precipitate collected by filtration, washed with water (10 ml) and dried under vacuum to give 2-(2-methoxy-3-pyridyl)acetic acid (950.0 mg, 28.1% yield) as a yellow solid.

**Step 4:** Preparation of methyl 2-(2-methoxy-3-pyridyl)acetate.

To a solution of 2-(2-methoxy-3-pyridyl)acetic acid (950.0 mg, 5.7 mmol, 1.0 eq) in MeOH (10.0 mL) was added $SOCl_2$ (1.7 g, 14.2 mmol, 1.0 mL, 2.5 eq) in one portion at 25°C under $N_2$. The mixture was heated to 60°C and stirred for 16 hours. The mixture was cooled to 25°C and concentrated under reduced pressure at 45°C. The residue was poured into ice-water (w/w = 1/1, 10.0 mL) and stirred for 15 min. The aqueous phase was extracted three times with ethyl acetate (10.0 mL). The combined organic phase was washed twice with brine (10.0 mL), dried over anhydrous $Na_2SO_4$, filtered and concentrated in vacuum. The residue was purified by silica gel chromatography (petroleum ether / ethyl acetate, 5:1 to 2:1 gradient) to give methyl 2-(2-methoxy-3-pyridyl)acetate (570.0 mg, 55.4% yield) as a yellow oil.

**Step 5:** Preparation of methyl 2-(5-chloro-2-methoxy-3-pyridyl)acetate.

To a solution of methyl 2-(2-methoxy-3-pyridyl)acetate (570.0 mg, 3.2 mmol, 1.0 eq) in DMF (10.0 mL) was added NCS (840.2 mg, 6.3 mmol, 2.0 eq) in one portion at 25°C under $N_2$. The mixture was heated to 60°C and stirred for 16 hours. The mixture was poured into ice-water (w/w = 1/1, 10.0 mL) and stirred for 15 min. The aqueous phase was extracted three times with ethyl acetate (15.0 mL). The combined organic phase was washed twice with brine (15.0 mL), dried over anhydrous $Na_2SO_4$, filtered and concentrated in vacuum. The residue was purified by silica gel chromatography (petroleum ether / ethyl acetate, 10:1 to 0/1 gradient) to give methyl 2-(5-chloro-2-methoxy-3-pyridyl)acetate (200.0 mg, 40.0% yield) as a yellow oil.

**Step 6:** Preparation of methyl 2-(5-chloro-2-methoxy-3-pyridyl)-2-[2-nitro-4-(trifluoromethyl)phenyl]acetate.

To a mixture of methyl 2-(5-chloro-2-methoxy-3-pyridyl)acetate (180.0 mg, 834.8 umol, 1.0 eq) in THF (3.0 mL) was added t-BuOK (234.2 mg, 2.1 mmol, 2.5 eq) and 1-fluoro-2-nitro-4-(trifluoromethyl)benzene (192.0 mg, 918.2 umol, 128.9 uL, 1.1 eq) in one portion at 25°C under $N_2$. The mixture was stirred at 25°C for 16 hours. The mixture was poured into ice-water (w/w = 1/1, 5.0 mL) and stirred for 15 min. The aqueous phase was extracted three times with ethyl acetate (5.0 mL). The combined organic phase was washed twice with brine (5.0 mL), dried with anhydrous $Na_2SO_4$, filtered and concentrated in vacuum. The residue was purified by prep-TLC to give 2-(5-chloro-2-methoxy-3-pyridyl)-2-[2-nitro-4-(trifluoromethyl)phenyl]acetate (150 mg) as a yellow oil.

**Step 7:** Preparation of methyl 2-(5-chloro-2-methoxy-3-pyridyl)-2-[2-nitro-4-(trifluoromethyl)phenyl]propanoate.

To a mixture of methyl 2-(5-chloro-2-methoxy-3-pyridyl)-2-[2-nitro-4-(trifluoromethyl)phenyl]-acetate (150.0 mg, 370.6 umol, 1.0 eq) and MeI (157.8 mg, 1.1 mmol, 69.2 uL, 3.0 eq) in DMF (5.0 mL) was added NaH (22.2 mg, 555.9 umol, 60% purity, 1.5 eq) in one portion at 0°C under $N_2$. The mixture was stirred at 50°C for 2 hours. The mixture was cooled to 25 °C and poured into ice-water (w/w = 1/1, 5.0 mL) and stirred for 15 min. The aqueous phase was extracted three times with ethyl acetate (5.0 mL). The combined organic phase was washed twice with brine (5.0 mL), dried with anhydrous $Na_2SO_4$, filtered and concentrated in vacuum. Methyl 2-(5-chloro-2-methoxy-3-pyridyl)-2-[2-nitro-4-(trifluoromethyl)phenyl]propanoate (150 mg) was obtained as yellow oil.

**Step 8:** Preparation of 3-(5-chloro-2-methoxy-3-pyridyl)-3-methyl-6-(trifluoromethyl)indolin-2-one.

To a mixture of methyl 2-(5-chloro-2-methoxy-3-pyridyl)-2-[2-nitro-4-(trifluoromethyl)phenyl]-propanoate (150.0 mg, 358.2 umol, 1.0 eq) in AcOH (3.0 mL) was added Fe (200.0 mg, 3.6 mmol, 10.0 eq) in one portion at 25°C under $N_2$. The mixture was heated to 50°C and stirred for 2 hours. The mixture was cooled to 25 °C and concentrated under reduced pressure at 45 °C. The residue was poured into ice-water (w/w = 1/1) (5.0 mL) and stirred for 15 min. The aqueous phase was extracted three times with ethyl acetate (5.0 mL). The combined organic phase was dried with anhydrous $Na_2SO_4$, filtered and concentrated in vacuum. The residue was purified by prep-HPLC (column: Waters Xbridge Prep OBD C18 150*40mm*10um; mobile phase: [water(0.04% $NH_3H_2O$+10mM $NH_4HCO_3$)-ACN]; B%: 65%-80%, 8min) to give 3-(5-chloro-2-methoxy-3-pyridyl)-3-methyl-6-(trifluoromethyl)indolin-2-one (65.0 mg, 65.0% yield) as a white solid.

LCMS: 355.0 $(M-H)^-$
$^1H$ NMR (400 MHz, MeOD): 8.108 (1H, d), 8.034 (1H, d), 7.264 (1H, d), 7.226 (1H, s), 7.061 (1H, d), 3.631 (3H, s), 1.728 (3H, s).

The compounds described in the following can be prepared in accordance with, or in analogy to, the procedures described in the preceding examples and in the general part of the present specification.

**Example 24: 6-chloro-3-(5-chloro-2-methoxyphenyl)-3-methylindolin-2-one**

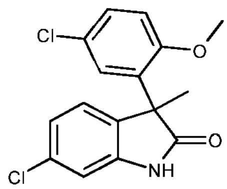

**Example 25: 6-chloro-3-(2-chloro-5-methoxypyridin-4-yl)-3-methylindolin-2-one**

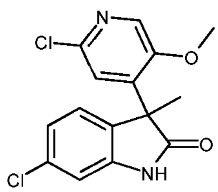

**Example 26: 3-(6-chloro-3-methoxypyridin-2-yl)-3-methyl-6-(trifluoromethyl)indolin-2-one**

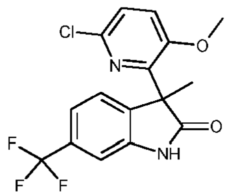

**Example 27: 6-chloro-3-(5-chloro-2-methoxyphenyl)-3-methyl-1H-pyrrolo[3,2-c]pyridin-2(3H)-one (reference)**

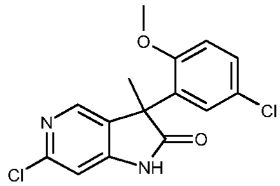

**Example 28: 6-chloro-3-(5-chloro-2-methoxypyridin-3-yl)-3-methylindolin-2-one**

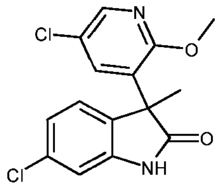

**Example 29:** 6-chloro-3-(2-methoxy-5-(1-methyl-1H-imidazol-2-yl)phenyl)-3-methylindolin-2-one

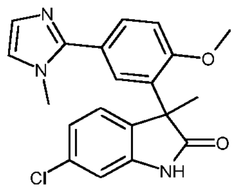

**Example 30:** 6-chloro-3-(5-chloro-2-methoxyphenyl)-3-methyl-1H-pyrrolo[3,2-b]pyridin-2(3H)-one (reference)

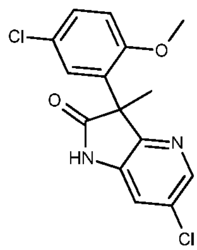

**Example 31:** 6-chloro-3-(6-chloro-3-methoxypyridin-2-yl)-3-methylindolin-2-one

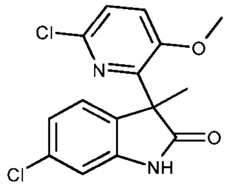

**Example 32:** 6-chloro-3-(2-methoxy-5-(1-methyl-1H-imidazol-2-yl)pyridin-3-yl)-3-methylindolin-2-one

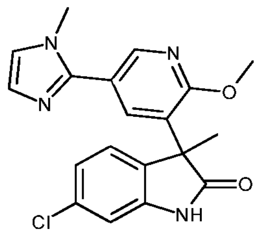

**Example 33:** 6-chloro-3-(5-chloro-2-methoxyphenyl)-3-methyl-1H-pyrrolo[2,3-b]pyridin-**2(3H)-one** (reference)

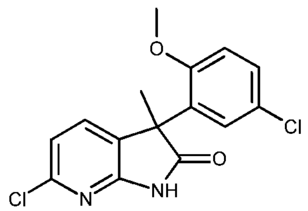

**Example 34:** 3-(5-chloro-2-methoxypyridin-3-yl)-3-methyl-6-(trifluoromethyl)-1H-pyrrolo[3,2-c]pyridin-2(3H)-one (reference)

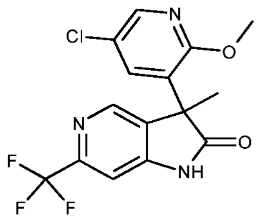

**Example 35:** 6-chloro-3-(5-methoxy-2-(1-methyl-1H-imidazol-2-yl)pyridin-4-yl)-3-methylindolin-2-one

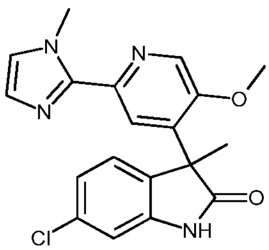

**Example 36:** 3-(5-chloro-2-methoxyphenyl)-3-methyl-6-(trifluoromethyl)-1H-pyrrolo[2,3-b]pyridin-2(3H)-one (reference)

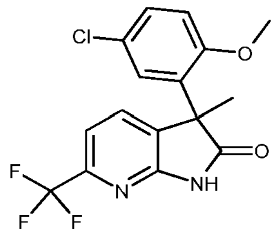

**Example 37:** 3-(5-chloro-2-methoxyphenyl)-3-methyl-6-(trifluoromethyl)-1H-pyrrolo[3,2-c]pyridin-2(3H)-one (reference)

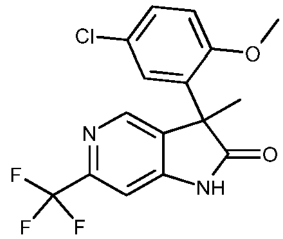

**Example 38: 3-(5-chloro-2-methoxyphenyl)-3-methyl-6-(trifluoromethyl)-1H-pyrrolo[3,2-b]pyridin-2(3H)-one (reference)**

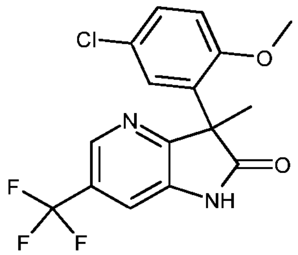

**Example 39: 6-(5-chloro-2-methoxyphenyl)-6-methyl-3-(trifluoromethyl)-4H-pyrrolo[2,3-d]isoxazol-5(6H)-one (reference)**

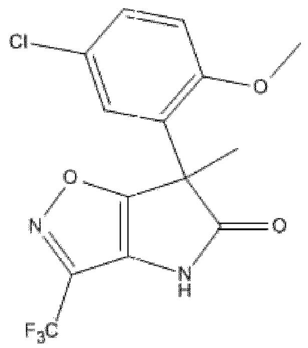

**Example 40: 6-(5-chloro-2-methoxyphenyl)-6-methyl-2-(trifluoromethyl)-4H-pyrrolo[3,2-d]oxazol-5(6H)-one (reference)**

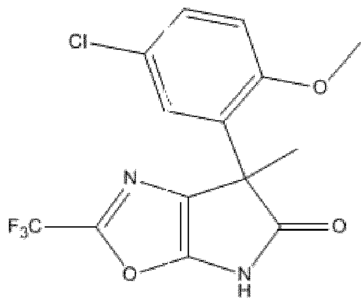

**Example 41: Assessment of BK channel stimulatory activity in a whole-cell patch clamp assay**

*Equipment*

Amplifier EPC-10, HEKA Electronics
Headstage Preamplifier EPC-10, HEKA Electronics
Software PatchMaster, HEKA Electronics

HEK-293 cell line expressing functional alpha subunits of BK channels were passaged at a confluence of 50 to 80%. Cells were seeded into 35 mm sterile culture dishes containing 2 mL culture complete medium. Cells were cultivated at a density that enables single cells (without visible connections to neighbouring cells) to be measured.

Cells were incubated at 37°C in a humidified atmosphere with 5% $CO_2$ (relative humidity about 95%). Cells were continuously maintained in and passaged in sterile culture flasks containing a 1:1 mixture of Dulbecco's modified eagle medium and nutrient mixture F-12 (D-MEM/F-12 1x, liquid, with L-glutamine) supplemented with 10% fetal bovine serum and 1.0% penicillin/streptomycin solution. The complete medium as indicated above was supplemented with 600 μg/ml geneticin and 4 μg/ml blasticidin.

The test compounds (as indicated in Table 1 below) were dissolved in DMSO to achieve a stock concentration of 10 mM and stored in deep freezer (-70°C to -90°C). All test solutions were prepared shortly prior to the electrophysiological experiments and kept at room temperature (19°C to 30°C) when in use.

*Bath Solution*

The final bath solution included the components outlined below:

Sodium Chloride 137 mM
Potassium Chloride 4 mM
Calcium Chloride 1.8 mM
Magnesium Chloride 1 mM
HEPES 10 mM
D-Glucose 10 mM
Cremophor 0.02%
pH (NaOH) 7.4

The 1x bath solution was prepared by diluting 10x bath solution without glucose and 100x glucose solution with water at least every 7 days. Both stock solutions had been prepared prior to the experimental start of the present study and stored at 1°C to 9°C (10x bath solution) or -10°C to -30° (100x glucose solution). When in use, the 1x bath solution was kept at room temperature (19°C to 30°C). When not in use, the 1x bath solution was stored at 1°C to 9°C.

*Pipette Solution*

The 1x pipette solutions included the components outlined below:

Potassium Fluoride 130 mM
Magnesium Chloride 1 mM
Calcium Chloride Total 2.8 mM
Mg-ATP 2 mM
HEPES 10 mM
EGTA 5 mM
pH (KOH) 7.2

The 1x pipette solution was thawed every day out of a frozen 1x pipette solution, stored at -10°C to -30°C. When in use, the 1x pipette solutions were filled into the patch-pipettes and kept at room temperature (19°C to 30°C).

*Voltage Protocol*

The 35 mm culture dishes upon which cells were seeded at a density allowing single cells to be recorded were placed on the dish holder of the microscope and continuously perfused (at approximately 1 mL/min) with the bath solution described. All solutions applied to cells including the pipette solution were maintained at room temperature (19°C to 30°C). After formation of a Gigaohm seal between the patch electrodes and an individual cell (pipette resistance range: 2.0 MΩ to 7.0 MΩ; seal resistance range: >1 GΩ) the cell membrane across the pipette tip was ruptured to assure electrical access to the cell interior (whole-cell patch configuration). In case the quality of the seal was poor, the process of seal formation was repeated with a different cell and a new pipette. As soon as a stable seal could be established currents were stimulated with a ramp protocol. Cells were depolarized from a holding potential of -80 mV to +50 mV within 350 ms and immediately at the end of the ramp, the cell was kept depolarized for another 50 ms at +50 mV. This protocol was run with a frequency of 0.5 Hz. Once control recordings had been accomplished, cells were continuously perfused with a bath solution containing the test compounds as detailed in Table 1 below. During wash-in of the test compounds the voltage protocol indicated above was run continuously until the steadystate level was reached.

*Data Analysis*

Values (in pA/nA) of the current amplitudes were generated for each voltage step. The recorded current amplitudes at the steady state level of current modulation (+50 mV) were compared to those from control conditions measured in the pre-treatment phase of the same cell with SigmaPlot. The amount of current modulation was calculated as percentage of control. Data from at least 2 individual experiments were collected and the corresponding mean values and standard errors calculated.

*Results*

The results of this study are summarized in the following Table 1:

**Table 1:** Mean stimulation of BK channel activity at +50 mV by different test compounds (whole-cell patch-clamp assay in stably transfected HEK-293 cells).

| **Compound** | **Mean stimulation of BK channel activity at +50 mV** | **SEM** | **n** | **Concentration** |
|---|---|---|---|---|
| Example 1 | 204.09% | 16.49% | (n=4) | 3 μM |
| BMS-204352 (reference) | 216.59% | 23.00% | (n=3) | 3 μM |

These results demonstrate that the compounds of the present invention, including in particular the compound of Example 1, exhibit a potent stimulating effect on BK channel activity, which is comparable to the effect of the highly potent but metabolically unstable reference compound BMS-204352 (MaxiPost™). This potent stimulating effect, in combination with an improved side effect profile, renders the compounds according to the present invention particularly advantageous for use in therapy, including as a chronic medication for the treatment or prevention of fragile X associated disorders, such as fragile X syndrome.

**Example 42: BK Syncropatch Electrophysiology**

HEK cells stably expressing human BK (also referred to as "KCa1.1" or KCNM1 isoform 1) channels were cultured in Ham's F-12 media supplemented with 10% Fetal Bovine Serum, 1X MEM non-essential amino acids, and 400 μg/ml G418 at 37 °C in 5% $CO_2$.

On the day of Syncropatch 384 PE (Nanion Technologies) electrophysiology testing of compounds, cells were harvested and prepared as follows. Cells were washed (1X) in Dulbecco's Phosphate Buffered Saline for approximately 30 seconds. 2 ml of cold TRP LE express was added and swirled around to cover the bottom of the flask and allowed to sit on the cells for about 3 minutes at 28 °C (approximately 90% of the cells were lifted by light tapping of the flask). 8 ml of cold media (F12 HAM's) was added to dilute the TRP LE. Cells were triturated until a single cell suspension was achieved and the cells were transferred to a 15 mL conical tube. Cell count was performed. Cells were centrifuged for 2 minutes at 800 rpm. All media was aspirated off and cells were re-suspended in 1 mL of Earle's balanced salt solution. Cells were diluted with Earle's Balanced Salt Solution (EBSS) to a concentration of 0.75 X $10^6$/ml and placed into the "cell hotel" on the deck of the SyncroPatch at 10 °C for ~35 minutes to recover. The cell suspension was dispensed into each well of a 384-well SyncroPatch chip by the onboard pipettor at the beginning of each SyncroPatch assay.

Test agents were dissolved in DMSO to give 10 mM stocks (DMSO final concentration 0.1%). Negative (0.1% DMSO) and positive (30 μM NS-1619) controls were included in each test run to assess pharmacological responsiveness.

Compound effects on BK (KCa1.1) channels were assayed using a voltage protocol in which cells were voltage-clamped at a holding potential of -80 mV. Currents were activated with a 190 ms voltage ramp from -100 mV to +60 mV. There were three 1 sec voltage steps to either -40 mV, -20 mV or 0 mV. There was a 15 second start-to-start interval between successive sweeps. Mean current amplitudes were measured for each of the voltage steps in control conditions and in the presence of test agent.

To evaluate test agent effects on BK (KCa1.1) currents, HEK-293 cells stably expressing human BK (KCa1.1) were added to each well of a Nanion Syncropatch 384 well recording plate with a 4 hole/well configuration in EBSS and then a seal enhancer buffer to form electrically tight seals. Sealed cells were washed with external recording extracellular buffer two to three times while intracellular side of each well was perfused with intracellular buffer solution prior to commencement of test agent evaluation. Compound evaluation comprised running the voltage protocol for ~ 1 minute in regular extracellular recording buffer, followed by a 10 minute application of test agent, control compound or vehicle. This was followed by 5 minute administration of reference BK (KCa1.1) activator NS-1619 (30 μM) and a 2 minute administration of the reference BK (KCa1.1) inhibitor paxilline (3 μM). Magnitude of test agent effect was determined by the following equation:

$$\%\,\text{Effect} = \frac{[\text{Maximal Current Amplitude Test Agent} - \text{Basal Current Amplitude}]}{[\text{Maximal Current Amplitude NS1619} - \text{Basal Current Amplitude}]} \times 100$$

The results thus obtained are summarized in the following Table 2:

**Table 2:** Effects of test compounds on BK channel activity (as determined by Syncropatch electrophysiology testing in stably transfected HEK-293 cells).

| Compound | Effect (%) | Concentration (μM) |
|---|---|---|
| Example 1 | 61 | 50 |
| Example 2 | 46 | 50 |
| Example 3 | 39 | 50 |
| Example 4 | 60 | 50 |
| Example 5 | 45 | 50 |
| Example 6 | 89 | 50 |
| Example 7 | 49 | 25 |
| Example 8 | 74 | 50 |
| Example 9 | 69 | 25 |
| Example 10 | 27 | 3 |
| Example 11 | 65 | 50 |
| Example 12 | 42 | 50 |

(continued)

| Compound | Effect (%) | Concentration (μM) |
|---|---|---|
| Example 13 | 50 | 50 |
| Example 14 | 31 | 50 |
| Example 15 | 27 | 50 |
| Example 17 | 20 | 25 |
| Example 18 | 30 | 13 |
| Example 19 | 24 | 50 |
| Example 20 | 19 | 50 |
| Example 21 | 47 | 13 |
| Example 22 | 56 | 50 |
| Example 23 | 37 | 50 |

These results show that the compounds of the invention exert a potent stimulating effect on BK channel activity, which makes these compounds suitable for use in therapy, particularly for the therapy of fragile X associated disorders, such as fragile X syndrome.

## Claims

**1.** A compound of formula (I)

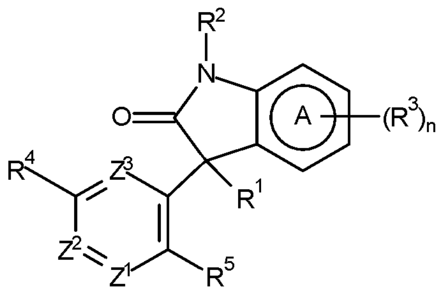

(I)

wherein:

$R^1$ is selected from $C_{1-5}$ alkyl, $C_{2-5}$ alkenyl, $C_{2-5}$ alkynyl, -($C_{0-4}$ alkylene)-CN, -($C_{1-4}$ alkylene)-COOH, -($C_{0-4}$ alkylene)-COO-($C_{1-5}$ alkyl), -($C_{1-4}$ alkylene)-O-CO-($C_{1-5}$ alkyl), -($C_{0-4}$ alkylene)-CO-($C_{1-5}$ alkyl), -($C_{0-4}$ alkylene)-CO-$NH_2$, -($C_{0-4}$ alkylene)-CO-NH($C_{1-5}$ alkyl), and -($C_{0-4}$ alkylene)-CO-N($C_{1-5}$ alkyl)($C_{1-5}$ alkyl), and $R^5$ is a group $R^{5A}$;
$R^2$ is hydrogen;
ring A is phenyl which is fused to the ring containing -CO-$NR^2$-;
each $R^3$ is independently halogen or $C_{1-5}$ haloalkyl;
n is an integer of 0 to 4;
one of the ring atoms $Z^1$, $Z^2$ and $Z^3$ is C(-$R^6$) or N, and the other two of said ring atoms are each C(-$R^6$);
$R^4$ is selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl),

$-(C_{0-4}$ alkylene)-CO-N($C_{1-5}$ alkyl)($C_{1-5}$ alkyl), $-(C_{0-4}$ alkylene)-NH-CO-($C_{1-5}$ alkyl), $-(C_{0-4}$ alkylene)-N($C_{1-5}$ alkyl)-CO-($C_{1-5}$ alkyl), $-(C_{0-4}$ alkylene)-NH-CO-O-($C_{1-5}$ alkyl), $-(C_{0-4}$ alkylene)-N($C_{1-5}$ alkyl)-CO-O-($C_{1-5}$ alkyl), $-(C_{0-4}$ alkylene)-O-CO-NH-($C_{1-5}$ alkyl), $-(C_{0-4}$ alkylene)-O-CO-N($C_{1-5}$ alkyl)-($C_{1-5}$ alkyl), $-(C_{0-4}$ alkylene)-$SO_2$-$NH_2$, $-(C_{0-4}$ alkylene)-$SO_2$-NH($C_{1-5}$ alkyl), $-(C_{0-4}$ alkylene)-$SO_2$-N($C_{1-5}$ alkyl)($C_{1-5}$ alkyl), $-(C_{0-4}$ alkylene)-NH-$SO_2$-($C_{1-5}$ alkyl), $-(C_{0-4}$ alkylene)-N($C_{1-5}$ alkyl)-$SO_2$-($C_{1-5}$ alkyl), $-(C_{0-4}$ alkylene)-$SO_2$-($C_{1-5}$ alkyl), $-(C_{0-4}$ alkylene)-SO-($C_{1-5}$ alkyl), $-(C_{0-4}$ alkylene)-carbocyclyl, and $-(C_{0-4}$ alkylene)-heterocyclyl, wherein the carbocyclyl moiety in said $-(C_{0-4}$ alkylene)-carbocyclyl and the heterocyclyl moiety in said $-(C_{0-4}$ alkylene)-heterocyclyl are each optionally substituted with one or more groups $R^{Cyc}$;

$R^{5A}$ is selected from hydrogen, $C_{1-5}$ alkyl, $C_{2-5}$ alkenyl, $C_{2-5}$ alkynyl, $-(C_{0-4}$ alkylene)-OH, $-(C_{0-4}$ alkylene)-O($C_{1-5}$ alkyl), $-(C_{0-4}$ alkylene)-O($C_{1-5}$ alkylene)-OH, $-(C_{0-4}$ alkylene)-O($C_{1-5}$ alkylene)-O($C_{1-5}$ alkyl), $-(C_{0-4}$ alkylene)-SH, $-(C_{0-4}$ alkylene)-S($C_{1-5}$ alkyl), $-(C_{0-4}$ alkylene)-$NH_2$, $-(C_{0-4}$ alkylene)-NH($C_{1-5}$ alkyl), $-(C_{0-4}$ alkylene)-N($C_{1-5}$ alkyl)($C_{1-5}$ alkyl), $-(C_{0-4}$ alkylene)-NH-OH, $-(C_{0-4}$ alkylene)-N($C_{1-5}$ alkyl)-OH, $-(C_{0-4}$ alkylene)-NH-O($C_{1-5}$ alkyl), $-(C_{0-4}$ alkylene)-N($C_{1-5}$ alkyl)-O($C_{1-5}$ alkyl), halogen, $C_{1-5}$ haloalkyl, $-(C_{0-4}$ alkylene)-O-($C_{1-5}$ haloalkyl), $-(C_{0-4}$ alkylene)-CN, $-(C_{0-4}$ alkylene)-CHO, $-(C_{0-4}$ alkylene)-CO-($C_{1-5}$ alkyl), $-(C_{0-4}$ alkylene)-COOH, $-(C_{0-4}$ alkylene)-CO-O-($C_{1-5}$ alkyl), $-(C_{0-4}$ alkylene)-O-CO-($C_{1-5}$ alkyl), $-(C_{0-4}$ alkylene)-CO-$NH_2$, $-(C_{0-4}$ alkylene)-CO-NH($C_{1-5}$ alkyl), $-(C_{0-4}$ alkylene)-CO-N($C_{1-5}$ alkyl)($C_{1-5}$ alkyl), $-(C_{0-4}$ alkylene)-NH-CO-($C_{1-5}$ alkyl), $-(C_{0-4}$ alkylene)-N($C_{1-5}$ alkyl)-CO-($C_{1-5}$ alkyl), $-(C_{0-4}$ alkylene)-NH-CO-O-($C_{1-5}$ alkyl), $-(C_{0-4}$ alkylene)-N($C_{1-5}$ alkyl)-CO-O-($C_{1-5}$ alkyl), $-(C_{0-4}$ alkylene)-O-CO-NH-($C_{1-5}$ alkyl), $-(C_{0-4}$ alkylene)-O-CO-N($C_{1-5}$ alkyl)-($C_{1-5}$ alkyl), $-(C_{0-4}$ alkylene)-$SO_2$-$NH_2$, $-(C_{0-4}$ alkylene)-$SO_2$-NH($C_{1-5}$ alkyl), $-(C_{0-4}$ alkylene)-$SO_2$-N($C_{1-5}$ alkyl)($C_{1-5}$ alkyl), $-(C_{0-4}$ alkylene)-NH-$SO_2$-($C_{1-5}$ alkyl), $-(C_{0-4}$ alkylene)-N($C_{1-5}$ alkyl)-$SO_2$-($C_{1-5}$ alkyl), $-(C_{0-4}$ alkylene)-$SO_2$-($C_{1-5}$ alkyl), $-(C_{0-4}$ alkylene)-SO-($C_{1-5}$ alkyl), $-(C_{0-4}$ alkylene)-carbocyclyl, and $-(C_{0-4}$ alkylene)-heterocyclyl, wherein the carbocyclyl moiety in said $-(C_{0-4}$ alkylene)-carbocyclyl and the heterocyclyl moiety in said $-(C_{0-4}$ alkylene)-heterocyclyl are each optionally substituted with one or more groups $R^{Cyc}$;

each $R^6$ is independently selected from hydrogen, $C_{1-5}$ alkyl, $C_{2-5}$ alkenyl, $C_{2-5}$ alkynyl, $-(C_{0-4}$ alkylene)-OH, $-(C_{0-4}$ alkylene)-O($C_{1-5}$ alkyl), $-(C_{0-4}$ alkylene)-O($C_{1-5}$ alkylene)-OH, $-(C_{0-4}$ alkylene)-O($C_{1-5}$ alkylene)-O($C_{1-5}$ alkyl), $-(C_{0-4}$ alkylene)-SH, $-(C_{0-4}$ alkylene)-S($C_{1-5}$ alkyl), $-(C_{0-4}$ alkylene)-$NH_2$, $-(C_{0-4}$ alkylene)-NH($C_{1-5}$ alkyl), $-(C_{0-4}$ alkylene)-N($C_{1-5}$ alkyl)($C_{1-5}$ alkyl), $-(C_{0-4}$ alkylene)-NH-OH, $-(C_{0-4}$ alkylene)-N($C_{1-5}$ alkyl)-OH, $-(C_{0-4}$ alkylene)-NH-O($C_{1-5}$ alkyl), $-(C_{0-4}$ alkylene)-N($C_{1-5}$ alkyl)-O($C_{1-5}$ alkyl), halogen, $C_{1-5}$ haloalkyl, $-(C_{0-4}$ alkylene)-O-($C_{1-5}$ haloalkyl), $-(C_{0-4}$ alkylene)-CN, $-(C_{0-4}$ alkylene)-CHO, $-(C_{0-4}$ alkylene)-CO-($C_{1-5}$ alkyl), $-(C_{0-4}$ alkylene)-COOH, $-(C_{0-4}$ alkylene)-CO-O-($C_{1-5}$ alkyl), $-(C_{0-4}$ alkylene)-O-CO-($C_{1-5}$ alkyl), $-(C_{0-4}$ alkylene)-CO-$NH_2$, $-(C_{0-4}$ alkylene)-CO-NH($C_{1-5}$ alkyl), $-(C_{0-4}$ alkylene)-CO-N($C_{1-5}$ alkyl)($C_{1-5}$ alkyl), $-(C_{0-4}$ alkylene)-NH-CO-($C_{1-5}$ alkyl), $-(C_{0-4}$ alkylene)-N($C_{1-5}$ alkyl)-CO-($C_{1-5}$ alkyl), $-(C_{0-4}$ alkylene)-NH-CO-O-($C_{1-5}$ alkyl), $-(C_{0-4}$ alkylene)-N($C_{1-5}$ alkyl)-CO-O-($C_{1-5}$ alkyl), $-(C_{0-4}$ alkylene)-O-CO-NH-($C_{1-5}$ alkyl), $-(C_{0-4}$ alkylene)-O-CO-N($C_{1-5}$ alkyl)-($C_{1-5}$ alkyl), $-(C_{0-4}$ alkylene)-$SO_2$-$NH_2$, $-(C_{0-4}$ alkylene)-$SO_2$-NH($C_{1-5}$ alkyl), $-(C_{0-4}$ alkylene)-$SO_2$-N($C_{1-5}$ alkyl)($C_{1-5}$ alkyl), $-(C_{0-4}$ alkylene)-NH-$SO_2$-($C_{1-5}$ alkyl), $-(C_{0-4}$ alkylene)-N($C_{1-5}$ alkyl)-$SO_2$-($C_{1-5}$ alkyl), $-(C_{0-4}$ alkylene)-$SO_2$-($C_{1-5}$ alkyl), $-(C_{0-4}$ alkylene)-SO-($C_{1-5}$ alkyl), $-(C_{0-4}$ alkylene)-carbocyclyl, and $-(C_{0-4}$ alkylene)-heterocyclyl, wherein the carbocyclyl moiety in said $-(C_{0-4}$ alkylene)-carbocyclyl and the heterocyclyl moiety in said $-(C_{0-4}$ alkylene)-heterocyclyl are each optionally substituted with one or more groups $R^{Cyc}$; and

each $R^{Cyc}$ is independently selected from $C_{1-5}$ alkyl, $C_{2-5}$ alkenyl, $C_{2-5}$ alkynyl, $-(C_{0-4}$ alkylene)-OH, $-(C_{0-4}$ alkylene)-O($C_{1-5}$ alkyl), $-(C_{0-4}$ alkylene)-O($C_{1-5}$ alkylene)-OH, $-(C_{0-4}$ alkylene)-O($C_{1-5}$ alkylene)-O($C_{1-5}$ alkyl), $-(C_{0-4}$ alkylene)-SH, $-(C_{0-4}$ alkylene)-S($C_{1-5}$ alkyl), $-(C_{0-4}$ alkylene)-$NH_2$, $-(C_{0-4}$ alkylene)-NH($C_{1-5}$ alkyl), $-(C_{0-4}$ alkylene)-N($C_{1-5}$ alkyl)($C_{1-5}$ alkyl), $-(C_{0-4}$ alkylene)-NH-OH, $-(C_{0-4}$ alkylene)-N($C_{1-5}$ alkyl)-OH, $-(C_{0-4}$ alkylene)-NH-O($C_{1-5}$ alkyl), $-(C_{0-4}$ alkylene)-N($C_{1-5}$ alkyl)-O($C_{1-5}$ alkyl), halogen, $C_{1-5}$ haloalkyl, $-(C_{0-4}$ alkylene)-O-($C_{1-5}$ haloalkyl), $-(C_{0-4}$ alkylene)-CN, $-(C_{0-4}$ alkylene)-CHO, $-(C_{0-4}$ alkylene)-CO-($C_{1-5}$ alkyl), $-(C_{0-4}$ alkylene)-COOH, $-(C_{0-4}$ alkylene)-CO-O-($C_{1-5}$ alkyl), $-(C_{0-4}$ alkylene)-O-CO-($C_{1-5}$ alkyl), $-(C_{0-4}$ alkylene)-CO-$NH_2$, $-(C_{0-4}$ alkylene)-CO-NH($C_{1-5}$ alkyl), $-(C_{0-4}$ alkylene)-CO-N($C_{1-5}$ alkyl)($C_{1-5}$ alkyl), $-(C_{0-4}$ alkylene)-NH-CO-($C_{1-5}$ alkyl), $-(C_{0-4}$ alkylene)-N($C_{1-5}$ alkyl)-CO-($C_{1-5}$ alkyl), $-(C_{0-4}$ alkylene)-NH-CO-O-($C_{1-5}$ alkyl), $-(C_{0-4}$ alkylene)-N($C_{1-5}$ alkyl)-CO-O-($C_{1-5}$ alkyl), $-(C_{0-4}$ alkylene)-O-CO-NH-($C_{1-5}$ alkyl), $-(C_{0-4}$ alkylene)-O-CO-N($C_{1-5}$ alkyl)-($C_{1-5}$ alkyl), $-(C_{0-4}$ alkylene)-$SO_2$-$NH_2$, $-(C_{0-4}$ alkylene)-$SO_2$-NH($C_{1-5}$ alkyl), $-(C_{0-4}$ alkylene)-$SO_2$-N($C_{1-5}$ alkyl)($C_{1-5}$ alkyl), $-(C_{0-4}$ alkylene)-NH-$SO_2$-($C_{1-5}$ alkyl), $-(C_{0-4}$ alkylene)-N($C_{1-5}$ alkyl)-$SO_2$-($C_{1-5}$ alkyl), $-(C_{0-4}$ alkylene)-$SO_2$-($C_{1-5}$ alkyl), and $-(C_{0-4}$ alkylene)-SO-($C_{1-5}$ alkyl);

or a pharmaceutically acceptable salt or solvate thereof;

for use in the treatment or prevention of a fragile X associated disorder.

**2.** The compound for use according to claim 1, wherein $R^1$ is $C_{1-5}$ alkyl.

**3.** The compound for use according to claim 1 or 2, wherein $R^6$ is -O($C_{1-5}$ alkyl) or -OH.

**4.** The compound for use according to any one of claims 1 to 3, wherein $Z^1$, $Z^2$ and $Z^3$ are each C(-$R^6$).

**5.** The compound for use according to any one of claims 1 to 4, wherein:

(i) $R^4$ is halogen, -$CF_3$, or -CN, preferably wherein $R^4$ is -Cl; and/or
(ii) each $R^6$ is independently selected from hydrogen, $C_{1-5}$ alkyl, -O($C_{1-5}$ alkyl), halogen, $C_{1-5}$ haloalkyl, -O-($C_{1-5}$ haloalkyl), and -CN, preferably wherein each $R^6$ is hydrogen.

**6.** The compound for use according to claim 1, wherein said compound is selected from:

3-(5-chloro-2-methoxyphenyl)-3-methyl-6-(trifluoromethyl)indolin-2-one;
3-(5-chloro-2-hydroxy-phenyl)-3-methyl-6-(trifluoromethyl)indolin-2-one;
3-(5-chloro-2-methoxyphenyl)-3-ethyl-6-(trifluoromethyl)indolin-2-one;
3-(5-chloro-2-methoxyphenyl)-3-propyl-6-(trifluoromethyl)indolin-2-one;
2-(3-(5-chloro-2-methoxyphenyl)-2-oxo-6-(trifluoromethyl)indolin-3-yl)acetonitrile;
3-allyl-3-(5-chloro-2-methoxyphenyl)-6-(trifluoromethyl)indolin-2-one;
2-(3-(5-chloro-2-methoxyphenyl)-2-oxo-6-(trifluoromethyl)indolin-3-yl)acetic acid;
3-(2-chloro-5-methoxy-4-pyridyl)-3-methyl-6-(trifluoromethyl)indolin-2-one;
3-(3-methoxy-4-pyridyl)-3-methyl-6-(trifluoromethyl)indolin-2-one;
3-methyl-3-(4-pyridyl)-6-(trifluoromethyl)indolin-2-one;
3-(2-chloro-4-pyridyl)-3-methyl-6-(trifluoromethyl)indolin-2-one;
3-[2-methoxy-5-(1-methylimidazol-2-yl)phenyl]-3-methyl-6-(trifluoromethyl)indolin-2-one;
3-(5-chloro-2-methoxy-phenyl)-6-(difluoromethyl)-3-methyl-indolin-2-one;
3-(5-chloro-2-methoxy-3-pyridyl)-3-methyl-6-(trifluoromethyl)indolin-2-one;
6-chloro-3-(5-chloro-2-methoxyphenyl)-3-methylindolin-2-one;
6-chloro-3-(2-chloro-5-methoxypyridin-4-yl)-3-methylindolin-2-one;
3-(6-chloro-3-methoxypyridin-2-yl)-3-methyl-6-(trifluoromethyl)indolin-2-one;
6-chloro-3-(5-chloro-2-methoxypyridin-3-yl)-3-methylindolin-2-one;
6-chloro-3-(2-methoxy-5-(1-methyl-1H-imidazol-2-yl)phenyl)-3-methylindolin-2-one;
6-chloro-3-(6-chloro-3-methoxypyridin-2-yl)-3-methylindolin-2-one;
6-chloro-3-(2-methoxy-5-(1-methyl-1H-imidazol-2-yl)pyridin-3-yl)-3-methylindolin-2-one;
6-chloro-3-(5-methoxy-2-(1-methyl-1H-imidazol-2-yl)pyridin-4-yl)-3-methylindolin-2-one;
or a pharmaceutically acceptable salt or solvate thereof;
preferably wherein said compound is 3-(5-chloro-2-methoxyphenyl)-3-methyl-6-(trifluoromethyl)indolin-2-one or a pharmaceutically acceptable salt or solvate thereof;
more preferably wherein said compound is (3R)-3-(5-chloro-2-methoxyphenyl)-3-methyl-6-(trifluoromethyl)indolin-2-one or a pharmaceutically acceptable salt or solvate thereof.

**7.** The compound for use according to any one of claims 1 to 6, wherein the fragile X associated disorder is selected from fragile X syndrome, fragile X-associated tremor/ataxia syndrome, fragile X-associated primary ovarian insufficiency, fragile X-associated polycystic ovarian syndrome, and fragile XE-associated mental retardation.

**8.** The compound for use according to any one of claims 1 to 7, wherein the fragile X associated disorder is fragile X syndrome.

**9.** *In vitro* use of a compound as defined in any one of claims 1 to 6 as a maxi-K potassium channel opener.

**10.** A compound of formula (Ia)

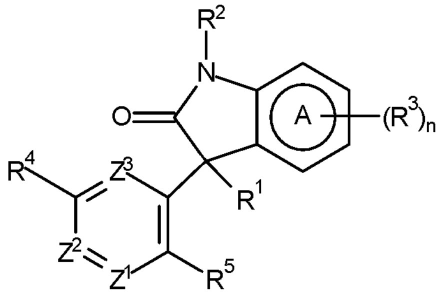

(Ia)

wherein:

$R^1$ is selected from $C_{1-5}$ alkyl, $C_{2-5}$ alkenyl, $C_{2-5}$ alkynyl, -($C_{0-4}$ alkylene)-CN, -($C_{1-4}$ alkylene)-COOH, -($C_{0-4}$ alkylene)-COO-($C_{1-5}$ alkyl), -($C_{1-4}$ alkylene)-O-CO-($C_{1-5}$ alkyl), -($C_{0-4}$ alkylene)-CO-($C_{1-5}$ alkyl), -($C_{0-4}$ alkylene)-CO-$NH_2$, -($C_{0-4}$ alkylene)-CO-NH($C_{1-5}$ alkyl), and -($C_{0-4}$ alkylene)-CO-N($C_{1-5}$ alkyl)($C_{1-5}$ alkyl), and $R^5$ is a group $R^{5A}$;

$R^2$ is hydrogen;

ring A is phenyl which is fused to the ring containing -CO-$NR^2$-;

each $R^3$ is independently halogen or $C_{1-5}$ haloalkyl;

n is 1, 2, 3 or 4;

one of the ring atoms $Z^1$, $Z^2$ and $Z^3$ is C(-$R^6$) or N, and the other two of said ring atoms are each C(-$R^6$);

$R^4$ is selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), -($C_{0-4}$ alkylene)-SO-($C_{1-5}$ alkyl), -($C_{0-4}$ alkylene)-carbocyclyl, and -($C_{0-4}$ alkylene)-heterocyclyl, wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more groups $R^{Cyc}$;

$R^{5A}$ is selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), -($C_{0-4}$ alkylene)-SO-($C_{1-5}$ alkyl), -($C_{0-4}$ alkylene)-carbocyclyl, and -($C_{0-4}$ alkylene)-heterocyclyl, wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more groups $R^{Cyc}$;

each $R^6$ is independently selected from hydrogen, $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}

alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), -($C_{0-4}$ alkylene)-SO-($C_{1-5}$ alkyl), -($C_{0-4}$ alkylene)-carbocyclyl, and -($C_{0-4}$ alkylene)-heterocyclyl, wherein the carbocyclyl moiety in said -($C_{0-4}$ alkylene)-carbocyclyl and the heterocyclyl moiety in said -($C_{0-4}$ alkylene)-heterocyclyl are each optionally substituted with one or more groups $R^{Cyc}$; and

each $R^{Cyc}$ is independently selected from $C_{1-5}$ alkyl, $C_{2-5}$ alkenyl, $C_{2-5}$ alkynyl, -($C_{0-4}$ alkylene)-OH, -($C_{0-4}$ alkylene)-O($C_{1-5}$ alkyl), -($C_{0-4}$ alkylene)-O($C_{1-5}$ alkylene)-OH, -($C_{0-4}$ alkylene)-O($C_{1-5}$ alkylene)-O($C_{1-5}$ alkyl), -($C_{0-4}$ alkylene)-SH, -($C_{0-4}$ alkylene)-S($C_{1-5}$ alkyl), -($C_{0-4}$ alkylene)-$NH_2$, -($C_{0-4}$ alkylene)-NH($C_{1-5}$ alkyl), -($C_{0-4}$ alkylene)-N($C_{1-5}$ alkyl)($C_{1-5}$ alkyl), -($C_{0-4}$ alkylene)-NH-OH, -($C_{0-4}$ alkylene)-N($C_{1-5}$ alkyl)-OH, -($C_{0-4}$ alkylene)-NH-O($C_{1-5}$ alkyl), -($C_{0-4}$ alkylene)-N($C_{1-5}$ alkyl)-O($C_{1-5}$ alkyl), halogen, $C_{1-5}$ haloalkyl, -($C_{0-4}$ alkylene)-O-($C_{1-5}$ haloalkyl), -($C_{0-4}$ alkylene)-CN, -($C_{0-4}$ alkylene)-CHO, -($C_{0-4}$ alkylene)-CO-($C_{1-5}$ alkyl), -($C_{0-4}$ alkylene)-COOH, -($C_{0-4}$ alkylene)-CO-O-($C_{1-5}$ alkyl), -($C_{0-4}$ alkylene)-O-CO-($C_{1-5}$ alkyl), -($C_{0-4}$ alkylene)-CO-$NH_2$, -($C_{0-4}$ alkylene)-CO-NH($C_{1-5}$ alkyl), -($C_{0-4}$ alkylene)-CO-N($C_{1-5}$ alkyl)($C_{1-5}$ alkyl), -($C_{0-4}$ alkylene)-NH-CO-($C_{1-5}$ alkyl), -($C_{0-4}$ alkylene)-N($C_{1-5}$ alkyl)-CO-($C_{1-5}$ alkyl), -($C_{0-4}$ alkylene)-NH-CO-O-($C_{1-5}$ alkyl), -($C_{0-4}$ alkylene)-N($C_{1-5}$ alkyl)-CO-O-($C_{1-5}$ alkyl), -($C_{0-4}$ alkylene)-O-CO-NH-($C_{1-5}$ alkyl), -($C_{0-4}$ alkylene)-O-CO-N($C_{1-5}$ alkyl)-($C_{1-5}$ alkyl), -($C_{0-4}$ alkylene)-$SO_2$-$NH_2$, -($C_{0-4}$ alkylene)-$SO_2$-NH($C_{1-5}$ alkyl), -($C_{0-4}$ alkylene)-$SO_2$-N($C_{1-5}$ alkyl)($C_{1-5}$ alkyl), -($C_{0-4}$ alkylene)-NH-$SO_2$-($C_{1-5}$ alkyl), -($C_{0-4}$ alkylene)-N($C_{1-5}$ alkyl)-$SO_2$-($C_{1-5}$ alkyl), -($C_{0-4}$ alkylene)-$SO_2$-($C_{1-5}$ alkyl), and -($C_{0-4}$ alkylene)-SO-($C_{1-5}$ alkyl);

or a pharmaceutically acceptable salt or solvate thereof;

wherein if n is 1 or 2, then at least one group $R^3$ is different from halogen.

**11.** The compound of claim 10, wherein $R^1$ is $C_{1-5}$ alkyl.

**12.** The compound of claim 10 or 11, wherein $R^5$ is -O($C_{1-5}$ alkyl) or -OH.

**13.** The compound of any one of claims 10 to 12, wherein $Z^1$, $Z^2$ and $Z^3$ are each C(-$R^6$).

**14.** The compound of any one of claims 10 to 13, wherein:

(i) $R^4$ is halogen, -$CF_3$, or -CN, preferably wherein $R^4$ is -Cl; and/or
(ii) each $R^6$ is independently selected from hydrogen, $C_{1-5}$ alkyl, -O($C_{1-5}$ alkyl), halogen, $C_{1-5}$ haloalkyl, -O-($C_{1-5}$ haloalkyl), and -CN, preferably wherein each $R^6$ is hydrogen.

**15.** The compound of claim 10, wherein said compound is selected from:

3-(5-chloro-2-methoxyphenyl)-3-methyl-6-(trifluoromethyl)indolin-2-one;
3-(5-chloro-2-hydroxy-phenyl)-3-methyl-6-(trifluoromethyl)indolin-2-one;
3-(5-chloro-2-methoxyphenyl)-3-ethyl-6-(trifluoromethyl)indolin-2-one;
3-(5-chloro-2-methoxyphenyl)-3-propyl-6-(trifluoromethyl)indolin-2-one;
2-(3-(5-chloro-2-methoxyphenyl)-2-oxo-6-(trifluoromethyl)indolin-3-yl)acetonitrile;
3-allyl-3-(5-chloro-2-methoxyphenyl)-6-(trifluoromethyl)indolin-2-one;
2-(3-(5-chloro-2-methoxyphenyl)-2-oxo-6-(trifluoromethyl)indolin-3-yl)acetic acid;
3-(2-chloro-5-methoxy-4-pyridyl)-3-methyl-6-(trifluoromethyl)indolin-2-one;
3-(3-methoxy-4-pyridyl)-3-methyl-6-(trifluoromethyl)indolin-2-one;
3-methyl-3-(4-pyridyl)-6-(trifluoromethyl)indolin-2-one;
3-(2-chloro-4-pyridyl)-3-methyl-6-(trifluoromethyl)indolin-2-one;
3-[2-methoxy-5-(1-methylimidazol-2-yl)phenyl]-3-methyl-6-(trifluoromethyl)indolin-2-one;
3-(5-chloro-2-methoxy-phenyl)-6-(difluoromethyl)-3-methyl-indolin-2-one;
3-(5-chloro-2-methoxy-3-pyridyl)-3-methyl-6-(trifluoromethyl)indolin-2-one;
3-(6-chloro-3-methoxypyridin-2-yl)-3-methyl-6-(trifluoromethyl)indolin-2-one;
or a pharmaceutically acceptable salt or solvate thereof;
preferably wherein said compound is 3-(5-chloro-2-methoxyphenyl)-3-methyl-6-(trifluoromethyl)indolin-2-one or

a pharmaceutically acceptable salt or solvate thereof;
more preferably wherein said compound is (3R)-3-(5-chloro-2-methoxyphenyl)-3-methyl-6-(trifluoromethyl)indolin-2-one or a pharmaceutically acceptable salt or solvate thereof.

**16.** The compound of any one of claims 10 to 15 for use as a medicament.

**17.** The compound of any one of claims 10 to 15 for use in the treatment or prevention of a disease/disorder selected from stroke, ischemia reperfusion injury, ischemic heart disease, hypertension, myocardial infarction, allergic rhinitis, asthma, chronic obstructive pulmonary disease, multiple sclerosis, spasticity, tremor, a muscular disorder, dyskinesia, bladder dysfunction, overactive bladder, urinary incontinence, irritable bowel syndrome, faecal incontinence, constipation, gastro-oesophageal reflux disorder, impaired gastrointenstinal passage, detrusor underactivity, erectile dysfunction, opioid-induced respiratory depression, anxiety, an anxiety disorder, sensory processing disorder, autism, an autism spectrum disorder, a social or pragmatic communication disorder, attention deficit hyperactivity disorder, social phobia, intellectual disability, pain, epilepsy, an epilepsy and/or seizure disorder, Dravet syndrome, generalized epilepsy and paroxysmal dyskinesia, temporal lobe epilepsy, psychosis, schizophrenia, negative symptoms of schizophrenia, cognitive impairment in schizophrenia, Phelan-McDermid syndrome, Rett syndrome, Pitt-Hopkins syndrome, 16p11.2 deletion syndrome, Williams-Beuren syndrome, open-angle ocular hypertension, glaucoma, tinnitus, diabetic retinopathy, tocolysis, migraine, Liang-Wang syndrome, Smith-Lemli-Opitz syndrome, Angelman syndrome, and Hutchinson-Gilford progeria syndrome.

## Patentansprüche

**1.** Eine Verbindung der Formel (I)

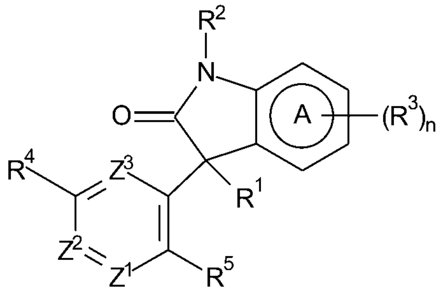

(I)

wobei:

$R^1$ ausgewählt ist aus $C_{1-5}$-Alkyl, $C_{2-5}$-Alkenyl, $C_{2-5}$-Alkinyl, -($C_{0-4}$-Alkylen)-CN, -($C_{1-4}$-Alkylen)-COOH, -($C_{0-4}$-Alkylen)-COO-($C_{1-5}$-Alkyl), -($C_{1-4}$-Alkylen)-O-CO-($C_{1-5}$-Alkyl), -($C_{0-4}$-Alkylen)-CO-($C_{1-5}$-Alkyl), -($C_{0-4}$-Alkylen)-CO-$NH_2$, -($C_{0-4}$-Alkylen)-CO-NH($C_{1-5}$-Alkyl) und -($C_{0-4}$-Alkylen)-CO-N($C_{1-5}$-Alkyl)($C_{1-5}$-Alkyl), und $R^5$ eine Gruppe $R^{5A}$ ist;
$R^2$ Wasserstoff ist;
Ring A Phenyl ist, das an den -CO-$NR^2$- enthaltenden Ring kondensiert ist;
jedes $R^3$ unabhängig Halogen oder $C_{1-5}$-Halogenalkyl ist;
n eine ganze Zahl von 0 bis 4 ist;
eines der Ringatome $Z^1$, $Z^2$ und $Z^3$ gleich C(-$R^6$) oder N ist und die anderen beiden der Ringatome jeweils C(-$R^6$) sind;
$R^4$ ausgewählt ist aus Wasserstoff, $C_{1-5}$-Alkyl, $C_{2-5}$-Alkenyl, $C_{2-5}$-Alkinyl, -($C_{0-4}$-Alkylen)-OH, -($C_{0-4}$-Alkylen)-O($C_{1-5}$-Alkyl), -($C_{0-4}$-Alkylen)-O($C_{1-5}$-Alkylen)-OH, -($C_{0-4}$-Alkylen)-O($C_{1-5}$-Alkylen)-O($C_{1-5}$-Alkyl), -($C_{0-4}$-Alkylen)-SH, -($C_{0-4}$-Alkylen)-S($C_{1-5}$-Alkyl), -($C_{0-4}$-Alkylen)-$NH_2$, -($C_{0-4}$-Alkylen)-NH($C_{1-5}$-Alkyl), -($C_{0-4}$-Alkylen)-N($C_{1-5}$-Alkyl)($C_{1-5}$-Alkyl), -($C_{0-4}$-Alkylen)-NH-OH, -($C_{0-4}$-Alkylen)-N($C_{1-5}$-Alkyl)-OH, -($C_{0-4}$-Alkylen)-NH-O($C_{1-5}$-Alkyl), -($C_{0-4}$-Alkylen)-N($C_{1-5}$-Alkyl)-O($C_{1-5}$-Alkyl), Halogen, $C_{1-5}$-Halogenalkyl, -($C_{0-4}$-Alkylen)-O-($C_{1-5}$-Halogenalkyl), -($C_{0-4}$-Alkylen)-CN, -($C_{0-4}$-Alkylen)-CHO, -($C_{0-4}$-Alkylen)-CO-($C_{1-5}$-Alkyl), -($C_{0-4}$-Alkylen)-COOH, -($C_{0-4}$-Alkylen)-CO-O-($C_{1-5}$-Alkyl), -($C_{0-4}$-Alkylen)-O-CO-($C_{1-5}$-Alkyl), -($C_{0-4}$-Alkylen)-CO-$NH_2$, -($C_{0-4}$-Alkylen)-CO-NH($C_{1-5}$-Alkyl), -($C_{0-4}$-Alkylen)-CO-N($C_{1-5}$-Alkyl)($C_{1-5}$-Alkyl), -($C_{0-4}$-Alkylen)-NH-

CO-($C_{1-5}$-Alkyl), -($C_{0-4}$-Alkylen)-N($C_{1-5}$-Alkyl)-CO-($C_{1-5}$-Alkyl), -($C_{0-4}$-Alkylen)-NH-CO-O-($C_{1-5}$-Alkyl), -($C_{0-4}$-Alkylen)-N($C_{1-5}$-Alkyl)-CO-O-($C_{1-5}$-Alkyl), -($C_{0-4}$-Alkylen)-O-CO-NH-($C_{1-5}$-Alkyl), -($C_{0-4}$-Alkylen)-O-CO-N($C_{1-5}$-Alkyl)-($C_{1-5}$-Alkyl), -($C_{0-4}$-Alkylen)-$SO_2$-$NH_2$, -($C_{0-4}$-Alkylen)-$SO_2$-NH($C_{1-5}$-Alkyl), -($C_{0-4}$-Alkylen)-$SO_2$-N($C_{1-5}$-Alkyl)($C_{1-5}$-Alkyl), -($C_{0-4}$-Alkylen)-NH-$SO_2$-($C_{1-5}$-Alkyl), -($C_{0-4}$-Alkylen)-N($C_{1-5}$-Alkyl)-$SO_2$-($C_{1-5}$-Alkyl), -($C_{0-4}$-Alkylen)-$SO_2$-($C_{1-5}$-Alkyl), -($C_{0-4}$-Alkylen)-SO-($C_{1-5}$-Alkyl), -($C_{0-4}$-Alkylen)-Carbocyclyl und -($C_{0-4}$-Alkylen)-Heterocyclyl, wobei der Carbocyclylrest in dem -($C_{0-4}$-Alkylen)-Carbocyclyl und der Heterocyclylrest in dem -($C_{0-4}$-Alkylen)-Heterocyclyl jeweils gegebenenfalls mit einer oder mehreren Gruppen $R^{Cyc}$ substituiert sind;

$R^{5A}$ ausgewählt ist aus Wasserstoff, $C_{1-5}$-Alkyl, $C_{2-5}$-Alkenyl, $C_{2-5}$-Alkinyl, -($C_{0-4}$-Alkylen)-OH, -($C_{0-4}$-Alkylen)-O($C_{1-5}$-Alkyl), -($C_{0-4}$-Alkylen)-O($C_{1-5}$-Alkylen)-OH, -($C_{0-4}$-Alkylen)-O($C_{1-5}$-Alkylen)-O($C_{1-5}$-Alkyl), -($C_{0-4}$-Alkylen)-SH, -($C_{0-4}$-Alkylen)-S($C_{1-5}$-Alkyl), -($C_{0-4}$-Alkylen)-$NH_2$, -($C_{0-4}$-Alkylen)-NH($C_{1-5}$-Alkyl), -($C_{0-4}$-Alkylen)-N($C_{1-5}$-Alkyl)($C_{1-5}$-Alkyl), -($C_{0-4}$-Alkylen)-NH-OH, -($C_{0-4}$-Alkylen)-N($C_{1-5}$-Alkyl)-OH, -($C_{0-4}$-Alkylen)-NH-O($C_{1-5}$-Alkyl), -($C_{0-4}$-Alkylen)-N($C_{1-5}$-Alkyl)-O($C_{1-5}$-Alkyl), Halogen, $C_{1-5}$-Halogenalkyl, -($C_{0-4}$-Alkylen)-O-($C_{1-5}$-Halogenalkyl), -($C_{0-4}$-Alkylen)-CN, -($C_{0-4}$-Alkylen)-CHO, -($C_{0-4}$-Alkylen)-CO-($C_{1-5}$-Alkyl), -($C_{0-4}$-Alkylen)-COOH, -($C_{0-4}$-Alkylen)-CO-O-($C_{1-5}$-Alkyl), -($C_{0-4}$-Alkylen)-O-CO-($C_{1-5}$-Alkyl), -($C_{0-4}$-Alkylen)-CO-$NH_2$, -($C_{0-4}$-Alkylen)-CO-NH($C_{1-5}$-Alkyl), -($C_{0-4}$-Alkylen)-CO-N($C_{1-5}$-Alkyl)($C_{1-5}$-Alkyl), -($C_{0-4}$-Alkylen)-NH-CO-($C_{1-5}$-Alkyl), -($C_{0-4}$-Alkylen)-N($C_{1-5}$-Alkyl)-CO-($C_{1-5}$-Alkyl), -($C_{0-4}$-Alkylen)-NH-CO-O-($C_{1-5}$-Alkyl), -($C_{0-4}$-Alkylen)-N($C_{1-5}$-Alkyl)-CO-O-($C_{1-5}$-Alkyl), -($C_{0-4}$-Alkylen)-O-CO-NH-($C_{1-5}$-Alkyl), -($C_{0-4}$-Alkylen)-O-CO-N($C_{1-5}$-Alkyl)-($C_{1-5}$-Alkyl), -($C_{0-4}$-Alkylen)-$SO_2$-$NH_2$, -($C_{0-4}$-Alkylen)-$SO_2$-NH($C_{1-5}$-Alkyl), -($C_{0-4}$-Alkylen)-$SO_2$-N($C_{1-5}$-Alkyl)($C_{1-5}$-Alkyl), -($C_{0-4}$-Alkylen)-NH-$SO_2$-($C_{1-5}$-Alkyl), -($C_{0-4}$-Alkylen)-N($C_{1-5}$-Alkyl)-$SO_2$-($C_{1-5}$-Alkyl), -($C_{0-4}$-Alkylen)-$SO_2$-($C_{1-5}$-Alkyl), -($C_{0-4}$-Alkylen)-SO-($C_{1-5}$-Alkyl), -($C_{0-4}$-Alkylen)-Carbocyclyl und -($C_{0-4}$-Alkylen)-Heterocyclyl, wobei der Carbocyclylrest in dem -($C_{0-4}$-Alkylen)-Carbocyclyl und der Heterocyclylrest in dem -($C_{0-4}$-Alkylen)-Heterocyclyl jeweils gegebenenfalls mit einer oder mehreren Gruppen $R^{Cyc}$ substituiert sind;

jedes $R^6$ unabhängig ausgewählt ist aus Wasserstoff, $C_{1-5}$-Alkyl, $C_{2-5}$-Alkenyl, $C_{2-5}$-Alkinyl, -($C_{0-4}$-Alkylen)-OH, -($C_{0-4}$-Alkylen)-O($C_{1-5}$-Alkyl), -($C_{0-4}$-Alkylen)-O($C_{1-5}$-Alkylen)-OH, -($C_{0-4}$-Alkylen)-O($C_{1-5}$-Alkylen)-O($C_{1-5}$-Alkyl), -($C_{0-4}$-Alkylen)-SH, -($C_{0-4}$-Alkylen)-S($C_{1-5}$-Alkyl), -($C_{0-4}$-Alkylen)-$NH_2$, -($C_{0-4}$-Alkylen)-NH($C_{1-5}$-Alkyl), -($C_{0-4}$-Alkylen)-N($C_{1-5}$-Alkyl)($C_{1-5}$-Alkyl), -($C_{0-4}$-Alkylen)-NH-OH, -($C_{0-4}$-Alkylen)-N($C_{1-5}$-Alkyl)-OH, -($C_{0-4}$-Alkylen)-NH-O($C_{1-5}$-Alkyl), -($C_{0-4}$-Alkylen)-N($C_{1-5}$-Alkyl)-O($C_{1-5}$-Alkyl), Halogen, $C_{1-5}$-Halogenalkyl, -($C_{0-4}$-Alkylen)-O-($C_{1-5}$-Halogenalkyl), -($C_{0-4}$-Alkylen)-CN, -($C_{0-4}$-Alkylen)-CHO, -($C_{0-4}$-Alkylen)-CO-($C_{1-5}$-Alkyl), -($C_{0-4}$-Alkylen)-COOH, -($C_{0-4}$-Alkylen)-CO-O-($C_{1-5}$-Alkyl), -($C_{0-4}$-Alkylen)-O-CO-($C_{1-5}$-Alkyl), -($C_{0-4}$-Alkylen)-CO-$NH_2$, -($C_{0-4}$-Alkylen)-CO-NH($C_{1-5}$-Alkyl), -($C_{0-4}$-Alkylen)-CO-N($C_{1-5}$-Alkyl)($C_{1-5}$-Alkyl), -($C_{0-4}$-Alkylen)-NH-CO-($C_{1-5}$-Alkyl), -($C_{0-4}$-Alkylen)-N($C_{1-5}$-Alkyl)-CO-($C_{1-5}$-Alkyl), -($C_{0-4}$-Alkylen)-NH-CO-O-($C_{1-5}$-Alkyl), -($C_{0-4}$-Alkylen)-N($C_{1-5}$-Alkyl)-CO-O-($C_{1-5}$-Alkyl), -($C_{0-4}$-Alkylen)-O-CO-NH-($C_{1-5}$-Alkyl), -($C_{0-4}$-Alkylen)-O-CO-N($C_{1-5}$-Alkyl)-($C_{1-5}$-Alkyl), -($C_{0-4}$-Alkylen)-$SO_2$-$NH_2$, -($C_{0-4}$-Alkylen)-$SO_2$-NH($C_{1-5}$-Alkyl), -($C_{0-4}$-Alkylen)-$SO_2$-N($C_{1-5}$-Alkyl)($C_{1-5}$-Alkyl), -($C_{0-4}$-Alkylen)-NH-$SO_2$-($C_{1-5}$-Alkyl), -($C_{0-4}$-Alkylen)-N($C_{1-5}$-Alkyl)-$SO_2$-($C_{1-5}$-Alkyl), -($C_{0-4}$-Alkylen)-$SO_2$-($C_{1-5}$-Alkyl), -($C_{0-4}$-Alkylen)-SO-($C_{1-5}$-Alkyl), -($C_{0-4}$-Alkylen)-Carbocyclyl und -($C_{0-4}$-Alkylen)-Heterocyclyl, wobei der Carbocyclylrest in dem -($C_{0-4}$-Alkylen)-Carbocyclyl und der Heterocyclylrest in dem -($C_{0-4}$-Alkylen)-Heterocyclyl jeweils gegebenenfalls mit einer oder mehreren Gruppen $R^{Cyc}$ substituiert sind; und

jedes $R^{Cyc}$ unabhängig ausgewählt ist aus $C_{1-5}$-Alkyl, $C_{2-5}$-Alkenyl, $C_{2-5}$-Alkinyl, -($C_{0-4}$-Alkylen)-OH, -($C_{0-4}$-Alkylen)-O($C_{1-5}$-Alkyl), -($C_{0-4}$-Alkylen)-O($C_{1-5}$-Alkylen)-OH, -($C_{0-4}$-Alkylen)-O($C_{1-5}$-Alkylen)-O($C_{1-5}$-Alkyl), -($C_{0-4}$-Alkylen)-SH, -($C_{0-4}$-Alkylen)-S($C_{1-5}$-Alkyl), -($C_{0-4}$-Alkylen)-$NH_2$, -($C_{0-4}$-Alkylen)-NH($C_{1-5}$-Alkyl), -($C_{0-4}$-Alkylen)-N($C_{1-5}$-Alkyl)($C_{1-5}$-Alkyl), -($C_{0-4}$-Alkylen)-NH-OH, -($C_{0-4}$-Alkylen)-N($C_{1-5}$-Alkyl)-OH, -($C_{0-4}$-Alkylen)-NH-O($C_{1-5}$-Alkyl), -($C_{0-4}$-Alkylen)-N($C_{1-5}$-Alkyl)-O($C_{1-5}$-Alkyl), Halogen, $C_{1-5}$-Halogenalkyl, -($C_{0-4}$-Alkylen)-O-($C_{1-5}$-Halogenalkyl), -($C_{0-4}$-Alkylen)-CN, -($C_{0-4}$-Alkylen)-CHO, -($C_{0-4}$-Alkylen)-CO-($C_{1-5}$-Alkyl), -($C_{0-4}$-Alkylen)-COOH, -($C_{0-4}$-Alkylen)-CO-O-($C_{1-5}$-Alkyl), -($C_{0-4}$-Alkylen)-O-CO-($C_{1-5}$-Alkyl), -($C_{0-4}$-Alkylen)-CO-$NH_2$, -($C_{0-4}$-Alkylen)-CO-NH($C_{1-5}$-Alkyl), -($C_{0-4}$-Alkylen)-CO-N($C_{1-5}$-Alkyl)($C_{1-5}$-Alkyl), -($C_{0-4}$-Alkylen)-NH-CO-($C_{1-5}$-Alkyl), -($C_{0-4}$-Alkylen)-N($C_{1-5}$-Alkyl)-CO-($C_{1-5}$-Alkyl), -($C_{0-4}$-Alkylen)-NH-CO-O-($C_{1-5}$-Alkyl), -($C_{0-4}$-Alkylen)-N($C_{1-5}$-Alkyl)-CO-O-($C_{1-5}$-Alkyl), -($C_{0-4}$-Alkylen)-O-CO-NH-($C_{1-5}$-Alkyl), -($C_{0-4}$-Alkylen)-O-CO-N($C_{1-5}$-Alkyl)-($C_{1-5}$-Alkyl), -($C_{0-4}$-Alkylen)-$SO_2$-$NH_2$, -($C_{0-4}$-Alkylen)-$SO_2$-NH($C_{1-5}$-Alkyl), -($C_{0-4}$-Alkylen)-$SO_2$-N($C_{1-5}$-Alkyl)($C_{1-5}$-Alkyl), -($C_{0-4}$-Alkylen)-NH-$SO_2$-($C_{1-5}$-Alkyl), -($C_{0-4}$-Alkylen)-N($C_{1-5}$-Alkyl)-$SO_2$-($C_{1-5}$-Alkyl), -($C_{0-4}$-Alkylen)-$SO_2$-($C_{1-5}$-Alkyl) und -($C_{0-4}$-Alkylen)-SO-($C_{1-5}$-Alkyl);

oder ein pharmazeutisch verträgliches Salz oder Solvat davon;

zur Verwendung bei der Behandlung oder Vorbeugung einer mit Fragilem-X assoziierten Störung.

**2.** Die Verbindung zur Verwendung nach Anspruch 1, wobei $R^1$ $C_{1-5}$-Alkyl ist.

**3.** Die Verbindung zur Verwendung nach Anspruch 1 oder 2, wobei $R^5$ -O($C_{1-5}$-Alkyl) oder -OH ist.

**4.** Die Verbindung zur Verwendung nach einem der Ansprüche 1 bis 3, wobei $Z^1$, $Z^2$ und $Z^3$ jeweils C(-$R^6$) sind.

**5.** Die Verbindung zur Verwendung nach einem der Ansprüche 1 bis 4, wobei:

(i) $R^4$ Halogen, -$CF_3$ oder -CN ist, vorzugsweise wobei $R^4$ -Cl ist; und/oder
(ii) jedes $R^6$ unabhängig ausgewählt ist aus Wasserstoff, $C_{1-5}$-Alkyl, -O($C_{1-5}$-Alkyl), Halogen, $C_{1-5}$-Halogenalkyl, -O-($C_{1-5}$-Halogenalkyl) und -CN, vorzugsweise wobei jedes $R^6$ Wasserstoff ist.

**6.** Die Verbindung zur Verwendung nach Anspruch 1, wobei die Verbindung ausgewählt ist aus:

3-(5-Chlor-2-methoxyphenyl)-3-methyl-6-(trifluormethyl)indolin-2-on;
3-(5-Chlor-2-hydroxyphenyl)-3-methyl-6-(trifluormethyl)indolin-2-on;
3-(5-Chlor-2-methoxyphenyl)-3-ethyl-6-(trifluormethyl)indolin-2-on;
3-(5-Chlor-2-methoxyphenyl)-3-propyl-6-(trifluormethyl)indolin-2-on;
2-(3-(5-Chlor-2-methoxyphenyl)-2-oxo-6-(trifluormethyl)indolin-3-yl)acetonitril;
3-Allyl-3-(5-chlor-2-methoxyphenyl)-6-(trifluormethyl)indolin-2-on;
2-(3-(5-Chlor-2-methoxyphenyl)-2-oxo-6-(trifluormethyl)indolin-3-yl)essigsäure;
3-(2-Chlor-5-methoxy-4-pyridyl)-3-methyl-6-(trifluormethyl)indolin-2-on;
3-(3-Methoxy-4-pyridyl)-3-methyl-6-(trifluormethyl)indolin-2-on;
3-Methyl-3-(4-pyridyl)-6-(trifluormethyl)indolin-2-on;
3-(2-Chlor-4-pyridyl)-3-methyl-6-(trifluormethyl)indolin-2-on;
3-[2-Methoxy-5-(1-methylimidazol-2-yl)phenyl]-3-methyl-6-(trifluormethyl)indolin-2-on;
3-(5-Chlor-2-methoxyphenyl)-6-(difluormethyl)-3-methylindolin-2-on;
3-(5-Chlor-2-methoxy-3-pyridyl)-3-methyl-6-(trifluormethyl)indolin-2-on;
6-Chlor-3-(5-chlor-2-methoxyphenyl)-3-methylindolin-2-on;
6-Chlor-3-(2-chlor-5-methoxypyridin-4-yl)-3-methylindolin-2-on;
3-(6-Chlor-3-methoxypyridin-2-yl)-3-methyl-6-(trifluormethyl)indolin-2-on;
6-Chlor-3-(5-chlor-2-methoxypyridin-3-yl)-3-methylindolin-2-on;
6-Chlor-3-(2-methoxy-5-(1-methyl-1H-imidazol-2-yl)phenyl)-3-methylindolin-2-on;
6-Chlor-3-(6-chlor-3-methoxypyridin-2-yl)-3-methylindolin-2-on;
6-Chlor-3-(2-methoxy-5-(1-methyl-1H-imidazol-2-yl)pyridin-3-yl)-3-methylindolin-2-on;
6-Chlor-3-(5-methoxy-2-(1-methyl-1H-imidazol-2-yl)pyridin-4-yl)-3-methylindolin-2-on;
oder ein pharmazeutisch verträgliches Salz oder Solvat davon;
vorzugsweise wobei die Verbindung 3-(5-Chlor-2-methoxyphenyl)-3-methyl-6-(trifluormethyl)indolin-2-on oder ein pharmazeutisch verträgliches Salz oder Solvat davon ist;
stärker bevorzugt wobei die Verbindung (3R)-3-(5-Chlor-2-methoxyphenyl)-3-methyl-6-(trifluormethyl)indolin-2-on oder ein pharmazeutisch verträgliches Salz oder Solvat davon ist.

**7.** Die Verbindung zur Verwendung nach einem der Ansprüche 1 bis 6, wobei die mit Fragilem-X assoziierte Störung ausgewählt ist aus dem Fragilen-X-Syndrom, dem mit Fragilem-X assoziierten Tremor/Ataxie-Syndrom, der mit Fragilem-X assoziierten primären Ovarialinsuffizienz, dem mit Fragilem-X assoziierten polyzystischen Ovarialsyndrom und der mit Fragilem-XE assoziierten geistigen Behinderung.

**8.** Die Verbindung zur Verwendung nach einem der Ansprüche 1 bis 7, wobei die mit Fragilem X assoziierte Störung das Fragile-X-Syndrom ist.

**9.** In-vitro-Verwendung einer Verbindung wie in einem der Ansprüche 1 bis 6 definiert als Maxi-K-Kaliumkanalöffner.

**10.** Eine Verbindung der Formel (la)

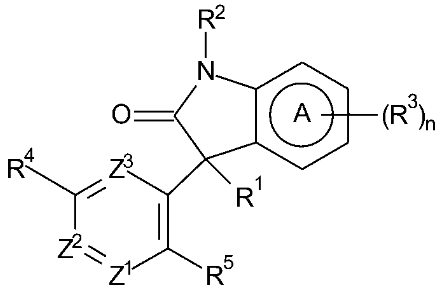

(Ia)

wobei:

$R^1$ ausgewählt ist aus $C_{1-5}$-Alkyl, $C_{2-5}$-Alkenyl, $C_{2-5}$-Alkinyl, -($C_{0-4}$-Alkylen)-CN, -($C_{1-4}$-Alkylen)-COOH, -($C_{0-4}$-Alkylen)-COO-($C_{1-5}$-Alkyl), -($C_{1-4}$-Alkylen)-O-CO-($C_{1-5}$-Alkyl), -($C_{0-4}$-Alkylen)-CO-($C_{1-5}$-Alkyl), -($C_{0-4}$-Alkylen)-CO-$NH_2$, -($C_{0-4}$-Alkylen)-CO-NH($C_{1-5}$-Alkyl) und -($C_{0-4}$-Alkylen)-CO-N($C_{1-5}$-Alkyl)($C_{1-5}$-Alkyl) und $R^5$ eine Gruppe $R^{5A}$ ist;
$R^2$ Wasserstoff ist;
Ring A Phenyl ist, das an den -CO-$NR^2$- enthaltenden Ring kondensiert ist;
jedes $R^3$ unabhängig Halogen oder $C_{1-5}$-Halogenalkyl ist;
n gleich 1, 2, 3 oder 4 ist;
eines der Ringatome $Z^1$, $Z^2$ und $Z^3$ gleich C(-$R^6$) oder N ist und die anderen beiden der Ringatome jeweils C(-$R^6$) sind;
$R^4$ ausgewählt ist aus Wasserstoff, $C_{1-5}$-Alkyl, $C_{2-5}$-Alkenyl, $C_{2-5}$-Alkinyl, -($C_{0-4}$-Alkylen)-OH, -($C_{0-4}$-Alkylen)-O($C_{1-5}$-Alkyl), -($C_{0-4}$-Alkylen)-O($C_{1-5}$-Alkylen)-OH, -($C_{0-4}$-Alkylen)-O($C_{1-5}$-Alkylen)-O($C_{1-5}$-Alkyl), -($C_{0-4}$-Alkylen)-SH, -($C_{0-4}$-Alkylen)-S($C_{1-5}$-Alkyl), -($C_{0-4}$-Alkylen)-$NH_2$, -($C_{0-4}$-Alkylen)-NH($C_{1-5}$-Alkyl), -($C_{0-4}$-Alkylen)-N($C_{1-5}$-Alkyl)($C_{1-5}$-Alkyl), -($C_{0-4}$-Alkylen)-NH-OH, -($C_{0-4}$-Alkylen)-N($C_{1-5}$-Alkyl)-OH, -($C_{0-4}$-Alkylen)-NH-O($C_{1-5}$-Alkyl), -($C_{0-4}$-Alkylen)-N($C_{1-5}$-Alkyl)-O($C_{1-5}$-Alkyl), Halogen, $C_{1-5}$-Halogenalkyl, -($C_{0-4}$-Alkylen)-O-($C_{1-5}$-Halogenalkyl), -($C_{0-4}$-Alkylen)-CN, -($C_{0-4}$-Alkylen)-CHO, -($C_{0-4}$-Alkylen)-CO-($C_{1-5}$-Alkyl), -($C_{0-4}$-Alkylen)-COOH, -($C_{0-4}$-Alkylen)-CO-O-($C_{1-5}$-Alkyl), -($C_{0-4}$-Alkylen)-O-CO-($C_{1-5}$-Alkyl), -($C_{0-4}$-Alkylen)-CO-$NH_2$, -($C_{0-4}$-Alkylen)-CO-NH($C_{1-5}$-Alkyl), -($C_{0-4}$-Alkylen)-CO-N($C_{1-5}$-Alkyl)($C_{1-5}$-Alkyl), -($C_{0-4}$-Alkylen)-NH-CO-($C_{1-5}$-Alkyl), -($C_{0-4}$-Alkylen)-N($C_{1-5}$-Alkyl)-CO-($C_{1-5}$-Alkyl), -($C_{0-4}$-Alkylen)-NH-CO-O-($C_{1-5}$-Alkyl), -($C_{0-4}$-Alkylen)-N($C_{1-5}$-Alkyl)-CO-O-($C_{1-5}$-Alkyl), -($C_{0-4}$-Alkylen)-O-CO-NH-($C_{1-5}$-Alkyl), -($C_{0-4}$-Alkylen)-O-CO-N($C_{1-5}$-Alkyl)-($C_{1-5}$-Alkyl), -($C_{0-4}$-Alkylen)-$SO_2$-$NH_2$, -($C_{0-4}$-Alkylen)-$SO_2$-NH($C_{1-5}$-Alkyl), -($C_{0-4}$-Alkylen)-$SO_2$-N($C_{1-5}$-Alkyl)($C_{1-5}$-Alkyl), -($C_{0-4}$-Alkylen)-NH-$SO_2$-($C_{1-5}$-Alkyl), -($C_{0-4}$-Alkylen)-N($C_{1-5}$-Alkyl)-$SO_2$-($C_{1-5}$-Alkyl), -($C_{0-4}$-Alkylen)-$SO_2$-($C_{1-5}$-Alkyl), -($C_{0-4}$-Alkylen)-SO-($C_{1-5}$-Alkyl), -($C_{0-4}$-Alkylen)-Carbocyclyl und -($C_{0-4}$-Alkylen)-Heterocyclyl, wobei der Carbocyclylrest in dem -($C_{0-4}$-Alkylen)-Carbocyclyl und der Heterocyclylrest in dem -($C_{0-4}$-Alkylen)-Heterocyclyl jeweils gegebenenfalls mit einer oder mehreren Gruppen $R^{Cyc}$ substituiert sind;
$R^{5A}$ ausgewählt ist aus Wasserstoff, $C_{1-5}$-Alkyl, $C_{2-5}$-Alkenyl, $C_{2-5}$-Alkinyl, -($C_{0-4}$-Alkylen)-OH, -($C_{0-4}$-Alkylen)-O($C_{1-5}$-Alkyl), -($C_{0-4}$-Alkylen)-O($C_{1-5}$-Alkylen)-OH, -($C_{0-4}$-Alkylen)-O($C_{1-5}$-Alkylen)-O($C_{1-5}$-Alkyl), -($C_{0-4}$-Alkylen)-SH, -($C_{0-4}$-Alkylen)-S($C_{1-5}$-Alkyl), -($C_{0-4}$-Alkylen)-$NH_2$, -($C_{0-4}$-Alkylen)-NH($C_{1-5}$-Alkyl), -($C_{0-4}$-Alkylen)-N($C_{1-5}$-Alkyl)($C_{1-5}$-Alkyl), -($C_{0-4}$-Alkylen)-NH-OH, -($C_{0-4}$-Alkylen)-N($C_{1-5}$-Alkyl)-OH, -($C_{0-4}$-Alkylen)-NH-O($C_{1-5}$-Alkyl), -($C_{0-4}$-Alkylen)-N($C_{1-5}$-Alkyl)-O($C_{1-5}$-Alkyl), Halogen, $C_{1-5}$-Halogenalkyl, -($C_{0-4}$-Alkylen)-O-($C_{1-5}$-Halogenalkyl), -($C_{0-4}$-Alkylen)-CN, -($C_{0-4}$-Alkylen)-CHO, -($C_{0-4}$-Alkylen)-CO-($C_{1-5}$-Alkyl), -($C_{0-4}$-Alkylen)-COOH, -($C_{0-4}$-Alkylen)-CO-O-($C_{1-5}$-Alkyl), -($C_{0-4}$-Alkylen)-O-CO-($C_{1-5}$-Alkyl), -($C_{0-4}$-Alkylen)-CO-$NH_2$, -($C_{0-4}$-Alkylen)-CO-NH($C_{1-5}$-Alkyl), -($C_{0-4}$-Alkylen)-CO-N($C_{1-5}$-Alkyl)($C_{1-5}$-Alkyl), -($C_{0-4}$-Alkylen)-NH-CO-($C_{1-5}$-Alkyl), -($C_{0-4}$-Alkylen)-N($C_{1-5}$-Alkyl)-CO-($C_{1-5}$-Alkyl), -($C_{0-4}$-Alkylen)-NH-CO-O-($C_{1-5}$-Alkyl), -($C_{0-4}$-Alkylen)-N($C_{1-5}$-Alkyl)-CO-O-($C_{1-5}$-Alkyl), -($C_{0-4}$-Alkylen)-O-CO-NH-($C_{1-5}$-Alkyl), -($C_{0-4}$-Alkylen)-O-CO-N($C_{1-5}$-Alkyl)-($C_{1-5}$-Alkyl), -($C_{0-4}$-Alkylen)-$SO_2$-$NH_2$, -($C_{0-4}$-Alkylen)-$SO_2$-NH($C_{1-5}$-Alkyl), -($C_{0-4}$-Alkylen)-$SO_2$-N($C_{1-5}$-Alkyl)($C_{1-5}$-Alkyl), -($C_{0-4}$-Alkylen)-NH-$SO_2$-($C_{1-5}$-Alkyl), -($C_{0-4}$-Alkylen)-N($C_{1-5}$-Alkyl)-$SO_2$-($C_{1-5}$-Alkyl), -($C_{0-4}$-Alkylen)-$SO_2$-($C_{1-5}$-Alkyl), -($C_{0-4}$-Alkylen)-SO-($C_{1-5}$-Alkyl), -($C_{0-4}$-Alkylen)-Carbocyclyl und -($C_{0-4}$-Alkylen)-Heterocyclyl, wobei der Carbocyclylrest in dem -($C_{0-4}$-Alkylen)-Carbocyclyl und der Heterocyclylrest in dem -($C_{0-4}$-Alkylen)-Heterocyclyl jeweils gegebenenfalls mit einer oder mehreren Gruppen $R^{Cyc}$ substituiert sind;
jedes $R^6$ unabhängig ausgewählt ist aus Wasserstoff, $C_{1-5}$-Alkyl, $C_{2-5}$-Alkenyl, $C_{2-5}$-Alkinyl, -($C_{0-4}$-Alkylen)-OH,

-($C_{0-4}$-Alkylen)-O($C_{1-5}$-Alkyl), -($C_{0-4}$-Alkylen)-O($C_{1-5}$-Alkylen)-OH, -($C_{0-4}$-Alkylen)-O($C_{1-5}$-Alkylen)-O($C_{1-5}$-Alkyl), -($C_{0-4}$-Alkylen)-SH, -($C_{0-4}$-Alkylen)-S($C_{1-5}$-Alkyl), -($C_{0-4}$-Alkylen)-$NH_2$, -($C_{0-4}$-Alkylen)-NH($C_{1-5}$-Alkyl), -($C_{0-4}$-Alkylen)-N($C_{1-5}$-Alkyl)($C_{1-5}$-Alkyl), -($C_{0-4}$-Alkylen)-NH-OH, -($C_{0-4}$-Alkylen)-N($C_{1-5}$-Alkyl)-OH, -($C_{0-4}$-Alkylen)-NH-O($C_{1-5}$-Alkyl), -($C_{0-4}$-Alkylen)-N($C_{1-5}$-Alkyl)-O($C_{1-5}$-Alkyl), Halogen, $C_{1-5}$-Halogenalkyl, -($C_{0-4}$-Alkylen)-O-($C_{1-5}$-Halogenalkyl), -($C_{0-4}$-Alkylen)-CN, -($C_{0-4}$-Alkylen)-CHO, -($C_{0-4}$-Alkylen)-CO-($C_{1-5}$-Alkyl), -($C_{0-4}$-Alkylen)-COOH, -($C_{0-4}$-Alkylen)-CO-O-($C_{1-5}$-Alkyl), -($C_{0-4}$-Alkylen)-O-CO-($C_{1-5}$-Alkyl), -($C_{0-4}$-Alkylen)-CO-$NH_2$, -($C_{0-4}$-Alkylen)-CO-NH($C_{1-5}$-Alkyl), -($C_{0-4}$-Alkylen)-CO-N($C_{1-5}$-Alkyl)($C_{1-5}$-Alkyl), -($C_{0-4}$-Alkylen)-NH-CO-($C_{1-5}$-Alkyl), -($C_{0-4}$-Alkylen)-N($C_{1-5}$-Alkyl)-CO-($C_{1-5}$-Alkyl), -($C_{0-4}$-Alkylen)-NH-CO-O-($C_{1-5}$-Alkyl), -($C_{0-4}$-Alkylen)-N($C_{1-5}$-Alkyl)-CO-O-($C_{1-5}$-Alkyl), -($C_{0-4}$-Alkylen)-O-CO-NH-($C_{1-5}$-Alkyl), -($C_{0-4}$-Alkylen)-O-CO-N($C_{1-5}$-Alkyl)-($C_{1-5}$-Alkyl), -($C_{0-4}$-Alkylen)-$SO_2$-$NH_2$, -($C_{0-4}$-Alkylen)-$SO_2$-NH($C_{1-5}$-Alkyl), -($C_{0-4}$-Alkylen)-$SO_2$-N($C_{1-5}$-Alkyl)($C_{1-5}$-Alkyl), -($C_{0-4}$-Alkylen)-NH-$SO_2$-($C_{1-5}$-Alkyl), -($C_{0-4}$-Alkylen)-N($C_{1-5}$-Alkyl)-$SO_2$-($C_{1-5}$-Alkyl), -($C_{0-4}$-Alkylen)-$SO_2$-($C_{1-5}$-Alkyl), -($C_{0-4}$-Alkylen)-SO-($C_{1-5}$-Alkyl), -($C_{0-4}$-Alkylen)-Carbocyclyl und -($C_{0-4}$-Alkylen)-Heterocyclyl, wobei der Carbocyclylrest in dem -($C_{0-4}$-Alkylen)-Carbocyclyl und der Heterocyclylrest in dem -($C_{0-4}$-Alkylen)-Heterocyclyl jeweils gegebenenfalls mit einer oder mehreren Gruppen $R^{Cyc}$ substituiert sind; und

jedes $R^{Cyc}$ unabhängig ausgewählt ist aus $C_{1-5}$-Alkyl, $C_{2-5}$-Alkenyl, $C_{2-5}$-Alkinyl, -($C_{0-4}$-Alkylen)-OH, -($C_{0-4}$-Alkylen)-O($C_{1-5}$-Alkyl), -($C_{0-4}$-Alkylen)-O($C_{1-5}$-Alkylen)-OH, -($C_{0-4}$-Alkylen)-O($C_{1-5}$-Alkylen)-O($C_{1-5}$-Alkyl), -($C_{0-4}$-Alkylen)-SH, -($C_{0-4}$-Alkylen)-S($C_{1-5}$-Alkyl), -($C_{0-4}$-Alkylen)-$NH_2$, -($C_{0-4}$-Alkylen)-NH($C_{1-5}$-Alkyl), -($C_{0-4}$-Alkylen)-N($C_{1-5}$-Alkyl)($C_{1-5}$-Alkyl), -($C_{0-4}$-Alkylen)-NH-OH, -($C_{0-4}$-Alkylen)-N($C_{1-5}$-Alkyl)-OH, -($C_{0-4}$-Alkylen)-NH-O($C_{1-5}$-Alkyl), -($C_{0-4}$-Alkylen)-N($C_{1-5}$-Alkyl)-O($C_{1-5}$-Alkyl), Halogen, $C_{1-5}$-Halogenalkyl, -($C_{0-4}$-Alkylen)-O-($C_{1-5}$-Halogenalkyl), -($C_{0-4}$-Alkylen)-CN, -($C_{0-4}$-Alkylen)-CHO, -($C_{0-4}$-Alkylen)-CO-($C_{1-5}$-Alkyl), -($C_{0-4}$-Alkylen)-COOH, -($C_{0-4}$-Alkylen)-CO-O-($C_{1-5}$-Alkyl), -($C_{0-4}$-Alkylen)-O-CO-($C_{1-5}$-Alkyl), -($C_{0-4}$-Alkylen)-CO-$NH_2$, -($C_{0-4}$-Alkylen)-CO-NH($C_{1-5}$-Alkyl), -($C_{0-4}$-Alkylen)-CO-N($C_{1-5}$-Alkyl)($C_{1-5}$-Alkyl), -($C_{0-4}$-Alkylen)-NH-CO-($C_{1-5}$-Alkyl), -($C_{0-4}$-Alkylen)-N($C_{1-5}$-Alkyl)-CO-($C_{1-5}$-Alkyl), -($C_{0-4}$-Alkylen)-NH-CO-O-($C_{1-5}$-Alkyl), -($C_{0-4}$-Alkylen)-N($C_{1-5}$-Alkyl)-CO-O-($C_{1-5}$-Alkyl), -($C_{0-4}$-Alkylen)-O-CO-NH-($C_{1-5}$-Alkyl), -($C_{0-4}$-Alkylen)-O-CO-N($C_{1-5}$-Alkyl)-($C_{1-5}$-Alkyl), -($C_{0-4}$-Alkylen)-$SO_2$-$NH_2$, -($C_{0-4}$-Alkylen)-$SO_2$-NH($C_{1-5}$-Alkyl), -($C_{0-4}$-Alkylen)-$SO_2$-N($C_{1-5}$-Alkyl)($C_{1-5}$-Alkyl), -($C_{0-4}$-Alkylen)-NH-$SO_2$-($C_{1-5}$-Alkyl), -($C_{0-4}$-Alkylen)-N($C_{1-5}$-Alkyl)-$SO_2$-($C_{1-5}$-Alkyl), -($C_{0-4}$-Alkylen)-$SO_2$-($C_{1-5}$-Alkyl) und -($C_{0-4}$-Alkylen)-SO-($C_{1-5}$-Alkyl);

oder ein pharmazeutisch verträgliches Salz oder Solvat davon;

wobei, wenn n gleich 1 oder 2 ist, dann ist mindestens eine Gruppe $R^3$ von Halogen verschieden.

**11.** Die Verbindung nach Anspruch 10, wobei $R^1$ $C_{1-5}$-Alkyl ist.

**12.** Die Verbindung nach Anspruch 10 oder 11, wobei $R^5$ -O($C_{1-5}$-Alkyl) oder -OH ist.

**13.** Die Verbindung nach einem der Ansprüche 10 bis 12, wobei $Z^1$, $Z^2$ und $Z^3$ jeweils C(-$R^6$) sind.

**14.** Die Verbindung nach einem der Ansprüche 10 bis 13, wobei:

(i) $R^4$ Halogen, -$CF_3$ oder -CN ist, vorzugsweise wobei $R^4$ -Cl ist; und/oder

(ii) jedes $R^6$ unabhängig ausgewählt ist aus Wasserstoff, $C_{1-5}$-Alkyl, -O($C_{1-5}$-Alkyl), Halogen, $C_{1-5}$-Halogenalkyl, -O-($C_{1-5}$-Halogenalkyl) und -CN, vorzugsweise wobei jedes $R^6$ Wasserstoff ist.

**15.** Die Verbindung nach Anspruch 10, wobei die Verbindung ausgewählt ist aus:

3-(5-Chlor-2-methoxyphenyl)-3-methyl-6-(trifluormethyl)indolin-2-on;
3-(5-Chlor-2-hydroxyphenyl)-3-methyl-6-(trifluormethyl)indolin-2-on;
3-(5-Chlor-2-methoxyphenyl)-3-ethyl-6-(trifluormethyl)indolin-2-on;
3-(5-Chlor-2-methoxyphenyl)-3-propyl-6-(trifluormethyl)indolin-2-on;
2-(3-(5-Chlor-2-methoxyphenyl)-2-oxo-6-(trifluormethyl)indolin-3-yl)acetonitril;
3-Allyl-3-(5-chlor-2-methoxyphenyl)-6-(trifluormethyl)indolin-2-on;
2-(3-(5-Chlor-2-methoxyphenyl)-2-oxo-6-(trifluormethyl)indolin-3-yl)essigsäure;
3-(2-Chlor-5-methoxy-4-pyridyl)-3-methyl-6-(trifluormethyl)indolin-2-on;
3-(3-Methoxy-4-pyridyl)-3-methyl-6-(trifluormethyl)indolin-2-on;
3-Methyl-3-(4-pyridyl)-6-(trifluormethyl)indolin-2-on;
3-(2-Chlor-4-pyridyl)-3-methyl-6-(trifluormethyl)indolin-2-on;
3-[2-Methoxy-5-(1-methylimidazol-2-yl)phenyl]-3-methyl-6-(trifluormethyl)indolin-2-on;
3-(5-Chlor-2-methoxyphenyl)-6-(difluormethyl)-3-methylindolin-2-on;

3-(5-Chlor-2-methoxy-3-pyridyl)-3-methyl-6-(trifluormethyl)indolin-2-on;
3-(6-Chlor-3-methoxypyridin-2-yl)-3-methyl-6-(trifluormethyl)indolin-2-on;
oder ein pharmazeutisch verträgliches Salz oder Solvat davon;
vorzugsweise wobei die Verbindung 3-(5-Chlor-2-methoxyphenyl)-3-methyl-6-(trifluormethyl)indolin-2-on oder ein pharmazeutisch verträgliches Salz oder Solvat davon ist;
stärker bevorzugt wobei die Verbindung (3R)-3-(5-Chlor-2-methoxyphenyl)-3-methyl-6-(trifluormethyl)indolin-2-on oder ein pharmazeutisch verträgliches Salz oder Solvat davon ist.

**16.** Die Verbindung nach einem der Ansprüche 10 bis 15 zur Verwendung als Medikament.

**17.** Die Verbindung nach einem der Ansprüche 10 bis 15 zur Verwendung bei der Behandlung oder Vorbeugung einer Krankheit/Störung, ausgewählt aus Schlaganfall, Ischämie-Reperfusionsverletzung, ischämischer Herzkrankheit, Hypertonie, Myokardinfarkt, allergischer Rhinitis, Asthma, chronisch obstruktiver Lungenerkrankung, multipler Sklerose, Spastik, Tremor, einer Muskelerkrankung, Dyskinesie, Blasenfunktionsstörung, überaktiver Blase, Harninkontinenz, Reizdarmsyndrom, Stuhlinkontinenz, Verstopfung, gastroösophagealer Refluxkrankheit, gestörter Magen-Darm-Passage, Detrusor-Unteraktivität, erektiler Dysfunktion, opioidinduzierter Atemdepression, Angst, einer Angststörung, sensorischer Verarbeitungsstörung, Autismus, einer Autismus-Spektrum-Störung, einer sozialen oder pragmatischen Kommunikationsstörung, Aufmerksamkeitsdefizit-Hyperaktivitätsstörung, sozialer Phobie, geistiger Behinderung, Schmerz, Epilepsie, einem Epilepsie- und/oder Anfallsleiden, Dravet-Syndrom, generalisierter Epilepsie und paroxysmaler Dyskinesie, Temporallappenepilepsie, Psychose, Schizophrenie, negativen Symptomen der Schizophrenie, kognitiver Beeinträchtigung bei Schizophrenie, Phelan-McDermid-Syndrom, Rett-Syndrom, Pitt-Hopkins-Syndrom, 16p11.2-Deletionssyndrom, Williams-Beuren-Syndrom, okulärer Offenwinkel-Hypertonie, Glaukom, Tinnitus, diabetischer Retinopathie, Tokolyse, Migräne, Liang-Wang-Syndrom, Smith-Lemli-Opitz-Syndrom, Angelman-Syndrom und Hutchinson-Gilford-Progerie-Syndrom.

## Revendications

**1.** Composé de formule (I)

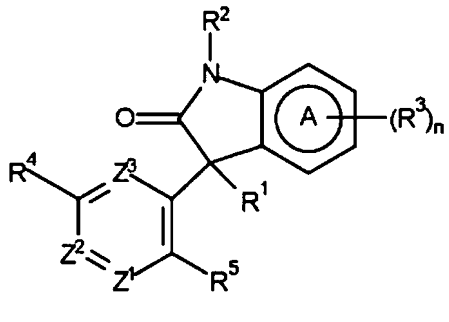

(I)

dans laquelle :

$R^1$ est choisi parmi un alkyle en $C_{1-5}$, alcényle en $C_{2-5}$, alcynyle en $C_{2-5}$, -(alkylène en $C_{0-4}$)-CN, -(alkylène en $C_{1-4}$)-COOH, -(alkylène en $C_{0-4}$)-COO-(alkyle en $C_{1-5}$), - (alkylène en $C_{1-4}$)-O-CO-(alkyle en $C_{1-5}$), -(alkylène en $C_{0-4}$)-CO-(alkyle en $C_{1-5}$), - (alkylène en $C_{0-4}$)-CO-$NH_2$, -(alkylène en $C_{0-4}$)-CO-NH(alkyle en $C_{1-5}$), et -(alkylène en $C_{0-4}$)-CO-N(alkyle en $C_{1-5}$)(alkyle en $C_{1-5}$), et $R^5$ est un groupe $R^{5A}$ ;
$R^2$ est un hydrogène ;
le cycle A est un phényle lequel est condensé au cycle contenant -CO-$NR^2$- ;
chaque $R^3$ est indépendamment un halogène ou halogénoalkyle en $C_{1-5}$ ;
n est un entier de 0 à 4 ;
un des atomes $Z^1$, $Z^2$ et $Z^3$ du cycle est C(-$R^6$) ou N, et les deux autres desdits atomes du cycle sont chacun C(-$R^6$) ;
$R^4$ est choisi parmi un hydrogène, alkyle en $C_{1-5}$, alcényle en $C_{2-5}$, alcynyle en $C_{2-5}$, -(alkylène en $C_{0-4}$)-OH, -(alkylène en $C_{0-4}$)-O(alkyle en $C_{1-5}$), -(alkylène en $C_{0-4}$)-O(alkylène en $C_{1-5}$)-OH, -(alkylène en $C_{0-4}$)-O(alkylène en $C_{1-5}$)-O(alkyle en $C_{1-5}$), - (alkylène en $C_{0-4}$)-SH, -(alkylène en $C_{0-4}$)-S(alkyle en $C_{1-5}$), -(alkylène en $C_{0-4}$)-$NH_2$,

-(alkylène en $C_{0-4}$)-NH(alkyle en $C_{1-5}$), -(alkylène en $C_{0-4}$)-N(alkyle en $C_{1-5}$)(alkyle en $C_{1-5}$), -(alkylène en $C_{0-4}$)-NH-OH, -(alkylène en $C_{0-4}$)-N(alkyle en $C_{1-5}$)-OH, - (alkylène en $C_{0-4}$)-NH-O(alkyle en $C_{1-5}$), -(alkylène en $C_{0-4}$)-N(alkyle en $C_{1-5}$)-O(alkyle en $C_{1-5}$), halogène, halogénoalkyle en $C_{1-5}$, -(alkylène en $C_{0-4}$)-O-(halogénoalkyle en $C_{1-5}$), -(alkylène en $C_{0-4}$)-CN, -(alkylène en $C_{0-4}$)-CHO, -(alkylène en $C_{0-4}$)-CO-(alkyle en $C_{1-5}$), -(alkylène en $C_{0-4}$)-COOH, -(alkylène en $C_{0-4}$)-CO-O-(alkyle en $C_{1-5}$), -(alkylène en $C_{0-4}$)-O-CO-(alkyle en $C_{1-5}$), -(alkylène en $C_{0-4}$)-CO-$NH_2$, -(alkylène en $C_{0-4}$)-CO-NH(alkyle en $C_{1-5}$), -(alkylène en $C_{0-4}$)-CO-N(alkyle en $C_{1-5}$)(alkyle en $C_{1-5}$), -(alkylène en $C_{0-4}$)-NH-CO-(alkyle en $C_{1-5}$), -(alkylène en $C_{0-4}$)-N(alkyle en $C_{1-5}$)-CO-(alkyle en $C_{1-5}$), -(alkylène en $C_{0-4}$)-NH-CO-O-(alkyle en $C_{1-5}$), -(alkylène en $C_{0-4}$)-N(alkyle en $C_{1-5}$)-CO-O-(alkyle en $C_{1-5}$), -(alkylène en $C_{0-4}$)-O-CO-NH-(alkyle en $C_{1-5}$), -(alkylène en $C_{0-4}$)-O-CO-N(alkyle en $C_{1-5}$)-(alkyle en $C_{1-5}$), -(alkylène en $C_{0-4}$)-$SO_2$-$NH_2$, -(alkylène en $C_{0-4}$)-$SO_2$-NH(alkyle en $C_{1-5}$), -(alkylène en $C_{0-4}$)-$SO_2$-N(alkyle en $C_{1-5}$)(alkyle en $C_{1-5}$), -(alkylène en $C_{0-4}$)-NH-$SO_2$-(alkyle en $C_{1-5}$), -(alkylène en $C_{0-4}$)-N(alkyle en $C_{1-5}$)-$SO_2$-(alkyle en $C_{1-5}$), -(alkylène en $C_{0-4}$)-$SO_2$-(alkyle en $C_{1-5}$), -(alkylène en $C_{0-4}$)-SO-(alkyle en $C_{1-5}$), -(alkylène en $C_{0-4}$)-carbocyclyle, et -(alkylène en $C_{0-4}$)-hétérocyclyle, où la fraction carbocyclyle dans ledit -(alkylène en $C_{0-4}$)-carbocyclyle et la fraction hétérocyclyle dans ledit -(alkylène en $C_{0-4}$)-hétérocyclyle sont chacune éventuellement substituées par un ou plusieurs groupes $R^{Cyc}$ ;

$R^{5A}$ est choisi parmi un hydrogène, alkyle en $C_{1-5}$, alcényle en $C_{2-5}$, alcynyle en $C_{2-5}$, -(alkylène en $C_{0-4}$)-OH, -(alkylène en $C_{0-4}$)-O(alkyle en $C_{1-5}$), -(alkylène en $C_{0-4}$)-O(alkylène en $C_{1-5}$)-OH, -(alkylène en $C_{0-4}$)-O(alkylène en $C_{1-5}$)-O(alkyle en $C_{1-5}$), - (alkylène en $C_{0-4}$)-SH, -(alkylène en $C_{0-4}$)-S(alkyle en $C_{1-5}$), -(alkylène en $C_{0-4}$)-$NH_2$, -(alkylène en $C_{0-4}$)-NH(alkyle en $C_{1-5}$), -(alkylène en $C_{0-4}$)-N(alkyle en $C_{1-5}$)(alkyle en $C_{1-5}$), -(alkylène en $C_{0-4}$)-NH-OH, -(alkylène en $C_{0-4}$)-N(alkyle en $C_{1-5}$)-OH, - (alkylène en $C_{0-4}$)-NH-O(alkyle en $C_{1-5}$), -(alkylène en $C_{0-4}$)-N(alkyle en $C_{1-5}$)-O(alkyle en $C_{1-5}$), halogène, halogénoalkyle en $C_{1-5}$, -(alkylène en $C_{0-4}$)-O-(halogénoalkyle en $C_{1-5}$), -(alkylène en $C_{0-4}$)-CN, -(alkylène en $C_{0-4}$)-CHO, -(alkylène en $C_{0-4}$)-CO-(alkyle en $C_{1-5}$), -(alkylène en $C_{0-4}$)-COOH, -(alkylène en $C_{0-4}$)-CO-O-(alkyle en $C_{1-5}$), -(alkylène en $C_{0-4}$)-O-CO-(alkyle en $C_{1-5}$), -(alkylène en $C_{0-4}$)-CO-$NH_2$, -(alkylène en $C_{0-4}$)-CO-NH(alkyle en $C_{1-5}$), -(alkylène en $C_{0-4}$)-CO-N(alkyle en $C_{1-5}$)(alkyle en $C_{1-5}$), -(alkylène en $C_{0-4}$)-NH-CO-(alkyle en $C_{1-5}$), -(alkylène en $C_{0-4}$)-N(alkyle en $C_{1-5}$)-CO-(alkyle en $C_{1-5}$), -(alkylène en $C_{0-4}$)-NH-CO-O-(alkyle en $C_{1-5}$), -(alkylène en $C_{0-4}$)-N(alkyle en $C_{1-5}$)-CO-O-(alkyle en $C_{1-5}$), -(alkylène en $C_{0-4}$)-O-CO-NH-(alkyle en $C_{1-5}$), -(alkylène en $C_{0-4}$)-O-CO-N(alkyle en $C_{1-5}$)-(alkyle en $C_{1-5}$), -(alkylène en $C_{0-4}$)-$SO_2$-$NH_2$, -(alkylène en $C_{0-4}$)-$SO_2$-NH(alkyle en $C_{1-5}$), -(alkylène en $C_{0-4}$)-$SO_2$-N(alkyle en $C_{1-5}$)(alkyle en $C_{1-5}$), -(alkylène en $C_{0-4}$)-NH-$SO_2$-(alkyle en $C_{1-5}$), -(alkylène en $C_{0-4}$)-N(alkyle en $C_{1-5}$)-$SO_2$-(alkyle en $C_{1-5}$), -(alkylène en $C_{0-4}$)-$SO_2$-(alkyle en $C_{1-5}$), -(alkylène en $C_{0-4}$)-SO-(alkyle en $C_{1-5}$), -(alkylène en $C_{0-4}$)-carbocyclyle, et -(alkylène en $C_{0-4}$)-hétérocyclyle, où la fraction carbocyclyle dans ledit -(alkylène en $C_{0-4}$)-carbocyclyle et la fraction hétérocyclyle dans ledit -(alkylène en $C_{0-4}$)-hétérocyclyle sont chacune éventuellement substituées par un ou plusieurs groupes $R^{Cyc}$ ;

chaque $R^6$ est choisi indépendamment parmi un hydrogène, alkyle en $C_{1-5}$, alcényle en $C_{2-5}$, alcynyle en $C_{2-5}$, -(alkylène en $C_{0-4}$)-OH, -(alkylène en $C_{0-4}$)-O(alkyle en $C_{1-5}$), -(alkylène en $C_{0-4}$)-O(alkylène en $C_{1-5}$)-OH, -(alkylène en $C_{0-4}$)-O(alkylène en $C_{1-5}$)-O(alkyle en $C_{1-5}$), -(alkylène en $C_{0-4}$)-SH, -(alkylène en $C_{0-4}$)-S(alkyle en $C_{1-5}$), - (alkylène en $C_{0-4}$)-$NH_2$, -(alkylène en $C_{0-4}$)-NH(alkyle en $C_{1-5}$), -(alkylène en $C_{0-4}$)-N(alkyle en $C_{1-5}$)(alkyle en $C_{1-5}$), -(alkylène en $C_{0-4}$)-NH-OH, -(alkylène en $C_{0-4}$)-N(alkyle en $C_{1-5}$)-OH, -(alkylène en $C_{0-4}$)-NH-O(alkyle en $C_{1-5}$), -(alkylène en $C_{0-4}$)-N(alkyle en $C_{1-5}$)-O(alkyle en $C_{1-5}$), halogène, halogénoalkyle en $C_{1-3}$, -(alkylène en $C_{0-4}$)-O-(halogénoalkyle en $C_{1-5}$), -(alkylène en $C_{0-4}$)-CN, -(alkylène en $C_{0-4}$)-CHO, - (alkylène en $C_{0-4}$)-CO-(alkyle en $C_{1-5}$), -(alkylène en $C_{0-4}$)-COOH, -(alkylène en $C_{0-4}$)-CO-O-(alkyle en $C_{1-5}$), -(alkylène en $C_{0-4}$)-O-CO-(alkyle en $C_{1-5}$), -(alkylène en $C_{0-4}$)-CO-$NH_2$, -(alkylène en $C_{0-4}$)-CO-NH(alkyle en $C_{1-5}$), -(alkylène en $C_{0-4}$)-CON(alkyle en $C_{1-5}$)(alkyle en $C_{1-5}$), -(alkylène en $C_{0-4}$)-NH-CO-(alkyle en $C_{1-5}$), - (alkylène en $C_{0-4}$)-N(alkyle en $C_{1-5}$)-CO-(alkyle en $C_{1-5}$), -(alkylène en $C_{0-4}$)-NH-CO-O-(alkyle en $C_{1-5}$), -(alkylène en $C_{0-4}$)-N(alkyle en $C_{1-5}$)-CO-O-(alkyle en $C_{1-5}$), - (alkylène en $C_{0-4}$)-O-CO-NH-(alkyle en $C_{1-5}$), -(alkylène en $C_{0-4}$)-O-CO-N(alkyle en $C_{1-5}$)-(alkyle en $C_{1-5}$), -(alkylène en $C_{0-4}$)-$SO_2$-$NH_2$, -(alkylène en $C_{0-4}$)-$SO_2$-NH(alkyle en $C_{1-5}$), -(alkylène en $C_{0-4}$)-$SO_2$-N(alkyle en $C_{1-5}$)(alkyle en $C_{1-5}$), - (alkylène en $C_{0-4}$)-NH-$SO_2$-(alkyle en $C_{1-5}$), -(alkylène en $C_{0-4}$)-N(alkyle en $C_{1-5}$)-$SO_2$-(alkyle en $C_{1-5}$), -(alkylène en $C_{0-4}$)-$SO_2$-(alkyle en $C_{1-5}$), -(alkylène en $C_{0-4}$)-SO-(alkyle en $C_{1-5}$), -(alkylène en $C_{0-4}$)-carbocyclyle, et -(alkylène en $C_{0-4}$)-hétérocyclyle, où la fraction carbocyclyle dans ledit -(alkylène en $C_{0-4}$)-carbocyclyle et la fraction hétérocyclyle dans ledit -(alkylène en $C_{0-4}$)-hétérocyclyle sont chacune éventuellement substituées par un ou plusieurs groupes $R^{Cyc}$ ; et

chaque $R^{Cyc}$ est choisi indépendamment parmi un alkyle en $C_{1-5}$, alcényle en $C_{2-5}$, alcynyle en $C_{2-5}$, -(alkylène en $C_{0-4}$)-OH, -(alkylène en $C_{0-4}$)-O(alkyle en $C_{1-5}$), - (alkylène en $C_{0-4}$)-O(alkylène en $C_{1-5}$)-OH, -(alkylène en $C_{0-4}$)-O(alkylène en $C_{1-5}$)-O(alkyle en $C_{1-5}$), -(alkylène en $C_{0-4}$)-SH, -(alkylène en $C_{0-4}$)-S(alkyle en $C_{1-5}$), -(alkylène en $C_{0-4}$)-$NH_2$, -(alkylène en $C_{0-4}$)-NH(alkyle en $C_{1-5}$), -(alkylène en $C_{0-4}$)-N(alkyle en $C_{1-5}$)(alkyle en $C_{1-5}$), -(alkylène en $C_{0-4}$)-NH-OH, -(alkylène en $C_{0-4}$)-N(alkyle en $C_{1-5}$)-OH, -(alkylène en $C_{0-4}$)-NH-O(alkyle en $C_{1-5}$), -(alkylène en $C_{0-4}$)-N(alkyle en $C_{1-5}$)-O(alkyle en $C_{1-5}$), halogène, halogénoalkyle en $C_{1-5}$, -(alkylène en $C_{0-4}$)-O-(halogénoalkyle en $C_{1-5}$), -(alkylène en $C_{0-4}$)-CN, -(alkylène en $C_{0-4}$)-CHO, - (alkylène en $C_{0-4}$)-CO-(al-

kyle en $C_{1-5}$), -(alkylène en $C_{0-4}$)-COOH, -(alkylène en $C_{0-4}$)-CO-O-(alkyle en $C_{1-5}$), -(alkylène en $C_{0-4}$)-O-CO-(alkyle en $C_{1-5}$), -(alkylène en $C_{0-4}$)-CO-$NH_2$, -(alkylène en $C_{0-4}$)-CO-NH(alkyle en $C_{1-5}$), -(alkylène en $C_{0-4}$)-CON(alkyle en $C_{1-5}$)(alkyle en $C_{1-5}$), -(alkylène en $C_{0-4}$)-NH-CO-(alkyle en $C_{1-5}$), - (alkylène en $C_{0-4}$)-N(alkyle en $C_{1-5}$)-CO-(alkyle en $C_{1-5}$), -(alkylène en $C_{0-4}$)-NH-CO-O-(alkyle en $C_{1-5}$), -(alkylène en $C_{0-4}$)-N(alkyle en $C_{1-5}$)-CO-O-(alkyle en $C_{1-5}$), - (alkylène en $C_{0-4}$)-O-CO-NH-(alkyle en $C_{1-5}$), -(alkylène en $C_{0-4}$)-O-CO-N(alkyle en $C_{1-5}$)-(alkyle en $C_{1-5}$), -(alkylène en $C_{0-4}$)-$SO_2$-$NH_2$, -(alkylène en $C_{0-4}$)-$SO_2$-NH(alkyle en $C_{1-5}$), -(alkylène en $C_{0-4}$)-$SO_2$-N(alkyle en $C_{1-5}$)(alkyle en $C_{1-5}$), - (alkylène en $C_{0-4}$)-NH-$SO_2$-(alkyle en $C_{1-5}$), -(alkylène en $C_{0-4}$)-N(alkyle en $C_{1-5}$)-$SO_2$-(alkyle en $C_{1-5}$), -(alkylène en $C_{0-4}$)-$SO_2$-(alkyle en $C_{1-5}$), et -(alkylène en $C_{0-4}$)-SO-(alkyle en $C_{1-5}$) ;
ou un sel ou solvate pharmaceutiquement acceptable de celui-ci ;
pour son utilisation dans le traitement ou la prévention d'un trouble associé à l'X fragile.

**2.** Composé pour son utilisation selon la revendication 1, dans lequel $R^1$ est un alkyle en $C_{1-5}$.

**3.** Composé pour son utilisation selon la revendication 1 ou 2, dans lequel $R^5$ est un -O(alkyle en $C_{1-5}$) ou -OH.

**4.** Composé pour son utilisation selon l'une quelconque des revendications 1 à 3, dans lequel $Z^1$, $Z^2$ et $Z^3$ sont chacun C(-$R^6$).

**5.** Composé pour son utilisation selon l'une quelconque des revendications 1 à 4, dans lequel :

(i) $R^4$ est un halogène, -$CF_3$ ou -CN, de préférence dans lequel $R^4$ est -Cl ; et/ou
(ii) chaque $R^6$ est choisi indépendamment parmi un hydrogène, alkyle en $C_{1-5}$, - O(alkyle en $C_{1-5}$), halogène, halogénoalkyle en $C_{1-5}$, -O-(halogénoalkyle en $C_{1-5}$), et - CN, de préférence dans lequel chaque $R^6$ est un hydrogène.

**6.** Composé pour son utilisation selon la revendication 1, dans lequel ledit composé est choisi parmi :

3-(5-chloro-2-méthoxyphényl)-3-méthyl-6-(trifluorométhyl)indolin-2-one ;
3-(5-chloro-2-hydroxy-phényl)-3-méthyl-6-(trifluorométhyl)indolin-2-one ;
3-(5-chloro-2-méthoxyphényl)-3-éthyl-6-(trifluorométhyl)indolin-2-one ;
3-(5-chloro-2-méthoxyphényl)-3-propyl-6-(trifluorométhyl)indolin-2-one ;
2-(3-(5-chloro-2-méthoxyphényl)-2-oxo-6-(trifluorométhyl)indolin-3-yl)acétonitrile ;
3-allyl-3-(5-chloro-2-méthoxyphényl)-6-(trifluorométhyl)indolin-2-one ;
acide 2-(3-(5-chloro-2-méthoxyphényl)-2-oxo-6-(trifluorométhyl)indolin-3-yl)acétique ;
3-(2-chloro-5-méthoxy-4-pyridyl)-3-méthyl-6-(trifluorométhyl)indolin-2-one ;
3-(3-méthoxy-4-pyridyl)-3-méthyl-6-(trifluorométhyl)indolin-2-one ;
3-méthyl-3-(4-pyridyl)-6-(trifluorométhyl)indolin-2-one ;
3-(2-chloro-4-pyridyl)-3-méthyl-6-(trifluorométhyl)indolin-2-one ;
3-[2-méthoxy-5-(1-méthylimidazol-2-yl)phényl]-3-méthyl-6-(trifluorométhyl)indolin-2- one ;
3-(5-chloro-2-méthoxy-phényl)-6-(difluorométhyl)-3-méthyl-indolin-2-one ;
3-(5-chloro-2-méthoxy-3-pyridyl)-3-méthyl-6-(trifluorométhyl)indolin-2-one ;
6-chloro-3-(5-chloro-2-méthoxyphényl)-3-méthylindolin-2-one ;
6-chloro-3-(2-chloro-5-méthoxypyridin-4-yl)-3-méthylindolin-2-one ;
3-(6-chloro-3-méthoxypyridin-2-yl)-3-méthyl-6-(trifluorométhyl)indolin-2-one ;
6-chloro-3-(5-chloro-2-méthoxypyridin-3-yl)-3-méthylindolin-2-one ;
6-chloro-3-(2-méthoxy-5-(1-méthyl-1H-imidazol-2-yl)phényl)-3-méthylindolin-2-one ;
6-chloro-3-(6-chloro-3-méthoxypyridin-2-yl)-3-méthylindolin-2-one ;
6-chloro-3-(2-méthoxy-5-(1-méthyl-1H-imidazol-2-yl)pyridin-3-yl)-3-méthylindolin-2-one ;
6-chloro-3-(5-méthoxy-2-(1-méthyl-1H-imidazol-2-yl)pyridin-4-yl)-3-méthylindolin-2-one ;
ou un sel ou solvate pharmaceutiquement acceptable de ceux-ci ;
de préférence dans lequel ledit composé est la 3-(5-chloro-2-méthoxyphényl)-3-méthyl-6-(trifluorométhyl)indolin-2-one ou un sel ou solvate pharmaceutiquement acceptable de celle-ci ; de manière encore préférée dans lequel ledit composé est la (3R)-3-(5-chloro-2-méthoxyphényl)-3-méthyl-6-(trifluorométhyl)indolin-2-one ou un sel ou solvate pharmaceutiquement acceptable de celle-ci.

**7.** Composé pour son utilisation selon l'une quelconque des revendications 1 à 6, dans lequel le trouble associé à l'X fragile est choisi parmi le syndrome de l'X fragile, le syndrome de tremblement/d'ataxie associé à l'X fragile,

l'insuffisance ovarienne primaire associée à l'X fragile, le syndrome ovarien polykystique associé à l'X fragile, et le retard mental associé au syndrome FRAXE.

**8.** Composé pour son utilisation selon l'une quelconque des revendications 1 à 7, dans lequel le trouble associé à l'X fragile est le syndrome de l'X fragile.

**9.** Utilisation *in vitro* d'un composé tel que défini selon l'une quelconque des revendications 1 à 6 en tant qu'agent d'ouverture du canal potassique maxi-K.

**10.** Composé selon la formule (Ia)

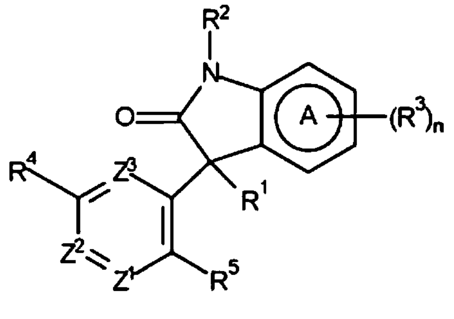

(Ia)

dans laquelle :

$R^1$ est choisi parmi un alkyle en $C_{1-5}$, alcényle en $C_{2-5}$, alcynyle en $C_{2-5}$, -(alkylène en $C_{0-4}$)-CN, -(alkylène en $C_{1-4}$)-COOH, -(alkylène en $C_{0-4}$)-COO-(alkyle en $C_{1-5}$), - (alkylène en $C_{1-4}$)-O-CO-(alkyle en $C_{1-5}$), -(alkylène en $C_{0-4}$)-CO-(alkyle en $C_{1-5}$), - (alkylène en $C_{0-4}$)-CO-$NH_2$, -(alkylène en $C_{0-4}$)-CO-NH(alkyle en $C_{1-5}$), et -(alkylène en $C_{0-4}$)-CO-N(alkyle en $C_{1-5}$)(alkyle en $C_{1-5}$), et $R^5$ est un groupe $R^{5A}$ ;
$R^2$ est un hydrogène ;
le cycle A est un phényle lequel est condensé au cycle contenant -CO-$NR^2$- ;
chaque $R^3$ est indépendamment un halogène ou halogénoalkyle en $C_{1-5}$ ;
n vaut 1, 2, 3 ou 4 ;
un des atomes $Z^1$, $Z^2$ et $Z^3$ du cycle est C(-$R^6$) ou N, et les deux autres desdits atomes du cycle sont chacun C(-$R^6$) ;
$R^4$ est choisi parmi un hydrogène, alkyle en $C_{1-3}$, alcényle en $C_{2-5}$, alcynyle en $C_{2-5}$, -(alkylène en $C_{0-4}$)-OH, -(alkylène en $C_{0-4}$)-O(alkyle en $C_{1-5}$), -(alkylène en $C_{0-4}$)-O(alkylène en $C_{1-5}$)-OH, -(alkylène en $C_{0-4}$)-O(alkylène en $C_{1-5}$)-O(alkyle en $C_{1-5}$), - (alkylène en $C_{0-4}$)-SH, -(alkylène en $C_{0-4}$)-S(alkyle en $C_{1-5}$), -(alkylène en $C_{0-4}$)-$NH_2$, -(alkylène en $C_{0-4}$)-NH(alkyle en $C_{1-5}$), -(alkylène en $C_{0-4}$)-N(alkyle en $C_{1-5}$)(alkyle en $C_{1-5}$), -(alkylène en $C_{0-4}$)-NH-OH, -(alkylène en $C_{0-4}$)-N(alkyle en $C_{1-5}$)-OH, - (alkylène en $C_{0-4}$)-NH-O(alkyle en $C_{1-5}$), -(alkylène en $C_{0-4}$)-N(alkyle en $C_{1-5}$)-O(alkyle en $C_{1-5}$), halogène, halogénoalkyle en $C_{1-5}$, -(alkylène en $C_{0-4}$)-O-(halogénoalkyle en $C_{1-5}$), -(alkylène en $C_{0-4}$)-CN, -(alkylène en $C_{0-4}$)-CHO, -(alkylène en $C_{0-4}$)-CO-(alkyle en $C_{1-5}$), -(alkylène en $C_{0-4}$)-COOH, -(alkylène en $C_{0-4}$)-CO-O-(alkyle en $C_{1-5}$), -(alkylène en $C_{0-4}$)-O-CO-(alkyle en $C_{1-5}$), -(alkylène en $C_{0-4}$)-CO-$NH_2$, -(alkylène en $C_{0-4}$)-CO-NH(alkyle en $C_{1-5}$), -(alkylène en $C_{0-4}$)-CO-N(alkyle en $C_{1-5}$) (alkyle en $C_{1-5}$), -(alkylène en $C_{0-4}$)-NH-CO-(alkyle en $C_{1-5}$), -(alkylène en $C_{0-4}$)-N(alkyle en $C_{1-5}$)-CO-(alkyle en $C_{1-5}$), -(alkylène en $C_{0-4}$)-NH-CO-O-(alkyle en $C_{1-5}$), -(alkylène en $C_{0-4}$)-N(alkyle en $C_{1-5}$)-CO-O-(alkyle en $C_{1-5}$), -(alkylène en $C_{0-4}$)-O-CO-NH-(alkyle en $C_{1-5}$), -(alkylène en $C_{0-4}$)-O-CO-N(alkyle en $C_{1-5}$)-(alkyle en $C_{1-5}$), -(alkylène en $C_{0-4}$)-$SO_2$-$NH_2$, -(alkylène en $C_{0-4}$)-$SO_2$-NH(alkyle en $C_{1-5}$), -(alkylène en $C_{0-4}$)-$SO_2$-N(alkyle en $C_{1-5}$)(alkyle en $C_{1-5}$), -(alkylène en $C_{0-4}$)-NH-$SO_2$-(alkyle en $C_{1-5}$), -(alkylène en $C_{0-4}$)-N(alkyle en $C_{1-5}$)-$SO_2$-(alkyle en $C_{1-5}$), -(alkylène en $C_{0-4}$)-$SO_2$-(alkyle en $C_{1-5}$), -(alkylène en $C_{0-4}$)-SO-(alkyle en $C_{1-5}$), -(alkylène en $C_{0-4}$)-carbocyclyle, et -(alkylène en $C_{0-4}$)-hétérocyclyle, où la fraction carbocyclyle dans ledit -(alkylène en $C_{0-4}$)-carbocyclyle et la fraction hétérocyclyle dans ledit -(alkylène en $C_{0-4}$)-hétérocyclyle sont chacune éventuellement substituées par un ou plusieurs groupes $R^{Cyc}$ ;
$R^{5A}$ est choisi parmi un hydrogène, alkyle en $C_{1-5}$, alcényle en $C_{2-5}$, alcynyle en $C_{2-5}$, -(alkylène en $C_{0-4}$)-OH, -(alkylène en $C_{0-4}$)-O(alkyle en $C_{1-5}$), -(alkylène en $C_{0-4}$)-O(alkylène en $C_{1-5}$)-OH, -(alkylène en $C_{0-4}$)-O(alkylène en $C_{1-5}$)-O(alkyle en $C_{1-5}$), - (alkylène en $C_{0-4}$)-SH, -(alkylène en $C_{0-4}$)-S(alkyle en $C_{1-5}$), -(alkylène en $C_{0-4}$)-$NH_2$, -(alkylène en $C_{0-4}$)-NH(alkyle en $C_{1-5}$), -(alkylène en $C_{0-4}$)-N(alkyle en $C_{1-5}$)(alkyle en $C_{1-5}$), -(alkylène en

$C_{0-4}$)-NH-OH, -(alkylène en $C_{0-4}$)-N(alkyle en $C_{1-5}$)-OH, - (alkylène en $C_{0-4}$)-NH-O(alkyle en $C_{1-5}$), -(alkylène en $C_{0-4}$)-N(alkyle en $C_{1-5}$)-O(alkyle en $C_{1-5}$), halogène, halogénoalkyle en $C_{1-5}$, -(alkylène en $C_{0-4}$)-O-(halogénoalkyle en $C_{1-5}$), -(alkylène en $C_{0-4}$)-CN, -(alkylène en $C_{0-4}$)-CHO, -(alkylène en $C_{0-4}$)-CO-(alkyle en $C_{1-5}$), -(alkylène en $C_{0-4}$)-COOH, -(alkylène en $C_{0-4}$)-CO-O-(alkyle en $C_{1-5}$), -(alkylène en $C_{0-4}$)-O-CO-(alkyle en $C_{1-5}$), -(alkylène en $C_{0-4}$)-CO-$NH_2$, -(alkylène en $C_{0-4}$)-CO-NH(alkyle en $C_{1-5}$), -(alkylène en $C_{0-4}$)-CO-N(alkyle en $C_{1-5}$)(alkyle en $C_{1-5}$), -(alkylène en $C_{0-4}$)-NH-CO-(alkyle en $C_{1-5}$), -(alkylène en $C_{0-4}$)-N(alkyle en $C_{1-5}$)-CO-(alkyle en $C_{1-5}$), -(alkylène en $C_{0-4}$)-NH-CO-O-(alkyle en $C_{1-5}$), -(alkylène en $C_{0-4}$)-N(alkyle en $C_{1-5}$)-CO-O-(alkyle en $C_{1-5}$), -(alkylène en $C_{0-4}$)-O-CO-NH-(alkyle en $C_{1-5}$), -(alkylène en $C_{0-4}$)-O-CO-N(alkyle en $C_{1-5}$)-(alkyle en $C_{1-5}$), -(alkylène en $C_{0-4}$)-$SO_2$-$NH_2$, -(alkylène en $C_{0-4}$)-$SO_2$-NH(alkyle en $C_{1-5}$), -(alkylène en $C_{0-4}$)-$SO_2$-N(alkyle en $C_{1-5}$)(alkyle en $C_{1-5}$), -(alkylène en $C_{0-4}$)-NH-$SO_2$-(alkyle en $C_{1-5}$), -(alkylène en $C_{0-4}$)-N(alkyle en $C_{1-5}$)-$SO_2$-(alkyle en $C_{1-5}$), -(alkylène en $C_{0-4}$)-$SO_2$-(alkyle en $C_{1-5}$), -(alkylène en $C_{0-4}$)-SO-(alkyle en $C_{1-5}$), -(alkylène en $C_{0-4}$)-carbocyclyle, et -(alkylène en $C_{0-4}$)-hétérocyclyle, où la fraction carbocyclyle dans ledit -(alkylène en $C_{0-4}$)-carbocyclyle et la fraction hétérocyclyle dans ledit -(alkylène en $C_{0-4}$)-hétérocyclyle sont chacune éventuellement substituées par un ou plusieurs groupes $R^{Cyc}$ ;

chaque $R^6$ est choisi indépendamment parmi un hydrogène, alkyle en $C_{1-5}$, alcényle en $C_{2-5}$, alcynyle en $C_{2-5}$, -(alkylène en $C_{0-4}$)-OH, -(alkylène en $C_{0-4}$)-O(alkyle en $C_{1-5}$), -(alkylène en $C_{0-4}$)-O(alkylène en $C_{1-5}$)-OH, -(alkylène en $C_{0-4}$)-O(alkylène en $C_{1-5}$)-O(alkyle en $C_{1-5}$), -(alkylène en $C_{0-4}$)-SH, -(alkylène en $C_{0-4}$)-S(alkyle en $C_{1-5}$), - (alkylène en $C_{0-4}$)-$NH_2$, -(alkylène en $C_{0-4}$)-NH(alkyle en $C_{1-5}$), -(alkylène en $C_{0-4}$)-N(alkyle en $C_{1-5}$)(alkyle en $C_{1-5}$), -(alkylène en $C_{0-4}$)-NH-OH, -(alkylène en $C_{0-4}$)-N(alkyle en $C_{1-5}$)-OH, -(alkylène en $C_{0-4}$)-NH-O(alkyle en $C_{1-5}$), -(alkylène en $C_{0-4}$)-N(alkyle en $C_{1-5}$)-O(alkyle en $C_{1-5}$), halogène, halogénoalkyle en $C_{1-5}$, -(alkylène en $C_{0-4}$)-O-(halogénoalkyle en $C_{1-5}$), -(alkylène en $C_{0-4}$)-CN, -(alkylène en $C_{0-4}$)-CHO, - (alkylène en $C_{0-4}$)-CO-(alkyle en $C_{1-5}$), -(alkylène en $C_{0-4}$)-COOH, -(alkylène en $C_{0-4}$)-CO-O-(alkyle en $C_{1-5}$),-(alkylène en $C_{0-4}$)-O-CO-(alkyle en $C_{1-5}$),-(alkylène en $C_{0-4}$)-CO-$NH_2$, -(alkylène en $Co_{-4}$)-CO-NH(alkyle en $C_{1-5}$), -(alkylène en $C_{0-4}$)-CON(alkyle en $C_{1-5}$)(alkyle en $C_{1-5}$), -(alkylène en $C_{0-4}$)-NH-CO-(alkyle en $C_{1-5}$), - (alkylène en $C_{0-4}$)-N(alkyle en $C_{1-5}$)-CO-(alkyle en $C_{1-5}$), -(alkylène en $C_{0-4}$)-NH-CO-O-(alkyle en $C_{1-5}$), -(alkylène en $C_{0-4}$)-N(alkyle en $C_{1-5}$)-CO-O-(alkyle en $C_{1-5}$), - (alkylène en $C_{0-4}$)-O-CO-NH-(alkyle en $C_{1-5}$), -(alkylène en $C_{0-4}$)-O-CO-N(alkyle en $C_{1-5}$)-(alkyle en $C_{1-5}$), -(alkylène en $C_{0-4}$)-$SO_2$-$NH_2$, -(alkylène en $C_{0-4}$)-$SO_2$-NH(alkyle en $C_{1-5}$), -(alkylène en $C_{0-4}$)-$SO_2$-N(alkyle en $C_{1-5}$)(alkyle en $C_{1-5}$), - (alkylène en $C_{0-4}$)-NH-$SO_2$-(alkyle en $C_{1-5}$), -(alkylène en $C_{0-4}$)-N(alkyle en $C_{1-5}$)-$SO_2$-(alkyle en $C_{1-5}$), -(alkylène en $C_{0-4}$)-$SO_2$-(alkyle en $C_{1-5}$), -(alkylène en $C_{0-4}$)-SO-(alkyle en $C_{1-5}$), -(alkylène en $C_{0-4}$)-carbocyclyle, et -(alkylène en $C_{0-4}$)-hétérocyclyle, où la fraction carbocyclyle dans ledit -(alkylène en $C_{0-4}$)-carbocyclyle et la fraction hétérocyclyle dans ledit -(alkylène en $C_{0-4}$)-hétérocyclyle sont chacune éventuellement substituées par un ou plusieurs groupes $R^{Cyc}$ ; et

chaque $R^{Cyc}$ est choisi indépendamment parmi un alkyle en $C_{1-5}$, alcényle en $C_{2-5}$, alcynyle en $C_{2-5}$, -(alkylène en $C_{0-4}$)-OH, -(alkylène en $C_{0-4}$)-O(alkyle en $C_{1-5}$), - (alkylène en $C_{0-4}$)-O(alkylène en $C_{1-5}$)-OH, -(alkylène en $C_{0-4}$)-O(alkylène en $C_{1-5}$)-O(alkyle en $C_{1-5}$), -(alkylène en $C_{0-4}$)-SH, -(alkylène en $C_{0-4}$)-S(alkyle en $C_{1-5}$), -(alkylène en $C_{0-4}$)-$NH_2$, -(alkylène en $C_{0-4}$)-NH(alkyle en $C_{1-5}$), -(alkylène en $C_{0-4}$)-N(alkyle en $C_{1-5}$)(alkyle en $C_{1-5}$), -(alkylène en $C_{0-4}$)-NH-OH, -(alkylène en $C_{0-4}$)-N(alkyle en $C_{1-5}$)-OH, -(alkylène en $C_{0-4}$)-NH-O(alkyle en $C_{1-5}$), -(alkylène en $C_{0-4}$)-N(alkyle en $C_{1-5}$)-O(alkyle en $C_{1-3}$), halogène, halogénoalkyle en $C_{1-5}$, -(alkylène en $C_{0-4}$)-O-(halogénoalkyle en $C_{1-5}$), -(alkylène en $C_{0-4}$)-CN, -(alkylène en $C_{0-4}$)-CHO, - (alkylène en $C_{0-4}$)-CO-(alkyle en $C_{1-5}$), -(alkylène en $C_{0-4}$)-COOH, -(alkylène en $C_{0-4}$)-CO-O-(alkyle en $C_{1-5}$),-(alkylène en $C_{0-4}$)-O-CO-(alkyle en $C_{1-5}$),-(alkylène en $C_{0-4}$)-CO-$NH_2$, -(alkylène en $C_{0-4}$)-CO-NH(alkyle en $C_{1-5}$), -(alkylène en $C_{0-4}$)-CON(alkyle en $C_{1-5}$)(alkyle en $C_{1-5}$), -(alkylène en $C_{0-4}$)-NH-CO-(alkyle en $C_{1-5}$), - (alkylène en $C_{0-4}$)-N(alkyle en $C_{1-5}$)-CO-(alkyle en $C_{1-5}$), -(alkylène en $C_{0-4}$)-NH-CO-O-(alkyle en $C_{1-5}$), -(alkylène en $C_{0-4}$)-N(alkyle en $C_{1-5}$)-CO-O-(alkyle en $C_{1-5}$), - (alkylène en $C_{0-4}$)-O-CO-NH-(alkyle en $C_{1-5}$), -(alkylène en $C_{0-4}$)-O-CO-N(alkyle en $C_{1-5}$)-(alkyle en $C_{1-5}$), -(alkylène en $C_{0-4}$)-$SO_2$-$NH_2$, -(alkylène en $C_{0-4}$)-$SO_2$-NH(alkyle en $C_{1-5}$), -(alkylène en $C_{0-4}$)-$SO_2$-N(alkyle en $C_{1-5}$)(alkyle en $C_{1-5}$), - (alkylène en $C_{0-4}$)-NH-$SO_2$-(alkyle en $C_{1-5}$), -(alkylène en $C_{0-4}$)-N(alkyle en $C_{1-5}$)-$SO_2$-(alkyle en $C_{1-5}$), -(alkylène en $C_{0-4}$)-$SO_2$-(alkyle en $C_{1-5}$), et -(alkylène en $C_{0-4}$)-SO-(alkyle en $C_{1-5}$) ;

ou un sel ou solvate pharmaceutiquement acceptable de celui-ci ;

dans lequel si n vaut 1 ou 2, alors au moins un groupe $R^3$ est différent d'un halogène.

**11.** Composé selon la revendication 10, dans lequel $R^1$ est un alkyle en $C_{1-5}$.

**12.** Composé selon la revendication 10 ou 11, dans lequel $R^5$ est un -O(alkyle en $C_{1-5}$) ou -OH.

**13.** Composé selon l'une quelconque des revendications 10 à 12, dans lequel $Z^1$, $Z^2$ et $Z^3$ sont chacun C(-$R^6$).

**14.** Composé selon l'une quelconque des revendications 10 à 13, dans lequel :

(i) $R^4$ est un halogène, $-CF_3$ ou -CN, de préférence dans lequel $R^4$ est -Cl ; et/ou
(ii) chaque $R^6$ est choisi indépendamment parmi un hydrogène, alkyle en $C_{1-5}$, - O(alkyle en $C_{1-5}$), halogène, halogénoalkyle en $C_{1-5}$, -O-(halogénoalkyle en $C_{1-5}$), et - CN, de préférence dans lequel chaque $R^6$ est un hydrogène.

**15.** Composé selon la revendication 10, dans lequel ledit composé est choisi parmi :

3-(5-chloro-2-méthoxyphényl)-3-méthyl-6-(trifluorométhyl)indolin-2-one ;
3-(5-chloro-2-hydroxy-phényl)-3-méthyl-6-(trifluorométhyl)indolin-2-one ;
3-(5-chloro-2-méthoxyphényl)-3-éthyl-6-(trifluorométhyl)indolin-2-one ;
3-(5-chloro-2-méthoxyphényl)-3-propyl-6-(trifluorométhyl)indolin-2-one ;
2-(3-(5-chloro-2-méthoxyphényl)-2-oxo-6-(trifluorométhyl)indolin-3-yl)acétonitrile ;
3-allyl-3-(5-chloro-2-méthoxyphényl)-6-(trifluorométhyl)indolin-2-one ;
acide 2-(3-(5-chloro-2-méthoxyphényl)-2-oxo-6-(trifluorométhyl)indolin-3-yl)acétique ;
3-(2-chloro-5--méthoxy-4-pyridyl)-3-méthyl-6-({trifluorométhyl)indolin-2-one ;
3-(3-méthoxy-4-pyridyl)-3-méthyl-6-(trifluorométhyl)indolin-2-one ;
3-méthyl-3-(4-pyridyl)-6-(trifluorométhyl)indolin-2-one ;
3-(2-chloro-4-pyridyl)-3-méthyl-6-(trifluorométhyl)indolin-2-one ;
3-[2-méthoxy-5-(1-méthylimidazol-2-yl)phényl]-3-méthyl-6-(trifluorométhyl)indolin-2- one ;
3-(5-chloro-2-méthoxy-phényl)-6--(difluorométhyl)-3-méthyl-indolin-2-one ;
3-(5-chloro-2-méthoxy-3-pyridyl)-3-méthyl-6-(trifluorométhyl)indolin-2-one ;
3-(6-chloro-3-méthoxypyridin-2-yl)-3-méthyl-6-(trifluorométhyl)indolin-2-one ;
ou un sel ou solvate pharmaceutiquement acceptable de ceux-ci ;
de préférence dans lequel ledit composé est la 3-(5-chloro-2-méthoxyphényl)-3-méthyl-6-(trifluorométhyl)indolin-2-one ou un sel ou solvate pharmaceutiquement acceptable de celle-ci ; de manière encore préférée dans lequel ledit composé est la (3R)-3-(5-chloro-2-méthoxyphényl)-3-méthyl-6-(trifluorométhyl)indolin-2-one ou un sel ou solvate pharmaceutiquement acceptable de celle-ci.

**16.** Composé selon l'une quelconque des revendications 10 à 15 pour son utilisation en tant que médicament.

**17.** Composé selon l'une quelconque des revendications 10 à 15 pour son utilisation dans le traitement ou la prévention d'une maladie/d'un trouble choisi parmi l'AVC, la lésion d'ischémie-reperfusion, la cardiopathie ischémique, l'hypertension, l'infarctus du myocarde, la rhinite allergique, l'asthme, la bronchopneumopathie chronique obstructive, la sclérose en plaques, la spasticité, les tremblements, un trouble musculaire, la dyskinésie, la dysfonction vésicale, la vessie hyperactive, l'incontinence urinaire, le syndrome de l'intestin irritable, l'incontinence fécale, la constipation, le reflux gastro-œsophagien, l'altération du transit gastro-intestinal, l'hypoactivité détrusorienne, la dysfonction érectile, la dépression respiratoire induite par les opioïdes, l'anxiété, un trouble anxieux, le trouble du traitement sensoriel, l'autisme, un trouble du spectre autistique, un trouble de la communication sociale ou pragmatique, un trouble du déficit de l'attention avec hyperactivité, la phobie sociale, la déficience intellectuelle, la douleur, l'épilepsie, un trouble épileptique et/ou convulsif, le syndrome de Dravet, l'épilepsie généralisée et la dyskinésie paroxysmale, l'épilepsie du lobe temporal, la psychose, la schizophrénie, les symptômes négatifs de la schizophrénie, le déclin cognitif dans la schizophrénie, le syndrome de Phelan-McDermid, le syndrome de Rett, le syndrome de Pitt-Hopkins, le syndrome de la délétion de 16p11.2, le syndrome de Williams-Beuren, l'hypertension oculaire à angle ouvert, le glaucome, les acouphènes, la rétinopathie diabétique, la tocolyse, la migraine, le syndrome de Liang-Wang, le syndrome de Smith-Lemli-Opitz, le syndrome d'Angelman, et le syndrome de la progéria de Hutchinson-Gilford.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5565483 A
- US 5602169 A
- WO 0200217 A
- WO 0232419 A
- WO 02066426 A
- WO 03080047 A
- WO 2013001412 A
- US 20070203184 A
- WO 0174775 A
- WO 2014154760 A


### Non-patent literature cited in the description


- **RILEY C et al.** *Pediatrics*, 2017, vol. 139, S147-S52
- **THURMAN AJ et al.** *Res Dev Disabil*, 2014, vol. 35, 1072-86
- **GRIGSBY J**. *Clin Neuropsychol*, 2016, vol. 30, 815-33
- **LATORRE R et al.** *Biol Res*, 2006, vol. 39 (3), 385-401
- **GRIBKOFF VK et al.** *Nat Med*, 2001, vol. 7, 471-7
- **LAUMONNIER F et al.** *Am J Psychiatry*, 2006, vol. 163, 1622-9
- **DENG PY et al.** *Neuron*, 2013, vol. 77, 696-711
- **DENG PY et al.** *J Physiol*, 2016, vol. 594, 83-97
- **HÉBERT B et al.** *Orphanet J Rare Dis*, 2014, vol. 9, 124
- **PIQUEMAL M et al.** *bioRxiv*, 2020
- **JENSEN BS**. *CNS Drug Rev*, 2002, vol. 8 (4), 353-60
- Influence of aromatic rings on ADME properties of drugs. **DALVIE D et al.** Metabolism, Pharmacokinetics and Toxicity of Functional Groups. Royal Society of Chemistry, 2010, 275-327
- **EVANS DC et al.** *Chem Res Toxicol*, 2004, vol. 17 (1), 3-16
- **HEWAWASAM P et al.** *Bioorg Med Chem Lett*, 2002, vol. 12 (7), 1023-6
- **KÜNDIG EP et al.** *Angew Chem Int Ed Engl*, 2007, vol. 46 (44), 8484-7
- **WÜRTZ S et al.** *J Am Chem Soc*, 2009, vol. 131 (24), 8344-5
- **LIU L et al.** *Org Lett*, 2011, vol. 13 (7), 1666-9
- **DEY C et al.** *Chem Commun (Camb)*, 2012, vol. 48 (25), 3064-6
- **KINTHADA LK et al.** *Org Biomol Chem*, 2014, vol. 12 (41), 8152-73
- **MADDALENA A et al.** *Genet Med*, 2001, vol. 3 (3), 200-5
- **MONAGHAN KG et al.** *Genet Med*, 2013, vol. 15 (7), 575-86
- **MULLEY JC et al.** *J Med Genet*, 1995, vol. 32 (3), 162-9
- **GECZ J**. *Ann Hum Genet*, 2000, vol. 64 (2), 95-106
- **MYRICK LK et al.** *Proc Natl Acad Sci USA*, 2015, vol. 112 (4), 949-56
- **HAGERMAN PJ et al.** *Ann N Y Acad Sci*, 2015, vol. 1338 (1), 58-70
- **JACQUEMONT S et al.** *Eur J Hum Genet*, 2011, vol. 19 (9)
- **HALL DA et al.** *Handb Clin Neurol*, 2018, vol. 147, 377-91
- FMR1 Disorders. **HUNTER JE et al.** GeneReviews. University of Washington, 1998
- **LOZANO R et al.** *Intractable Rare Dis Res*, 2014, vol. 3 (4), 134-46
- **LIANG L et al.** *Hum Mol Genet*, 2019, vol. 28 (17), 2937-51
- **ATZRODT J et al.** *Bioorg Med Chem*, 2012, vol. 20 (18), 5658-67
- **WILLIAM JS et al.** *Journal of Labelled Compounds and Radiopharmaceuticals*, 2010, vol. 53 (11-12), 635-44
- **MODVIG A et al.** *J Org Chem*, 2014, vol. 79, 5861-8
- *CHEMICAL ABSTRACTS*, 70142-34-6
- Remington: The Science and Practice of Pharmacy. Pharmaceutical Press